# EUROPEAN PATENT SPECIFICATION

(11) **EP 1 951 692 B1**
(45) Date of publication and mention of the grant of the patent: **18.08.2010**
(21) Application number: 06837214.3
(22) Date of filing: 07.11.2006
(51) Int. Cl.: C07D 263/48, C07D 277/42, A61K 31/422, A61K 31/427, A61P 3/00, A61P 9/00, A61P 17/00, A61P 25/28, A61P 29/00

(54) **OXAZOLE AND THIAZOLE PPAR MODULATOR**
OXAZOL- UND THIAZOL-VERBINDUNGEN ALS PPAR-MODULATOREN
MODULATEURS DES PPAR CONTENANT DE L'OXAZOLE ET DU THIAZOLE

(30) Priority: 07.11.2005 US 734678 P
(43) Date of publication of application: 06.08.2008
(73) Proprietor: IRM LLC, Hamilton HM 11 (BM)
(72) Inventor: EPPLE, Robert, San Diego, California 92122 (US); COW, Christopher, San Diego, California 92128 (US); AZIMIOARA, Mihai, La Jolla, California 92037 (US); RUSSO, Ross, Encinitas, California 92024 (US)
(74) Representative: Kiddle, Simon John
(86) International application number: PCT/US2006/043586
(87) International publication number: WO 2007/056496

(56) References cited:
- EP-A1- 1 354 879
- EP-A1- 1 371 650
- EP-A1- 1 445 256
- WO-A-2004/000785
- WO-A-2006/028970
- US-A1- 2006 160 868

## Description

### CROSS-REFERENCE TO RELATED APPLICATIONS

This application claims the benefit of priority to U.S. Provisional Patent Application Number 60/734,678, filed 07 November 2005.

### BACKGROUND OF THE INVENTION

### Field of the Invention

The invention provides compounds, pharmaceutical compositions comprising such compounds and methods of using such compounds to treat or prevent diseases or disorders associated with the activity of the Peroxisome Proliferator-Activated Receptor (PPAR) families.

### Background

Peroxisome Proliferator Activated Receptors (PPARs) are members of the nuclear hormone receptor super family, which are ligand-activated transcription factors regulating gene expression. Certain PPARs are associated with a number of disease states including dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, IBDs (irritable bowel disease), ulcerative colitis and Crohn's disease. Accordingly, molecules that modulate the activity of PPARs are useful as therapeutic agents in the treatment of such diseases.

WO 2006/028970 was published on 16 March 2006, after the priority date of the present application but before the filing date. It entered the European Regional Phase in 2007, and therefore is citeable against the present application under Article 54(3) EPC. It describes compounds of the following general formula (see claim 1), which are said to have activity in the modulation of tyrosine phosphatise:

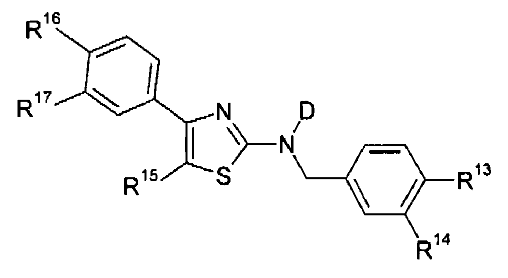

WO 2004/000785 describes compounds of the following general formula, which are said to be PPAR activators:

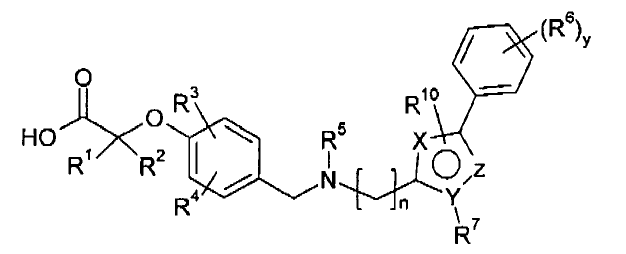

EP 1 445 256 describes compounds of the following general formula, which are said to have activity as PPAR regulators:

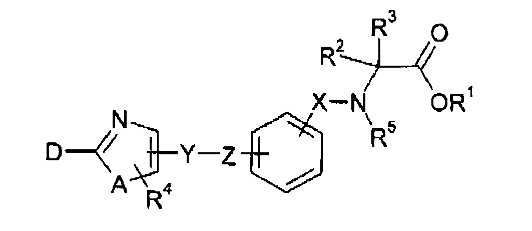

EP 1 371 650 describes compounds of the following general formula, which are said to be activators of PPARδ:

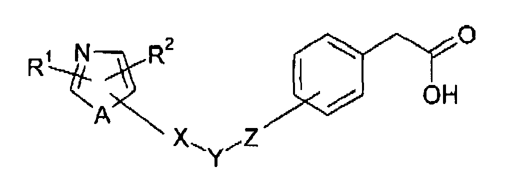

EP 1 354 879 describes compounds of the following general formula, which are said to be regulators of PPAR:

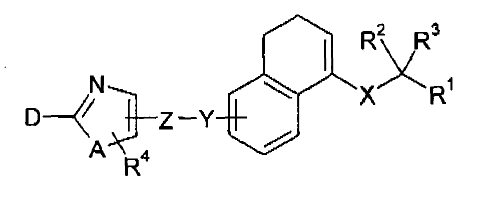

WO 2006/077206, corresponding to US 2006/160868, was published on 27 July 2006, after the priority date of the present application but before the filing date. It entered the European Regional Phase in 2007, and therefore is citeable against the present application under Article 54(3) EPC. It describes compounds of the following general formula, which are said to have activity as modulators of PPARγ: 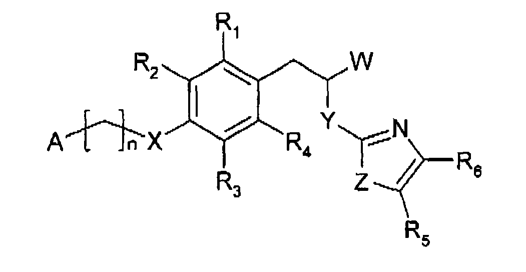 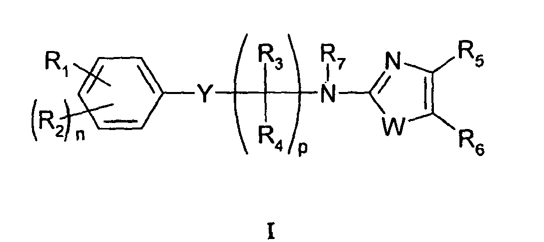 in which
n is selected from 0, 1, 2 and 3;
p is selected from 0, 1, 2 and 3;
Y is selected from O, S(O)₀₋₂, and NR₇ₐ; wherein R₇ₐ is selected from hydrogen and C₁₋₆alkyl;
W is selected from O and S;
R₁ is selected from -X₁CR₉R₁₀X₂CO₂R₁₁, -X₁SCR₉R₁₀X₂CO₂R₁₁ and-X₁OCR₉R₁₀X₂CO₂R₁₁; wherein X₁ and X₂ are independently selected from a bond and C₁₋₄alkylene; and R₉ and R₁₀ are independently selected from hydrogen, C₁₋₄alkyl and C₁₋₄alkoxy; or R₉ and R₁₀ together with the carbon atom to which R₉ and R₁₀ are attached form C₃₋₁₂cycloalkyl; and R₁₁ is selected from hydrogen and C₁₋₆alkyl; each
R₂ is independently selected from halo, C₁₋₆alkyl, C₂₋₆alkenyl, C₁₋₄alkoxy, C₁₋₄alkylthio, C₃₋₁₂cycloalkyl, C₃₋₈heterocycloalkyl, C₆₋₁₀aryl and C₅₋₁₀heteroaryl; wherein any aryl, heteroaryl, cycloalkyl or heterocycloalkyl of R₂ is optionally substituted with 1 to 3 radicals independently selected from halo, C₁₋₆alkyl, C₁₋₆alkoxy, C₂₋₆alkenyl, C₁₋₆alkylthio, halo-substituted-C₁₋₆alkyl, halo-substituted-C₁₋₆alkoxy, -C(O)R₁₄ₐ and NR₁₄ₐR_{14b}; wherein R₁₄ₐ and R_{14b} are independently selected from hydrogen and C₁₋₆alkyl;
R₃ and R₄ are independently selected from hydrogen and C₁₋₆alkyl;
R₅ is C₆₋₁₀aryl optionally substituted with 1 to 3 radicals independently selected from halo, nitro, cyano, C₁₋₆alkyl, C₁₋₆alkoxy, C₁₋₆alkylthio, hydroxy-C₁₋₆alkyl, halo-substituted-C₁₋₆alkyl, halo-substituted-C₁₋₆alkoxy, C₃₋₁₂cycloalkyl, C₃₋₈heterocydoalkyl, C₆₋₁₀aryl, C₅₋₁₃heteroaryl and -XNR¹²R¹²; wherein R¹² is selected from hydrogen and C₁₋₆alkyl;
R₆ is selected from hydrogen and methyl; and

R₇ is selected from hydrogen, C₁₋₆alkyl, C₆₋₁₂aryl-C₀₋₄alkyl, C₃₋₁₂cycloalkyl-C₀₋₄alkyl; -XOR₁₄ₐ and -XNR₁₄ₐR_{14b}; wherein X is a bond or C₁₋₄alkylene; and R₁₄ₐ and R_{14b} are independently selected from hydrogen and C₁₋₆alkyl; and the N-oxide derivatives, individual isomers and mixture of isomers thereof; and the pharmaceutically acceptable salts and solvates (e.g. hydrates) of such compounds.

In a second aspect, the present invention provides a pharmaceutical composition that contains a compound of Formula I or a N-oxide derivative, individual isomers and mixture of isomers thereof; or a pharmaceutically acceptable salt thereof, in admixture with one or more suitable excipients.

Also described herein is a method of treating a disease in an animal in which modulation of PPAR activity can prevent, inhibit or ameliorate the pathology and/or symptomology of the diseases, which method comprises administering to the animal a therapeutically effective amount of a compound of Formula I or a N-oxide derivative, individual isomers and mixture of isomers thereof, or a pharmaceutically acceptable salt thereof.

In a third aspect, the present invention provides the use of a compound of Formula I in the manufacture of a medicament for treating a disease in an animal in which PPAR activity activity contributes to the pathology and/or symptomology of the disease.

In a fourth aspect, the present invention provides a process for preparing compounds of Formula I and the N-oxide derivatives, prodrug derivatives, protected derivatives, individual isomers and mixture of isomers thereof, and the pharmaceutically acceptable salts thereof.

### DETAILED DESCRIPTION OF THE INVENTION

### Definitions

"Alkyl" as a group and as a structural element of other groups, for example halo-substituted-alkyl and alkoxy, can be either straight-chained or branched. C₁₋₆alkoxy includes, methoxy, ethoxy, and the like. Halo-substituted alkyl includes trifluoromethyl, pentafluoroethyl, and the like.

"Aryl" means a monocyclic or fused bicyclic aromatic ring assembly containing six to ten ring carbon atoms. For example, aryl can be phenyl or naphthyl, preferably phenyl. "Arylene" means a divalent radical derived from an aryl group. "Heteroaryl" is as defined for aryl where one or more of the ring members are a heteroatom. For example heteroaryl includes pyridyl, indolyl, indazolyl, quinoxalinyl, quinolinyl, benzofuranyl, benzopyranyl, benzothiopyranyl, benzo[1,3]dioxole, imidazolyl, benzo-imidazolyl, pyrimidinyl, furanyl, oxazolyl, isoxazolyl, triazolyl, tetrazolyl, pyrazolyl, thienyl, etc. "C₆₋₁₀arylC₀₋₄alkyl" means an aryl as described above connected via a alkylene grouping. For example, C₆₋₁₀arylC₀₋₄alkyl includes phenethyl, benzyl, etc.

"Cycloalkyl" means a saturated or partially unsaturated, monocyclic, fused bicyclic or bridged polycyclic ring assembly containing the number of ring atoms indicated. For example, C₃₋₁₀cycloalkyl includes cyclopropyl, cyclobutyl, cyclopentyl, cyclohexyl, etc. "Heterocycloalkyl" means cycloalkyl, as defined in this application, provided that one or more of the ring carbons indicated, are replaced by a moiety selected from -O-, -N=, -NR-, -C(O) -, -S-, -S(O) - or -S(O)₂-, wherein R is hydrogen, C₁₋₄alkyl or a nitrogen protecting group. For example, C₃₋₈heterocycloalkyl as used in this application to describe compounds of the invention includes morpholino, pyrrolidinyl, piperazinyl, piperidinyl, piperidinylone, 1,4-dioxa-8-aza-spiro[4.5]dec-8-yl, etc.

"Halogen" (or halo) preferably represents chloro or fluoro, but can also be bromo or iodo.

"Treat", "treating" and "treatment" refer to a method of alleviating or abating a disease and/or its attendant symptoms.

### Description of the Preferred Embodiments

The present invention provides compounds and compositions for the treatment of diseases in which modulation of one or more PPARs can prevent, inhibit or ameliorate the pathology and/or symptomology of the diseases, and describes a method of treatment which comprises administering to the animal a therapeutically effective amount of a compound of Formula I.

In one embodiment, with reference to compounds of Formula I:
n is selected from 0, 1, 2 and 3;
p is selected from 0, 1 and 2;
Y is selected from O and S(O)₀₋₂;
W is selected from O and S;
R₁ is selected from -X₁CR₉R₁₀X₂CO₂R₁₁, -X₁SCR₉R₁₀X₂CO₂R₁₁ and - X₁OCR₉R₁₀X₂CO₂R₁₁; wherein X₁ and X₂ are independently selected from a bond and C₁₋₄alkylene; and R₉ and R₁₀ are independently selected from hydrogen, C₁₋₄alkyl and C₁₋₄alkoxy; or R₉ and R₁₀ together with the carbon atom to which R₉ and R₁₀ are attached form C₃₋₁₂cycloalkyl; and R₁₁ is selected from hydrogen and C₁₋₆alkyl; each
R₂ is independently selected from C₁₋₆alkyl, C₂₋₆alkenyl, C₁₋₄alkoxy, C₁₋₄alkylthio, C₃₋₁₂cycloalkyl, C₃₋₈heterocycloalkyl, C₆₋₁₀aryl and C₅₋₁₀heteroaryl; wherein any aryl, heteroaryl, cycloalkyl or heterocycloalkyl of R₂ is optionally substituted with 1 to 3 radicals independently selected from halo, C₁₋₆alkoxy, C₁₋₆alkylthio, halo-substituted-C₁₋₆alkoxy, -C(O)R₁₄ₐ and NR₁₄ₐR_{14b}; wherein R₁₄ₐ and R_{14b} are independently selected from hydrogen and C₁₋₆alkyl;
R₃ and R₄ are independently selected from hydrogen and C₁₋₆alkyl;
R₅ is C₆₋₁₀aryl optionally substituted with 1 to 3 radicals independently selected from halo, nitro, cyano, C₁₋₆alkyl, C₁₋₆alkoxy, C₁₋₆alkylthio, hydroxy-C₁₋₆alkyl, halo-substituted-C₁₋₆alkyl, halo-substituted-C₁₋₆alkoxy, C₃₋₁₂cycloalkyl, C₃₋₈heterocycloalkyl, C₆₋₁₀aryl, C₅₋₁₃heteroaryl and -XNR¹²R¹²; wherein R¹² is selected from hydrogen and C₁₋₆alkyl;
R₆ is selected from hydrogen and methyl; and

R₇ is selected from hydrogen, C₁₋₆alkyl, C₆₋₁₂aryl-C₀₋₄alkyl, C₃₋₁₂cycloalkyl-C₀₋₄alkyl, -XOR₁₄ₐ and -XNR₁₄ₐR_{14b}; wherein X is a bond or C₁₋₄alkylene; and R₁₄ₐ and R_{14b} are independently selected from hydrogen and C₁₋₆alkyl.

In another embodiment, R₁ is selected from -CH₂CR₅R₆CO₂H, - OCR₅R₆CO₂H, -SCR₅R₆CO₂H, -CR₅R₆CH₂CO₂H and -CR₅R₆CO₂H; wherein R₅ and R₆ are independently selected from hydrogen, methyl, methoxy and ethoxy; or R₅ and R₆ together with the carbon atom to which R₅ and R₆ are attached form cyclopentyl.

In another embodiment, each R₂ is independently selected from methyl, ethyl, cyclopropyl, methoxy, furanyl, phenyl, pyridinyl, thienyl, pyrrolidinyl and benzo[1,3]dioxolyl; wherein said pyridinyl or phenyl of R₂ is optionally substituted with 1 to 3 radicals independently selected from halo, methyl-carbonyl, dimethyl-amino, methoxy, halo-substituted-methoxy, methyl-thio, ethenyl, hexenyl and propyloxy.

In another embodiment, R₇ is selected from hydrogen, methyl, isopropyl, propyl, pentyl, isobutyl, methoxy-ethyl, benzyl, phenethyl, cyclohexyl-methyl, cyclobutyl-methyl, cyclopropyl-methyl and diethyl-amino-ethyl.

Preferred compounds of Formula I are selected from: 2-Methyl-2-[2-methyl-4-(2-{methyl-[4-(4-trifluoromethyl-phenyl)-thiazol-2-yl]-amino}-ethoxy)-phenoxy]-propionic acid; 2-Methyl-2-(2-methyl-4-{2-[4-(4-trifluoromethyl-phenyl)-thiazol-2-ylamino]-ethoxy}-phenoxy)-propionic acid; 2-Methyl-2-[2-methyl-4-(2-{propyl-[4-(4-trifluoromethyl-phenyl)-thiazol-2-yl]-amino}-ethoxy)-phenoxy]-propionic acid; 2-Methyl-2-[2-methyl-4-(2-{pentyl-[4-(4-trifluoromethyl-phenyl)-thiazol-2-yl]-amino}-ethoxy)-phenoxy]-propionic acid; 2-[4-(2-{Isopropyl-[4-(4-trifluoromethyl-phenyl)-thiazol-2-yl]-amino}-ethoxy)-2-methyl-phenoxy]-2-methyl-propionic acid; 2-[4-(2-{Isobutyl-[4-(4-trifluoromethyl-phenyl)-thiazol-2-yl]-amino}-ethoxy)-2-methyl-phenoxy]-2-methyl-propionic acid; 2-[4-(2-{(2-Methoxy-ethyl)-[4-(4-trifluoromethyl-phenyl)-thiazol-2-yl]-amino} -ethoxy)-2-methyl-phenoxy]-2-methyl-propionic acid; 2-[4-(2-{Benzyl-[4-(4-trifluoromethyl-phenyl)-thiazol-2-yl]-amino}-ethoxy)-2-methyl-phenoxy]-2-methyl-propionic acid; 2-Methyl-2-[2-methyl-4-(2-{phenethyl-[4-(4-trifluoromethyl-phenyl)-thiazol-2-yl]-amino}-ethoxy)-phenoxy]-propionic acid; 2-[4-(2-{Cyclohexylmethyl-[4-(4-trifluoromethyl-phenyl)-thiazol-2-yl]-amino}-ethoxy)-2-methyl-phenoxy]-2-methyl-propionic acid; 2-[4-(2-{Cyclobutylmethyl-[4-(4-trifluoromethyl-phenyl)-thiazol-2-yl]-amino}-ethoxy)-2-methyl-phenoxy]-2-methyl-propionic acid; 2-[4-(2-{Cyclopropylmethyl-[4-(4-trifluoromethyl-phenyl)-thiazol-2-yl]-amino}-ethoxy)-2-methyl-phenoxy]-2-methyl-propionic acid; 2-[4-(2-{(2-Diethylamino-ethyl)-[4-(4-trifluoromethyl-phenyl)-thiazol-2-yl]-amino}-ethoxy)-2-methyl-phenoxy]-2-methyl-propionic acid; 2-[2,5-Dimethyl-4-(2-{methyl-[4-(4-trifluoromethyl-phenyl)-thiazol-2-yl]-amino}-ethoxy)-phenoxy]-2-methyl-propionic acid; 2-(2,5-Dimethyl-4-{2-[4-(4-trifluoromethyl-phenyl)-thiazol-2-ylamino]-ethoxy}-phenoxy)-2-methyl-propionic acid; 2-[2,5-Dimethyl-4-(3-{methyl-[4-(4-trifluoromethyl-phenyl)-thiazol-2-yl]-amino}-propoxy)-phenoxy]-2-methyl-propionic acid; 2-(2,5-Dimethyl-4-{3-[4-(4-trifluoromethyl-phenyl)-thiazol-2-ylamino]-propoxy}-phenoxy)-2-methyl-propionic acid; 2-[2,5-Dimethyl-4-(4-{methyl-[4-(4-trifluoromethyl-phenyl)-thiazol-2-yl]-amino}-butoxy)-phenoxy]-2-methyl-propionic acid; 2-(2,5-Dimethyl-4-{4-[4-(4-trifluoromethyl-phenyl)-thiazol-2-ylamino]-butoxy}-phenoxy)-2-methyl-propionic acid; 2-(2,5-Dimethyl-4-{2-[4-(4-trifluoromethoxy-phenyl)-thiazol-2-ylamino]-ethoxy}-phenoxy)-2-methyl-propionic acid; 2-[2,5-Dimethyl-4-(2-{methyl-[4-(4-trifluoromethoxyphenyl)-thiazol-2-yl]-amino}-ethoxy)-phenoxy]-2-methyl-propionic acid; 2-(4-{2-[4-(4-Methoxy-phenyl)-thiazol-2-ylamino]-ethoxy}-2,5-dimethyl-phenoxy)-2-methyl-propionic acid; 2-[4-(2-{[4-(4-Methoxy-phenyl)-thiazol-2-yl]-methyl-amino}-ethoxy)-2,5-dimethyl-phenoxy]-2-methyl-propionic acid; 2-(4-{2-[(4-Biphenyl-4-yl-thiazol-2-yl)-methyl-amino]-ethoxy}-2,5-dimethyl-phenoxy)-2-methyl-propionic acid; 2-(2,5-Dimethyl-4-{2-[4-(4-trifluoromethyl-phenyl)-oxazol-2-ylamino]-ethoxy}-phenoxy)-2-methyl-propionic acid; 2-[2,5-Dimethyl-4-(2-{methyl-[4-(4-trifluoromethyl-phenyl)-oxazol-2-yl]-amino}-ethoxy)-phenoxy]-2-methyl-propionic acid; 2-(2,5-Dimethyl-4-{3-[4-(4-tnfluoromethoxy-phenyl)-thiazol-2-ylamino]-propoxy}-phenoxy)-2-methyl-propionic acid; 2-[2,5-Dimethyl-4-(3-{methyl-[4-(4-trifluoromethoxy-phenyl)-thiazol-2-yl]-amino}-propoxy)-phenoxy]-2-methyl-propionic acid; 2-(4-{3-[4-(4-Methoxy-phenyl)-thiazol-2-ylamino]-propoxy}-2,5-dimethyl-phenoxy)-2-methyl-propionic acid; 2-[4-(3-{[4-(4-Methoxy-phenyl)-thiazol-2-yl]-methyl-amino} -propoxy)-2,5-dimethyl-phenoxy]-2-methyl-propionic acid; 2-{4-[3-(4-Biphenyl-4-yl-thiazol-2-ylamino)-propoxy]-2,5-dimethyl-phenoxy}-2-methyl-propionic acid; 2-(4-{3-[(4-Biphenyl-4-yl-thiazol-2-yl)-methyl-amino]-propoxy}-2,5-dimethyl-phenoxy)-2-methyl-propionic acid; 2-(2,5-Dimethyl-4-{3-[4-(4-trifluoromethyl-phenyl)-oxazol-2-ylamino]-propoxy}-phenoxy)-2-methyl-propionic acid; 2-[2,5-Dimethyl-4-(3-{metbyl-[4-(4-trifluoromethyl-phenyl)-oxazol-2-yl]-amino}-propoxy)-phenoxy]-2-methyl-propionic acid; 2-(2,5-Dimethyl-4-{2-[4-(4-trifluoromethyl-phenyl)-thiazol-2-ylamino]-ethylsulfanyl}-phenoxy)-2-methyl-propionic acid; 2-(2,5-Dimethyl-4-{3-[4-(4-trifluoromethyl-phenyl)-thiazol-2ylamino]-propylsulfanyl}phenoxy)2-methyl-propionic acid; 2-[2,5-Dimethyl-4-(2-{methyl-[4-(4-trifluoromethyl-phenyl)-thiazol-2-yl]-amino}-ethylsulfanyl)-phenoxy]-2-methyl-propionic acid; 2-[2,5-Dimethyl-4-(3-{methyl-[4-(4-trifluoromethyl-phenyl)-thiazol-2-yl]-amino}-propylsulfanyl)-phenoxy]-2-methyl-propionic acid; 3-(2,5-Dimethyl-4-{2-[4-(4-trifluoramethyl-phenyl)-thiazol-2-ylamino]-othoxy}-phenyl)-2,2-dimethyl-propionic acid; 3-(2,5-Dimethyl-4-{3-[4-(4-trifluoromethyl-phenyl)-thiszol-2-ylamino]-propoxy}-phenyl)-2,2-dimethyl-propionic acid; 3-[2,5-Dimethyl-4-(2-{methyl-[4-(4-trifluoromethyl-phenyl)-thiazol-2-yl]-amino}-ethoxy)-phenyl]-2,2-dimethyl-propionic acid; 3-[2,5-Dimethyl-4-(3-{methyl-[4-(4-trifluoromethyl-phenyl)-thiazol-2-yl]-amino}-propoxy)-phenyl]-2,2-dimethyl-propionic acid; 2-(2,5-Dimethyl-4-{2-[4-(4-trifluoromethyl-phenyl)-thiazol-2-ylamino]-ethoxy}-phenylsulfanyl)-2-methyl-propionic acid; 2-Methyl-2-(2-methyl-4- {methyl-[4-(4-trifluoromethyl-phenyl)-thiazol-2-yl]-sulfamoyl}-phenoxy)-propionic acid; (2-Methyl-4-{methyl-[4-(4-trifluoromethyl-phenyl)-thiazol-2-yl]-sulfamoyl}-phonoxy)-acetic acid; and 2-(2,5-Dimethyl-4-{methyl-[4-(4-trifluoromethyl-phenyl)-thiazol-2-yl]-sulfamoyl}-phenoxy)-2-methyl-propionic acid.

Further preferred compounds of Formula I are detailed in the Examples, *infra*.

### Pharmacology and Utility

Compounds of the invention modulate the activity of PPARs and, as such, are useful for treating diseases or disorders in which PPARs contributes to the pathology and/or symptomology of the disease. This invention further provides compounds of this invention for use in the preparation of medicaments for the treatment of diseases or disorders in which PPARs contributes to the pathology and/or symptomology of the disease.

Such compounds may therefore be employed for the treatment of prophylaxis, dyslipidemia, hyperlipidemia, hypercholesteremia, atherosclerosis, atherogenesis, hypertriglyceridemia, heart failure, hyper cholesteremia, myocardial infarction, vascular diseases, cardiovascular diseases, hypertension, obesity, cachexia, HIV wasting syndrome, inflammation, arthritis, cancer, Alzheimer's disease, anorexia, anorexia nervosa, bulimia, skin disorders, respiratory diseases, ophthalmic disorders, IBDs (irritable bowel disease), ulcerative colitis and Crohn's disease. Preferably for the treatment of prophylaxis, dyslipidemia, hyperlipidemia, hypercholesteremia, atherosclerosis, atherogenesis, hypertriglyceridemia, cardiovascular diseases, hypertension, obesity, inflammation, cancer, skin disorders, IBDs (irritable bowel disease), ulcerative colitis and Crohn's disease.

Compounds of the invention can also be employed to treat long term critical illness, increase muscle mass and/or muscle strength, increase lean body mass, maintain muscle strength and function in the elderly, enhance muscle endurance and muscle function, and reverse or prevent frailty in the elderly.

Further, the compounds of the present invention may be employed in mammals as hypoglycemic agents for the treatment and prevention of conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome X. Preferably type-1 and type-2 diabetes, Impaired Glucose Metabolism (IGM), Impaired Glucose Tolerance (IGT) and Impaired Fasting Glucose (IFG).

In accordance with the foregoing, described herein is a method for preventing or treating any of the diseases or disorders described above in a subject in need of such treatment, which method comprises administering to said subject a therapeutically effective amount (*See,* "*Administration and Pharmaceutical Compositions*", *infra*) of a compound of the invention or a pharmaceutically acceptable salt thereof. For any of the above uses, the required dosage will vary depending on the mode of administration, the particular condition to be treated and the effect desired. The present invention also concerns: i) a compound of the invention or a pharmaceutically acceptable salt thereof for use as a medicament; and ii) the use of a compound of the invention or a pharmaceutically acceptable salt thereof for the manufacture of a medicament for preventing or treating any of the diseases or disorders described above.

### Administration and Pharmaceutical Compositions

In general, compounds of the invention will be administered in therapeutically effective amounts via any of the usual and acceptable modes known in the art, either singly or in combination with one or more therapeutic agents. A therapeutically effective amount can vary widely depending on the severity of the disease, the age and relative health of the subject, the potency of the compound used and other factors. In general, satisfactory results are indicated to be obtained systemically at daily dosages of from about 0.03 to 2.5mg/kg per body weight. An indicated daily dosage in the larger mammal, e.g. humans, is in the range from about 0.5mg to about 100mg, conveniently administered, e.g. in divided doses up to four times a day or in retard form. Suitable unit dosage forms for oral administration comprise from ca. 1 to 50mg active ingredient.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrollidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions can be sterilized and/or contain adjuvants, such as preserving, stabilizing, wetting or emulsifying agents, solution promoters, salts for regulating the osmotic pressure and/or buffers. In addition, they can also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations can also be used. Suitable formulations for topical application, e.g., to the skin and eyes, are preferably aqueous solutions, ointments, creams or gels well-known in the art. Such can contain solubilizers, stabilizers, tonicity enhancing agents, buffers and preservatives.

This invention also concerns a pharmaceutical composition comprising a therapeutically effective amount of a compound as described herein in combination with one or more pharmaceutically acceptable carriers.

Compounds of the invention can be administered in therapeutically effective amounts in combination with one or more therapeutic agents (pharmaceutical combinations).

Thus, the present invention also relates to pharmaceutical combinations, such as a combined preparation or pharmaceutical composition (fixed combination), comprising: 1) a compound of the invention as defined above or a pharmaceutical acceptable salt thereof; and 2) at least one active ingredient selected from:

a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; DPPIV (dipeptidyl peptidase IV) inhibitors such as DPP728, LAF237 (vildagliptin - Example 1 of WO 00/34241), MK-0431, saxagliptin, GSK23A ; an AGE breaker; a thiazolidone derivative (glitazone) such as pioglitazone, rosiglitazone, or *(R)*-1-{4-[5-methyl-2-(4-trifluoromethyl-phenyl)-oxazol-4-ylmethoxy]-benzenesulfonyl}-2,3-dihydro-1*H*-indole-2-carboxylic acid described in the patent application WO 03/043985, as compound 19 of Example 4, a non-glitazone type PPARγ agonist e.g. GI-262570;

b) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (famesoid X receptor) and LXR (liver X receptor) ligands; cholestyramine; fibrates; nicotinic acid and aspirin;

c) an anti-obesity agent or appetite regulating agent such as phentermine, leptin, bromocriptine, dexamphetamine, amphetamine, fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;

d) anti-hypertensive agents, e.g., loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;

e) a HDL increasing compound;

f) Cholesterol absorption modulator such as Zetia® and KT6-971;

g) Apo-A1 analogues and mimetics;

h) thrombin inhibitors such as Ximelagatran;

i) aldosterone inhibitors such as anastrazole, fadrazole, eplerenone;

j) Inhibitors of platelet aggregation such as aspirin, clopidogrel bisulfate;

k) estrogen, testosterone, a selective estrogen receptor modulator, a selective androgen receptor modulator;

1) a chemotherapeutic agent such as aromatase inhibitors e.g. femara, antiestrogens, topoisomerase I inhibitors, topoisomerase II inhibitors, microtubule active agents, alkylating agents, antineoplastic antimetabolites, platin compounds, compounds decreasing the protein kinase activity such as a PDGF receptor tyrosine kinase inhibitor preferably Imatinib ({N-{5-[4-(4-methyl-piperazino-methyl)-benzoylamido]-2-methylphenyl}-4-(3-pyridyl)-2-pyrimidine-amine }) described in the European patent application EP-A-0 564 409 as example 21 or 4-Methyl-N-[3-(4-methyl-imidazol-1-yl)-5-trifluoromethyl-phenyl]-3-(4-pyridin-3-yl-pyrimidin-2-ylamino)-benzamide described in the patent application WO 04/005281 as example 92; and

m) an agent interacting with a 5-HT₃ receptor and/or an agent interacting with 5-HT₄ receptor such as tegaserod described in the US patent No. 5510353 as example 13, tegaserod hydrogen maleate, cisapride, cilansetron;

or, in each case a pharmaceutically acceptable salt thereof; and optionally a pharmaceutically acceptable carrier.

Most preferred combination partners are tegaserod, imatinib, vildagliptin, metformin, a thiazolidone derivative (glitazone) such as pioglitazone, rosiglitazone, or *(R)*-1-{4-[5-methyl-2-(4-trifluoromethyl-phenyl)-oxazol-4-ylmethoxy]-benzenesulfonyl}-2,3-dihydro-1*H*-indole-2-carboxylic acid, a sulfonylurea receptor ligand, aliskiren, valsartan, orlistat or a statin such as pitavastatin, simvastatin, fluvastatin or pravastatin.

Preferably the pharmaceutical combinations contains a therapeutically effective amount of a compound of the invention as defined above, in a combination with a therapeutically effective amount of another therapeutic agent as described above, e.g., each at an effective therapeutic dose as reported in the art. Combination partners (1) and (2) can be administered together, one after the other or separately in one combined unit dosage form or in two separate unit dosage forms. The unit dosage form may also be a fixed combination.

The structure of the active agents identified by generic or trade names may be taken from the actual edition of the standard compendium "The Merck Index" or the Physician's Desk Reference or from databases, e.g. Patents International (e.g. IMS World Publications) or Current Drugs. The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

In another preferred aspect the invention concerns a pharmaceutical composition (fixed combination) comprising a therapeutically effective amount of a compound as described herein, in combination with a therapeutically effective amount of at least one active ingredient selected from the above described group a) to m), or, in each case a pharmaceutically acceptable salt thereof.

A pharmaceutical composition or combination as described herein for the manufacture of a medicament for the treatment of for the treatment of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

Such therapeutic agents include estrogen, testosterone, a selective estrogen receptor modulator, a selective androgen receptor modulator, insulin, insulin derivatives and mimetics; insulin secretagogues such as the sulfonylureas, e.g., Glipizide and Amaryl; insulinotropic sulfonylurea receptor ligands, such as meglitinides, e.g., nateglinide and repaglinide; insulin sensitizers, such as protein tyrosine phosphatase-1B (PTP-1B) inhibitors, GSK3 (glycogen synthase kinase-3) inhibitors or RXR ligands; biguanides, such as metformin; alpha-glucosidase inhibitors, such as acarbose; GLP-1 (glucagon like peptide-1), GLP-1 analogs, such as Exendin-4, and GLP-1 mimetics; DPPIV (dipeptidyl peptidase IV) inhibitors, e.g. isoleucin-thiazolidide; DPP728 and LAF237, hypolipidemic agents, such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin, fluindostatin and rivastatin, squalene synthase inhibitors or FXR (liver X receptor) and LXR (famesoid X receptor) ligands, cholestyramine, fibrates, nicotinic acid and aspirin. A compound of the present invention may be administered either simultaneously, before or after the other active ingredient, either separately by the same or different route of administration or together in the same pharmaceutical formulation.

The invention also provides for pharmaceutical combinations, e.g. a kit, comprising: a) a first agent which is a compound of the invention as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time. The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g. a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Processes for Making Compounds of the Invention

The present invention also includes processes for the preparation of compounds of the invention. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

Compounds of Formula I, in which R₁ is defined by -X₁CR₉R₁₀X₂CO₂R₁₁ (shown below), X₁SCR₉R₁₀X₂CO₂R₁₁ and -X₁OCR₉R₁₀X₂CO₂R₁₁, wherein R₇ is an alkyl group e.g., methyl or ethyl for a compound of formula 4 converting to hydrogen in formula I, can be prepared by proceeding as in reaction scheme 1:

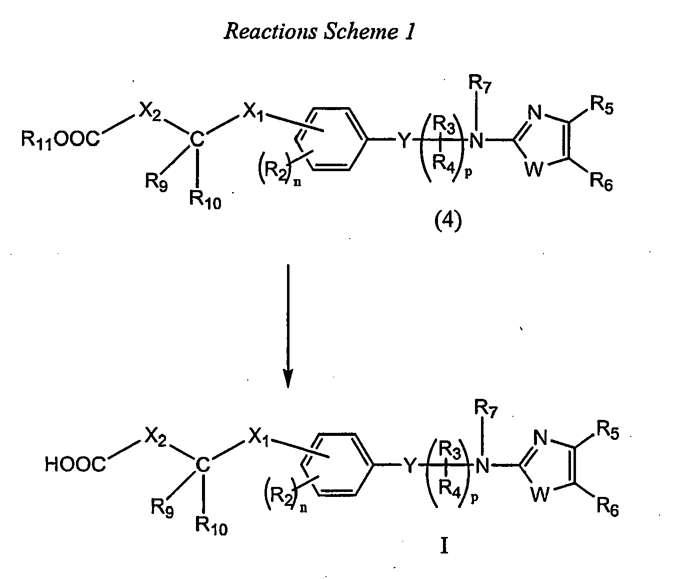

in which n, p, R₁, R₂, R₃, R₄, R₅, R₆, R₇, R₉, R₁₀, X₁, X₂, Y, Z and W are as defined for Formula I. Compounds of Formula I are prepared by reacting a compound of formula 4 in the presence of a suitable base (e.g., lithium hydroxide, or the like) and a suitable solvent (e.g., THF, water or the like). The reaction is carried out in the temperature range of about 0°C to about 50°C and takes up to about 30 hours to complete.

Compounds of Formula 11 can be prepared by proceeding as in reaction scheme 2:

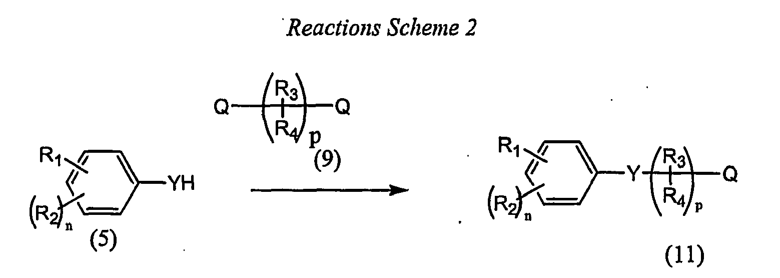

in which n, p, R₁, R₂, R₃, R₄, Y and Z are as defined for Formula I in the Summary of the Invention; and Q is a halogen, preferably C1, I or Br. Compounds of formula 11 are formed by reacting a compound of formula 5 with a compound of formula 9. The reaction proceeds in the presence of a suitable solvent (for example, acetonitrile, acetone, and the like); a suitable inorganic base (for example, Cs₂CO₃, K₂CO₃, and the like). The reaction is carried out in the temperature range of about 10 to about 100°C and takes up to about 24 hours to complete.

Compounds of Formula 14 can be prepared by proceeding as in reaction scheme 3:

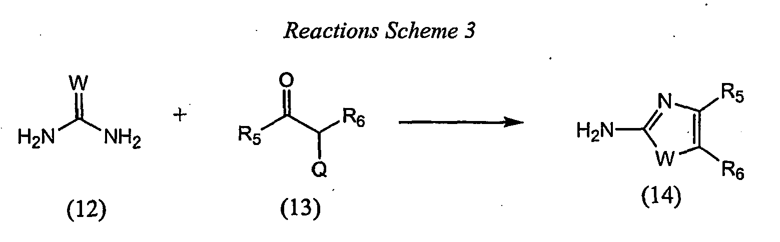

in which R₅, R₆ and W are as defined for Formula I in the Summary of the Invention; and Q is a halogen, preferably C1, I or Br. Compounds of formula 14 are formed by reacting a compound of formula 12 with a compound of formula 13 in the presence of a suitable solvent (for example, acetone, and the like). The reaction is carried out in the temperature range of about 50 to about 80°C and takes up to about 6 hours to complete.

Compounds of Formula I, where R₇ is hydrogen, can be prepared by proceeding as in reaction scheme 4: 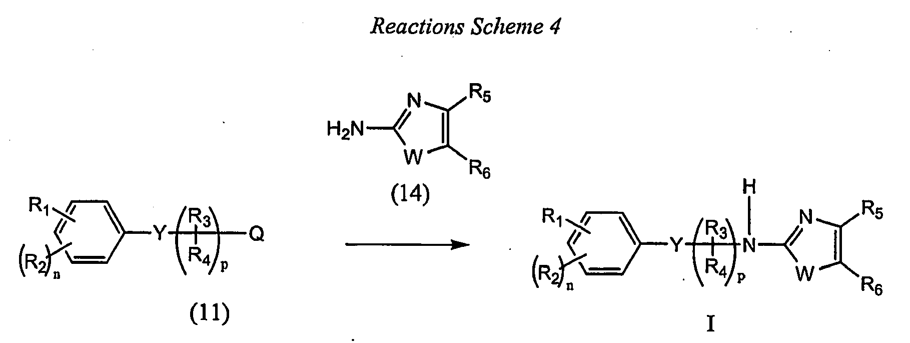 in which n, p, R₁, R₂, R₃, R₄, R₅, R₆, Y and W are as defined for Formula I. Compounds of Formula I are prepared by reacting a compound of 11 with a compound of formula 14 in the presence of a suitable solvent (for example, acetonitrile, and the like) and a suitable inorganic base (for example, K₂CO₃, and the like). The reaction is carried out in the temperature range of about 60 to about 120°C and takes up to about 24 hours to complete.

Compounds of Formula I can be prepared by proceeding as in reaction scheme 5: 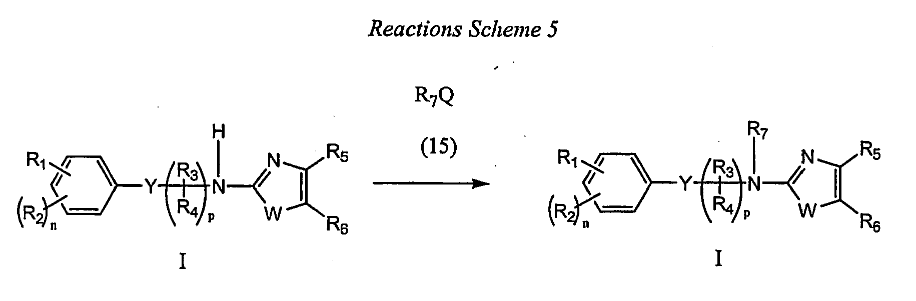 in which n, p, R₁, R₂, R₃, R₄, R₅, R₆, R₇, Y and W are as defined for Formula I; and Q is a halogen, preferably C1, I or Br. Compounds of Formula I are prepared by reacting a compound of formula I (where R₇ is hydrogen) with a compound of formula 15 in the presence of a suitable solvent (for example, acetonitrile, and the like) and a suitable inorganic base (for example, Cs₂CO₃, and the like). The reaction is carried out in the temperature range of about 60 to about 120°C and takes up to about 24 hours to complete.

Compounds of Formula 17, where R₇ is hydrogen, can be prepared by proceeding as in reaction scheme 6: 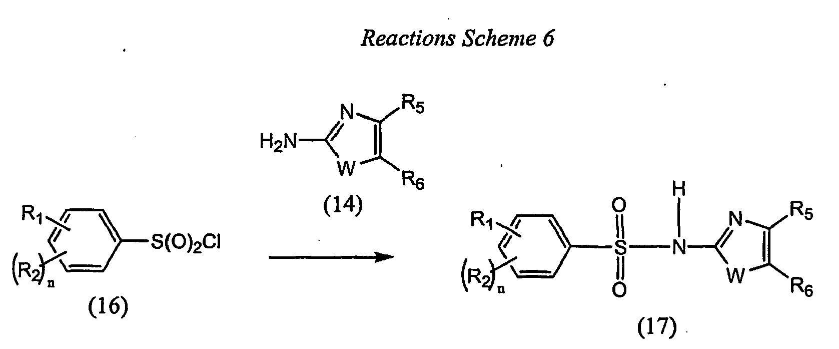 in which n, p, R₁, R₂, R₅, R₆ and W are as defined for Formula I. Compounds of Formula 17 are prepared by reacting a compound of 16 with a compound of formula 14 in the presence of a suitable solvent (for example, DCM, and the like) and a suitable inorganic base (for example, K₂CO₃, and the like) or organic base (for example, triethylamine, and the like). The reaction is carried out in the temperature range of about 0 to about 50°C and takes up to about 24 hours to complete.

Compounds of Formula I can be prepared by proceeding as in reaction scheme 7: 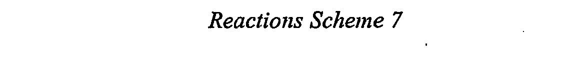 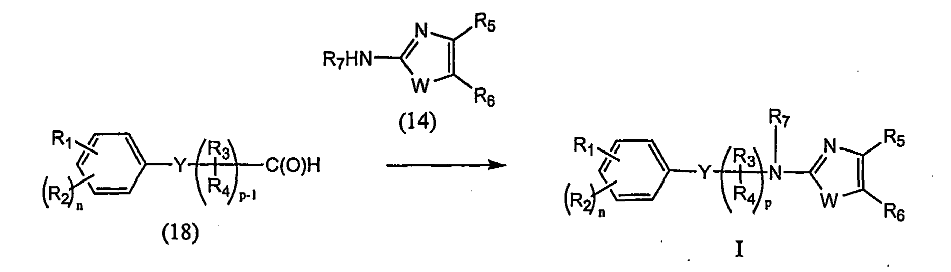 in which n, p, R₁, R₂, R₃, R₄, R₅, R₆, R₇, Y and W are as defined for Formula I. Compounds of Formula I are prepared by reacting a compound of 18 with a compound of formula 14 in the presence of a suitable solvent (for example, THF, and the like) and a suitable dehydrating agent (for example, triethylorthoacetate, and the like) and a suitable reducing agent (for example, sodium triacetoxyborohydride, and the like). The reaction is carried out in the temperature range of about 0 to about 50°C and takes up to about 24 hours to complete.

Detailed reaction conditions are described in the examples, *infra*.

### Additional Processes for Making Compounds of the Invention

A compound of the invention can be prepared as a pharmaceutically acceptable acid addition salt by reacting the free base form of the compound with a pharmaceutically acceptable inorganic or organic acid. Alternatively, a pharmaceutically acceptable base addition salt of a compound of the invention can be prepared by reacting the free acid form of the compound with a pharmaceutically acceptable inorganic or organic base. Alternatively, the salt forms of the compounds of the invention can be prepared using salts of the starting materials or intermediates.

The free acid or free base forms of the compounds of the invention can be prepared from the corresponding base addition salt or acid addition salt from, respectively. For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Protected derivatives of the compounds of the invention can be made by means known to those of ordinary skill in the art. A detailed description of techniques applicable to the creation of protecting groups and their removal can be found in T. W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:

(a) that of reaction schemes 1 through 7; and

(b) optionally converting a compound of the invention into a pharmaceutically acceptable salt;

(c) optionally converting a salt form of a compound of the invention to a non-salt form;

(d) optionally converting an unoxidized form of a compound of the invention into a pharmaceutically acceptable N-oxide;

(e) optionally converting an N-oxide form of a compound of the invention to its unoxidized form;

(f) optionally resolving an individual isomer of a compound of the invention from a mixture of isomers;

(g) optionally converting a non-derivatized compound of the invention into a pharmaceutically acceptable prodrug derivative; and

(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### Examples

The present invention is further exemplified, but not limited, by the following intermediates and examples that illustrate the preparation of compounds of Formula I according to the invention.

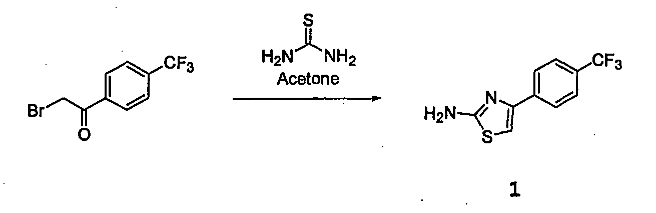

### Intermediate 1: 4-(4-Trifluoromethyl-phonyl)-thiazol-2-ylamine.

2-Bromo-1-(4-trifluoromethyl-phenyl)-ethanone (10 g, 37.4 mmol) and thiourea (2.85 g, 37.4 mmol) are dissolved in dry acetone (100 mL) and heated at reflux for 2 h. The solution is cooled and stirred at rt for 2 h, then filtered and washed with acetone to give 4-(4-trifluoromethyl-phenyl)-thiazol-2-ylamine **1** (9.35 g, 100%) as white crystals. ¹H-NMR (400 MHz, DMSO-d₆) δ = 8.30 (br. s, 2H), 7.98 (d, *J* = 8.0 Hz, 2H), 7.84 (d, *J* = 8.0 Hz, 2H), 7.42 (s, 1H). MS calcd. for C₁₀H₈F₃N₂S (M+H⁺) 245.0, found 245.1.

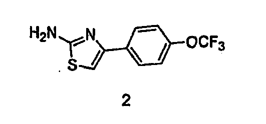

### Intermediate 2: 4-(4-(Trifluoromethoxy)phenyl)thiazol-2-amine.

Following the procedure for Intermediate **1,** except substituting 2-bromo-1-(4-(trifluoromethoxy)phenyl)ethanone for 2-bromo-1-(4-trifluoromethyl-phenyl)-ethanone, the title compound is prepared as a white solid: ¹H-NMR (400 MHz, DMSO-d₆) δ = 7.88 (d, J = 8.8 Hz, 2H), 7.44 (d, *J* = 8.4 Hz, 2H), 7.21 (s, 1H). MS calculated for C₁₀H₈F₃N₂OS (M+H⁺) 261.0, found 261.0.

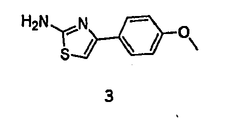

### Intermediate 3: 4-(4-methoxyphenyl)thiazol-2-amine.

Following the procedure for Intermediate **1,** except substituting 2-bromo-1-(4-methoxyphenyl)ethanone for 2-bromo-1-(4-trifluoromethyl-phenyl)-ethanone, the title compound is prepared as a white solid: ¹H-NMR (400 MHz, DMSO-d₆) δ = 7.67 (d, *J* = 8.8 Hz, 2H), 7.06 (s, 1H), 7.05 (d, J = 8.8 Hz, 2H), 3.81 (s, 3H). MS calculated for C₁₀H₁₁N₂OS (M+H⁺) 207.0, found 207.0.

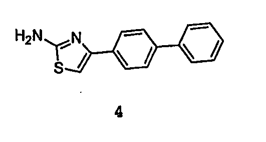

### Intermediate 4: 4-(4-Biphenyl)thiazol-2-amine.

Following the procedure for Intermediate **1,** except substituting 2-bromo-1-(4-biphenyl)ethanone for 2-bromo-1-(4-trifluoromethyl-phenyl)-ethanone, the title compound is prepared as a white solid: ¹H-NMR (400 MHz, DMSO-d₆) δ = 7.84 (d, *J* = 8.8 Hz, 2H), 7.79 (d, *J* = 8.8 Hz, 2H), 7.73(d, *J* = 8.8 Hz, 2H), 7.50 (t, *J* = 7.6 Hz, 2H), 7.40 (t, *J* = 7.6 Hz, 1H), 7.28 (s, 1H). MS calculated for C₁₅H₁₃N₂S (M+H⁺) 253.1, found 253.0.

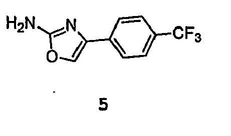

### Intermediate 5: 4-(4-(Trifluoromethyl)phenyl)oxazol-2-amine.

Following the procedure for Intermediate **1**, except substituting urea for thiourea, the title compound is prepared as a white solid: ¹H-NMR (400 MHz, DMSO-d₆) δ = 8.07 (s, 1H), 7.84 (d, *J* = 8.4 Hz, 2H), 7.72 (d, *J* = 8.4 Hz, 2H), 6.85 (s, 2H). MS calculated for C₁₅H₁₃N₂S (M+H⁺) 229.1, found 229.0.

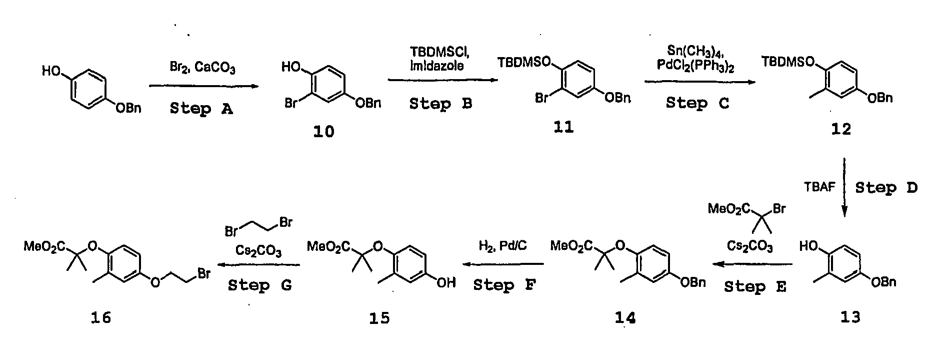

### Intermediate 16: 2-[4-(2-Bromo-ethoxy)-2-methyl-phenoxy]-2-methyl-propionic acid methyl ester.

**Step A:** 4-Benzyloxy-phenol (32.04 g, 160.2 mmol) is dissolved in 550 mL of dichloromethane and 20 mL methanol. Powdered calcium carbonate (21.83 g, 218.1 mmol, 1.36 equiv.) is suspended in the solution. While the suspension is vigorously stirred, a solution of bromine (8.30 mL, 161.5 mmol, 1.01 equiv.) in 50 mL dichloromethane is added dropwise. After the addition is completed, the suspension is stirred at room temperature for 30 min, then the solids are filtered off. The filtrate is dried over solid NaHCO₃ and MgSO₄, then filtered and concentrated to yield an oil. Precipitation of unreacted 4-benzyloxy-phenol using diethyl ether/petroleum ether at -20°C yielded 4-benzyloxy-2-bromo-phenol **10** as a colorless oil that slowly solidified (43.65 g, 156.4 mmol, 97.6%). ¹H-NMR (400 MHz, CDCl₃) δ = 7.38 (m, 5H), 7.10 (d, *J* = 2.8 Hz, 1H), 6.94 (d, *J* = 8.9 Hz, 1H), 6.87 (dd, *J* = 8.9, 2.8 Hz, 1H), 4.99 (s, 2H).
**Step B:** 4-Benzyloxy-2-bromo-phenol **10** (43.6 g, 156.3 mmol) is dissolved in 400 mL dichloromethane. Imidazole (14.9 g, 218.9 mmol, 1.4 equiv.) is added; the mixture is stirred at room temperature until homogenous. *tert*-Butyl dimethylchlorosilane (23.6 g, 156.6 mmol, 1.0 equiv.) is added; the cloudy mixture is stirred at room temperature for 18h. Washing with water, drying over MgSO₄ and concentration yielded (4-benzyloxy-2-bromo-phenoxy)-*tert*-butyl-dimethyl-silane **11** as an oil (60.91 g, 154.8 mmol, 99%). ¹H-NMR (400 MHz, CDCl₃) δ = 7.40 (m, 5H), 7.10 (s, 1H), 6.79 (s, 2H), 4.98 (s, 2H), 1.03 (s, 9H), 0.22 (s, 6H).
**Step C:** (4-Benzyloxy-2-bromo-phenoxy)-*tert*-butyl-dimethyl-silane **11** (10.05 g, 25.6 mmol) is dissolved in 45 mL dimethylformamide. The mixture is degassed using argon. Dichloro bis(triphenylphosphino)palladium(II) (3.49 g, 4.97 mmol, 0.19 equiv.) is added, followed by tetramethyltin (5.0 mL, 36.3 mmol, 1.42 equiv.). The mixture is heated to 100 °C for 3h, after which it became homogenous. Cooling, concentration, and silica gel chromatography purification (0-50% gradient, ethyl acetate in hexanes) yielded (4-benzyloxy-2-methyl-phenoxy)-tert-butyl-dimethyl-silane **12** as an oil that solidifies into a white solid (5.03 g, 15.3 mmol, 60%). ¹H-NMR (400 MHz, CDCl₃) δ = 7.42 (m, 2H), 7.37 (m, 2H), 7.31 (m, 1H), 6.79 (d, *J* = 2.2 Hz, 1H), 6.67 (m, 2H), 4.99 (s, 2H), 2.18 (s, 3H), 1.01 (s, 9H), 0.18 (s, 6H). MS calcd. for C₂₀H₂₉O₂Si (M+H⁺) 329.2, found 329.2.
**Step D:** (4-Benzyloxy-2-methyl-phenoxy)-*tert*-butyl-dimethyl-silane **12** (5.03 g, 15.3 mmol) is dissolved in 30 mL THF. A 1.0 M solution of tetra-(*n*-butyl)ammonium fluoride in THF (18 mL, 18 mmol, 1.5 equiv.) is added; the mixture is stirred at room temperature for 4h. Concentration to dryness and purification by silica gel chromatography (10-30% gradient, ethyl acetate in hexanes) yielded 4-benzyloxy-2-methyl-phenol **13** (3.06 g, 14.3 mmol, 93%). ¹H-NMR (400 MHz, CDCl₃) δ = 7.42 (m, 4H), 7.31 (m, 1H), 6.78 (s, 1H), 6.69 (s, 2H), 4.99 (s, 2H), 2.27 (s, 3H).
**Step E:** 4-benzyloxy-2-methyl-phenol **13** (3.06 g, 14.3 mmol) is dissolved in 60 mL acetonitrile. Powdered cesium carbonate (8.71 g, 26.7 mmol, 1.78 equiv.) is added to the vigorously stirring solution. 2-Bromo-2-methyl-propionic acid methyl ester (2.20 mL, 17.0 mmol, 1.13 equiv.) is added and the mixture is stirred at 60°C for 6h. Filtration and concentration yielded 2-(4-benzyloxy-2-methyl-phenoxy)-2-methyl-propionic acid methyl ester **14** as an oil (5.11 g, quantitative). The crude product is used as such in the next step. ¹H-NMR (400 MHz, CDCl₃) δ = 7.37 (m, 5H), 6.80 (d, *J* = 2.4 Hz, 1H), 6.65 (d, *J* = 2.8 Hz, 1H), 6.64 (s, 1H), 4.98 (s, 2H), 3.80 (s, 3H), 2.21 (s, 3H), 1.54 (s, 6H). MS calcd. for C₁₉H₂₂NaO₄ (M+Na⁺) 337.2, found 337.2.
**Step F:** 2-(4-Benzyloxy-2-methyl-phenoxy)-2-methyl-propionic acid methyl ester **14** from **Step E** above is dissolved in 120 mL ethanol. The solution is degassed using nitrogen, then treated with 5% palladium black on carbon (1.50 g, 0.70 mmol, 4 mol%). The solution is shaken under 60 psi hydrogen for 15h. Filtration and concentration yielded an oil; silica gel chromatography (hexanes to 60% ethyl acetate in hexanes) yields 2-(4-hydroxy-2-methyl-phenoxy)-2-methyl-propionic acid methyl ester **15** as an oil (3.42 g, 15.3 mmol, quantitative). ¹H-NMR (400 MHz, CDCl₃) δ = 6.64 (d, *J* = 3.0 Hz, 1H), 6.59 (d, *J* = 8.7 Hz, 1H), 6.51 (dd, *J* = 8.7, 3.1 Hz, 1H), 4.62 (s, 1H), 3.80 (s, 3H), 2.19 (s, 3H), 1.53 (s, 6H). MS calcd. for C₁₂H₁₆NaO₄ (M+Na⁺) 247.1, found 247.1.
**Step G:** Intermediate **15** (1.0 g, 4.5 mmol), 1,2-dibromoethane (3.8 mL, 44.6 mmol) and Cs₂CO₃ (7.3 g, 22.3 mmol) are suspended in dry acetonitrile (25 mL). The mixture is heated to 80°C overnight. The reaction mixture is cooled to room temperature, filtered and the solvent is removed *in vacuo*. The remainder is purified by flash chromatography (silica, DCM/MeOH gradient) to afford 2-[4-(2-bromo-ethoxy)-2-methyl-phenoxy]-2-methyl-propionic acid methyl ester **16** (0.7 g, 47%) as a colourless oil: MS calculated for C₁₄H₂₀BrO₄ (M+H⁺) 331.1, found 331.0.

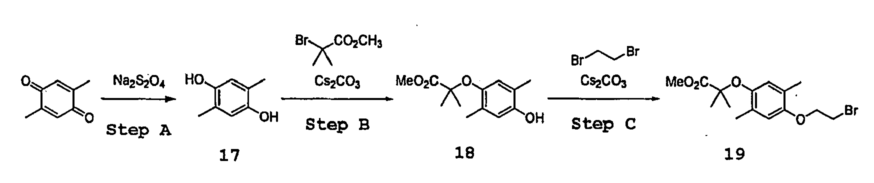

### Intermediate 19: 2-[4-(2-Bromo-ethoxy)-2,5-dimethyl-phenoxy]-2-methyl-propionic acid methyl ester.

**Step A:** 2,5-Dimethylquinone (5.41 g, 39.7 mmol) is suspended in diethyl ether (70 mL). Water (100 mL) is added, followed by solid sodium dithionite (20.30 g, 116.6 mmol). The resulting mixture is shaken vigorously. The initially yellow suspension slowly turned deep red, then colorless. Separation of the organic layer, followed by washing with water and brine, drying over Na₂SO₄ and concentration yielded 2,5-dimethylhydroquinone **17** as a white solid (4.37 g, 31.6 mmol, 80%). ¹H-NMR (400 MHz, DMSO-d₆) δ = 8.32 (s, 2H), 6.45 (s, 2H), 1.99 (s, 6H).
**Step B:** 2,5-Dimethylhydroquinone **17** (3.73 g, 27 mmol) is dissolved in dimethylformamide (20 mL) and acetonitrile (60 mL). Powdered cesium carbonate (9.16 g, 28.1 g, 1.04 equiv.) is added to the vigorously stirring solution, followed by 2-bromo-2-methyl-propionic acid methyl ester (3.50 mL, 27.0 mmol, 1 equiv.). The mixture is stirred at 75°C for 18 h. Filtration and concentration, followed by purification by silica gel chromatography (5-30% gradient, ethyl acetate in hexanes) yielded 2-(4-hydroxy-2,5-dimethyl-phenoxy)-2-methyl-propionic acid methyl ester **18** as and oil (1.92 g, 8.06 mmol, 30%). The chromatography also yielded recovered hydroquinone **17** (1.20 g, 8.68 mmol, 32%). **18:** ¹H-NMR (400 MHz, CDCl₃) δ = 6.57 (s, 1H), 6.50 (s, 1H), 4.44 (s, 1H), 2.15 (s, 3H), 2.14 (s, 3H), 1.52 (s, 6H). MS calcd. for C₁₃H₁₈NaO₄ (M+Na⁺) 261.1, found 261.1.
**Step C:** Intermediate **18** (0.25 g, 1.05 mmol), 1,2-dibromoethane (0.90 mL, 10.5 mmol) and Cs₂CO₃ (1.7 g, 5.25 mmol) are suspended in dry acetonitrile (7 mL). The mixture is heated to 80°C overnight. The reaction mixture is cooled to room temperature, filtered and the solvent is removed in vacuo. The remainder is purified by flash chromatography (silica, DCM/MeOH gradient) to afford 2-[4-(2-bromo-ethoxy)-2-methyl-phenoxy]-2-methyl-propionic acid methyl ester **19** (0.24 g, 66%) as a colourless oil: ¹H-NMR (400 MHz, CDCl₃) δ = 6.59 (s, 1H), 6.52 (s, 1H), 4.22 (t, *J* = 6.2 Hz, 2H), 3.80 (s, 3H), 3.62 (t, *J* = 6.2 Hz, 2H), 2.18 (s, 3H), 2.15 (s, 3H), 1.53 (s, 6H). MS calculated for C₁₅H₂₂BrO₄ (M+H⁺) 345.1, found 345.0.

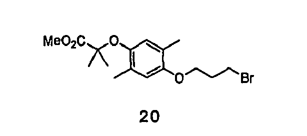

### Intermediate 20: 2-[4-(3-Bromo-propoxy)-2,5-dimethyl-phenoxy]-2-methyl-propionic acid methyl ester.

Following the procedure for Intermediate **19,** except substituting 1,3-dibromopropane for 1,2-dibromoethane, the title compound is prepared as a clear oil: ¹H-NMR (400 MHz, CDCl₃) δ = 6.49 (s, 1H), 6.40 (s, 1H), 3.90 (t, *J* = 5.7 Hz, 2H), 3.68 (s, 3H), 3.49 (t, *J* = 6.5 Hz, 2H), 2.18 (m, 2H), 2.07 (s, 3H), 1.99 (s, 3H), 1.40 (s, 6H). MS calculated for C₁₆H₂₄BrO₄(M+H⁺) 359.1, found 3 59.0.

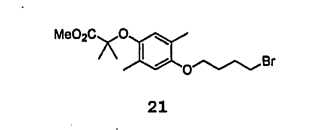

### Intermediate 21: 2-[4-(4-Bromo-butoxy)-2,5-dimethyl-phenoxy]-2-methyl-propionic acid methyl ester.

Following the procedure for Intermediate **19,** except substituting 1,4-dibromobutane for 1,2-dibromoethane, the title compound is prepared as a clear oil: ¹H-NMR (400 MHz, CDCl₃) δ = 6.55 (s, 1H), 6.49 (s, 1H), 3.90 (t, *J* = 6.0 Hz, 2H), 3.78 (s, 3H), 3.47 (t, *J* = 6.6 Hz, 2H), 2.16 (s, 3H), 2.09 (s, 3H), 2.05 (m, 2H), 1.90 (m, 2H), 1.49 (s, 6H). MS calculated for C₁₇H₂₆BrO₄ (M+H⁺) 373.1, found 373.0.

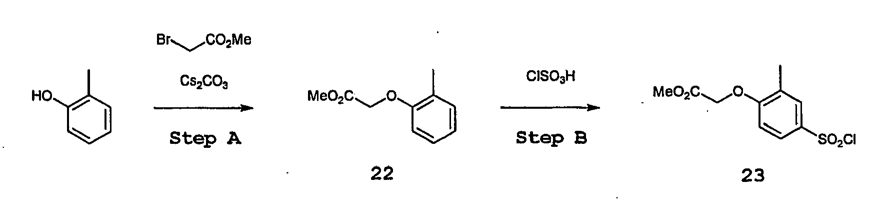

### Intermediate 23: (4-Chlorosulfonyl-2-methyl-phenoxy)-acetic acid methyl ester.

**Step A:** *o*-Cresol (10.0 g, 0.092 mmol) is dissolved in dry DMF (100 mL). Bromoacetic acid methyl ester (15.0 g, 0.098 mmol) and cesium carbonate (40.0 g, 0.123 mmol) are added. The reaction is kept stirring at rt for 3 h. Water is added and the reaction is extracted three times with ethyl acetate. The organic phase is washed with brine and dried with MgSO₄. The solvent is evaporated to give crude product **22.** MS calcd. for C₁₀H₁₃O₃ (M+H⁺) 181.08, found 181.10.
**Step B:** A round bottom flask is charged with o-tolyloxy-acetic acid methyl ester **22** (5.0 g, 27.8 mmol). Chlorosulfonic acid (13.05 g, 112.0 mmol) is added at rt over 5 min. The reaction mixture is poured onto ice, stirred for another 5 min. Then it is filtered, the residue dissolved in DCM and washed with water three times. The organic phase is washed with sat. NaHCO₃ and brine and dried by MgSO₄. The solvent is evaporated. The crude product is purified by silica gel chromatography (ethyl acetate/hexane: 0-30%) to give 23 (4.8 g, 15.6 mmol, yield 78%) as a white solid: ¹H-NMR (400 MHz, CDCl₃) δ = 7.78 (m, 2H), 6.72 (d, *J* = 9.2 Hz, 1H), 4.71 (s, 2H), 3.76 (s, 3H), 2.30 (s, 3H). MS calcd. for C₁₀H₁₁O₅S (M-Cl⁺) 243.0, found 243.0.

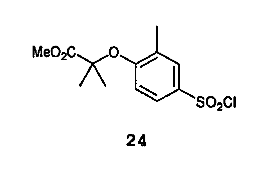

### Intermediate 24: 2-(4-Chlorosulfonyl-2-methyl-phenoxy)-2-methyl-propionic acid methyl ester.

Following the procedure for Intermediate **23,** except substituting 2-bromo-2-methyl-propionic acid methyl ester for bromoacetic acid methyl ester, the title compound is prepared as a white solid: ¹H-NMR (400 MHz, CDCl₃) δ = 7.82 (d, J = 1.6 Hz, 1H), 7.77 (dd, *J* = 2.8 Hz, 8.8 Hz, 1H), 6.64 (d, *J* = 8.8 Hz, 1H), 3.77 (s, 3H), 2.31 (s, 3H), 1.70(s, 6H). MS calcd. for C₁₂H₁₆ClO₅S (M+H⁺) 307.03, found 307.00.

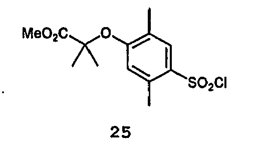

### Intermediate 25: 2-(4-Chlorosulfonyl-2,5-dimethyl-phenoxy)-2-methyl-propionic acid methyl ester.

Following the procedure for Intermediate **23,** except using 2-bromo-2-methyl-propionic acid methyl ester and 2,5-dimethylphenol, the title compound is prepared as a white solid: ¹H-NMR (400 MHz, CDCl₃) δ = 7.51 (s, 1H), 6.31 (s, 1H), 3.71 (s, 3H), 2.40 (s, 3H), 2.10 (s, 3H), 1.51 (s, 6H). MS calcd. for C₁₃H₁₈ClO₅S (M+H⁺) 320.0, found 320.0.

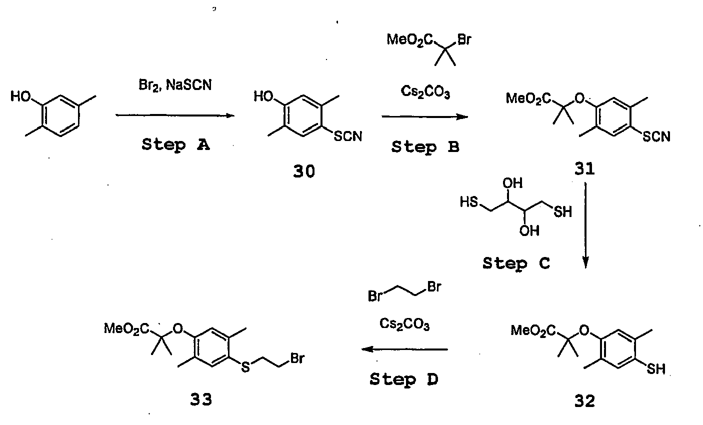

### Intermediate 33: 2-[4-(2-Bromo-ethylsulfanyl)-2,5-dimethyl-phenoxy]-2-methyl-propionic acid methyl ester.

**Step A:** 2,5-Dimethylphenol (10.04 g, 82.2 mmol) is dissolved in methanol (40 mL). Sodium thiocyanate (15.87 g, 195.8 mmol) and sodium bromide (7.37 g, 71.6 mmol) are added and the mixture is stirred at 0°C. Bromine (4.50 mL, 87.6 mmol) dissolved in methanol (40 mL) is added dropwise while stirring vigorously. Upon the completion of the addition, the mixture is stirred at 50°C for 1 h. The mixture is cooled and concentrated. The residue is taken up in ethyl acetate and filtered. The filtrate is washed with saturated aqueous NaHCO₃, water, and brine, dried over Na₂SO₄ and concentrated to afford 2,5-dimethyl-4-thiocyanato-phenol **30** (11.54 g, 78%) as an oil that solidified upon drying under high vacuum: ¹H-NMR (400 MHz, CDCl₃) δ = 7.38 (s, 1H), 6.73 (s, 1H), 5.22 (s, 1H), 2.45 (s, 3H), 2.21 (s, 3H).
**Step B:** 2,5-Dimethyl-4-thiocyanato-phenol **30** (5.75 g, 32.1 mmol) is dissolved in acetonitrile (25 mL). Powdered cesium carbonate (15.32 g, 47.0 mmol) is added. Then 2-bromo-2-methyl-propionic acid methyl ester (4.50 mL, 34.8 mmol) is added and the mixture is stirred at 60°C for 18 h. Filtration and concentration, followed by silica gel chromatography (0-50% ethyl acetate in hexanes) yielded 2-(2,5-dimethyl-4-thiocyanatophenoxy)-2-methyl-propionic acid methyl ester **31** (3.88 g, 43%) as an oil: ¹H-NMR (400 MHz, CDCl₃) (rotamers are present; the data given is for the most abundant isomer) δ = 7.39 (s, 1H), 6.50 (s, 1H), 3.78 (s, 3H), 2.42 (s, 3H), 2.20 (s, 3H), 1.62 (s, 6H). MS calcd. for C₁₄H₁₇NNaO₃S (M+Na⁺) 302.1, found 302.1.
**Step C:** 2-(2,5-dimethyl-4-thiocyanato-phenoxy)-2-methyl-propionic acid methyl ester **31** (3.88 g, 13.9 mmol) is dissolved in methanol (50 mL). Potassium dihydrogenphosphate (0.23 g, 1.69 mmol), water (6 mL), and dithiothreitol (2.80 g, 18.2 mmol) are added and the mixture is stirred at reflux for 3 h. After cooling and concentration, the residue is taken up in ethyl acetate, washed with water and brine, dried over Na₂SO₄ and concentrated to yield an oil. Silica gel chromatography purification (0-65% ethyl acetate in hexanes) afforded 2-(4-mercapto-2,5-dimethyl-phenoxy)-2-methyl-propionic acid methyl ester **32** as a colourless oil (1.92 g, 54%): ¹H-NMR (400 MHz, CDCl₃) δ = 7.09 (s, 1H), 6.47 (s, 1H), 3.79 (s, 1H), 3.10 (s, 1H), 2.24 (s, 3H), 2.15 (s, 3H), 1.56 (s, 6H).
**Step D:** 2-(4-mercapto-2,5-dimethyl-phenoxy)-2-methyl-propionic acid methyl ester **32** (0.51 g, 2.0 mmol) is dissolved in acetonitrile (4 mL), followed by 1,2-dibromoethane (1.7 mL, 20 mmol) and potassium carbonate (0.53 mg, 4.0 mmol). The mixture is stirred at room temperature for 12 h, after which the acetonitrile is evaporated and the remaining solid dissolved in dichloromethane (20 mL) and washed with water. The solvent is removed and the crude oil is purified by preparatory HPLC to afford 2-[4-(2-bromo-ethylsulfanyl)-2,5-dimethyl-phenoxy]-2-methyl-propionic acid methyl ester **33** as a clear oil (0.5 g, 69%) MS calcd. for C₁₅H₂₁BrO₃S (M+H⁺) 361.0, found 361.1

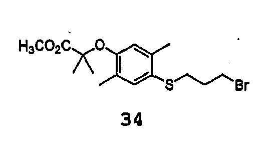

### Intermediate 34: 2-[4-(3-Bromo-propylsulfanyl)-2,5-dimethyl-phenoxy]-2-methyl-propionic acid methyl ester. Following the procedure for Intermediate 33, except substituting 1,3-dibromopropane for 1,2-dibromoethane, the title compound is prepared as a clear oil. (0.6 g, 80%) MS calcd. for C₁₆H₂₃BrO₃S (M+H⁺) 375.1, found 375.1

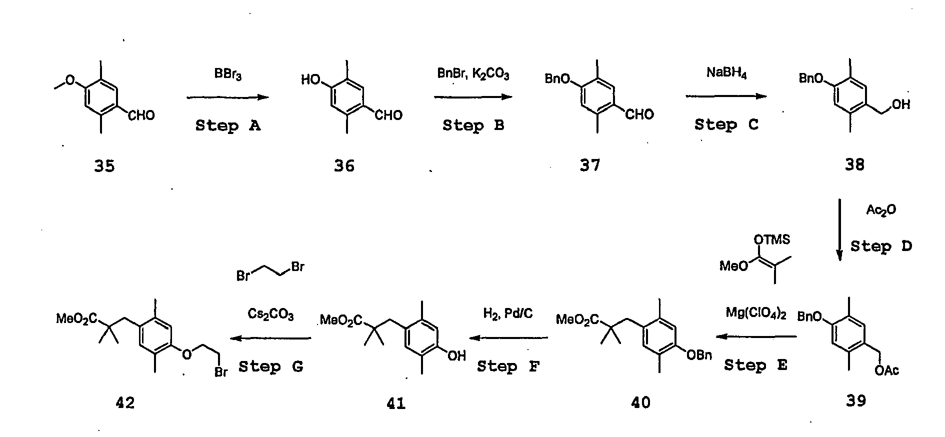

### Intermediate 42: 3-[4-(2-Bromo-ethoxy)-2,5-dimethyl-phenyl]-2,2-dimethyl-propionic acid methyl ester.

**Step A:** 4-Methoxy-2,5-dimethyl-benzaldehyde **35** (1.24 g, 7.55 mmol) is dissolved in dry dichloromethane (12 mL). Neat boron tribromide (1.75 g, 18.5 mmol) is added dropwise, with stirring. A tan-coloured precipitate started to form. The suspension is stirred at room temperature for 5 d. The homogenous mixture is poured over 150 g ice. After the ice melted, the solid phenol **36** is isolated by filtration and dried (1.28 g, quantitative). ¹H-NMR (400 MHz, dmso-d₆) δ = 10.40 (s 1H), 9.98 (s, 1H), 7.54 (s, 1H), 6.68 (s, 1H), 3.36 (s, 1H), 2.49 (s, 3H), 2.13 (s, 3H).
**Step B:** 4-Hydroxy-2,5-dimethyl-benzaldehyde **36** (30.56 g" 0.2 mol) is dissolved in acetonitrile (150 mL). Benzyl bromide (24 mL, 0.2 mol) is added, followed by powdered potassium carbonate (36.92 g, 0.27 mol). The mixture is stirred at 60°C for 18h. Cooling and concentration, followed by silica gel chromatography (0-20% ethyl acetate in hexanes) yielded 4-benzyloxy-2,5-dimethyl-benzaldehyde **37** as a colorless oil (27.6 g, 57%). ¹H-NMR (400 MHz, CDCl₃) δ = 10.13 (s, 1H), 7.61 (s, 1H), 7.43 (m, 5H), 6.72 (s, 1H), 5.15 (s, 2H), 2.63 (s, 3H), 2.28 (s, 3H). MS calcd. for C₁₆H₁₇O₂ (M+H⁺) 241.1, found 241.1.
**Step C:** 4-Benzyloxy-2,5-dimethyl-benzaldehyde **37** (4.77 g" 20 mmol) is dissolved in diethyl ether (30 mL). Sodium borohydride (1.0 g, 27 mmol) is added in one portion, followed by 5 mL absolute ethanol. The mixture is vigorously stirred for 3h at room temperature, then carefully poured over 100 mL 1N aqueous HCl. Extraction with ethyl acetate, washing with water and brine, then concentration yielded (4-benzyloxy-2,5-dimethyl-phenyl)-methanol **38** as a soft solid (4.79 g, 99%). ¹H-NMR (400 MHz, CDCl₃) δ = 7.39 (m, 5H), 7.11 (s, 1H), 6.73 (s, 1H), 5.07 (s, 2H), 4.61 (s, 2H), 2.35 (s, 3H), 2.25 (s, 3H).
**Step D:** (4-Benzyloxy-2,5-dimethyl-phenyl)-methanol **38** (4.79 g, 19.7 mmol) and ethyl diisopropylamine (6.0 mL, 34.4 mmol) are dissolved in dichloromethane (80 mL). Acetic anhydride (2.5 mL, 26.4 mmol) is added in one portion and the mixture is stirred at room temperature for 18h. Washing with 1N HCl, water, saturated aqueous NaHCO₃, saturated aqueous NH₄Cl and brine, followed by drying over MgSO₄ and concentration yields acetic acid 4-benzyloxy-2,5-dimethyl-benzyl ester **39** as an oil (4.93 g, quant.). ¹H-NMR (400 MHz, CDCl₃) δ = 7.39 (m, 5H), 7.11 (s, 1H), 6.73 (s, 1H), 5.07 (s, 2H), 5.04 (s, 2H), 2.32 (s, 3H), 2.24 (s, 3H), 2.07 (s, 3H).
**Step E:** Acetic acid 4-benzyloxy-2,5-dimethyl-benzyl ester **39** (0.56 g, 2 mmol) is dissolved in dry dichloromethane (5 mL). (1-Methoxy-2-methyl-propenyloxy)-trimethylsilane (1 mL, 5 mmol) and magnesium perchlorate (0.09 g, 0.4 mmol) are added and the suspension is stirred overnight. Filtration and silica gel chromatography (0-30% ethyl acetate in hexanes) yielded 3-(4-benzyloxy-2,5-dimethyl-phenyl)-2,2-dimethyl-propionic acid methyl ester **40** as an oil (0.45 g, 69%). ¹H-NMR (400 MHz, CDCl₃) δ = 7.37 (m, 5H), 6.81 (s, 1H), 6.67 (s, 1H), 5.02 (s, 2H), 2.82 (s, 3H), 2.25 (s, 3H), 2.20 (s, 3H), 1.18 (s, 6H).
**Step F:** 3-(4-Benzyloxy-2,5-dimethyl-phenyl)-2,2-dimethyl-propionic acid methyl ester **40** (0.45 g, 1.4 mmol) is dissolved in ethanol (20 mL). Palladium black on carbon (5%; 0.16 g, 5 mol%) is added and the mixture is vigorously stirred under 1 atm. hydrogen for 18h. Filtration and concentration yielded 3-(4-hydroxy-2,5-dimethyl-phenyl)-2,2-dimethyl-propionic acid methyl ester **41** as an oil (0.11 g, 34%). ¹H-NMR (400 MHz, CDCl₃) δ = 6.75 (s, 1H), 6.56 (s, 1H), 3.67 (s, 3H), 2.80 (s, 2H), 2.20 (s, 3H), 2.16 (s, 3H), 1.17 (s, 6H).
**Step G:** 3-(4-hydroxy-2,5-dimethyl-phenyl)-2,2-dimethyl-propionic acid methyl ester **41** (0.47 g, 2.0 mmol) is dissolved in acetonitrile (15 mL), followed by 1,2-dibromoethane (1.7 mL, 20 mmol) and cesium carbonate (3.25 g, 10 mmol). The mixture is stirred at room temperature for 8 h, followed by filtration and silica gel chromatography (0-30% ethyl acetate in hexanes) yielded 3-[4-(2-bromo-ethoxy)-2,5-dimethyl-phenyl]-2,2-dimethyl-propionic acid methyl ester **42** as a clear oil (0.5 g, 69%) MS calcd. for C₁₆H₂₃BrO₃ (M+H⁺) 343.1, found 343.1

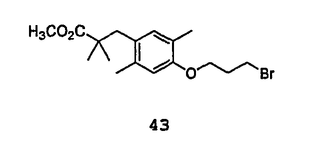

### Intermediate 43: 3-[4-(3-Bromo-propoxy)-2,5-dimethyl-phenyl]-2,2-dimethyl-propionic acid methyl ester Following the procedure for Intermediate 42, except substituting 1,3-dibromopropane for 1,2-dibromoethane, the title compound is prepared as a clear oil. (0.6 g, 84%) MS calcd. for C₁₇H₂₅BrO₃ (M+H⁺) 357.1, found 357.1

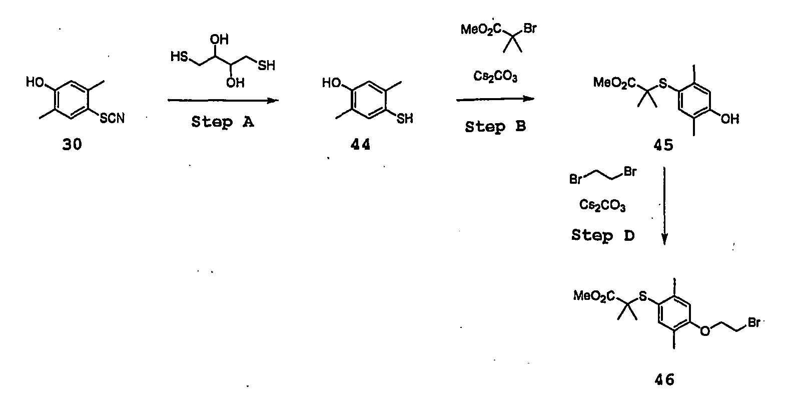

### Intermediate 46: 2-[4-(2-Bromo-ethoxy)-2,5-dimethyl-phenylsulfanyl]-2-methyl-propionic acid methyl ester.

**Step A:** 2,5-Dimethyl-4-thiocyanato-phenol **30** (1.50 g, 8.4 mmol) is dissolved in methanol (30 mL). Potassium dihydrogenphosphate (0.32 g, 2.35 mmol), water (4 mL), and dithiothreitol (2.17 g, 14.1 mmol) are added and the mixture is stirred at reflux for 3 h. After cooling and concentration, the residue is taken up in ethyl acetate, washed with water and brine, dried over Na₂SO₄ and concentrated to yield an oil. It is used as such in the next step: ¹H-NMR (400 MHz, CDCl₃) δ = 7.10 (s, 1H), 6.63 (s, 1H), 4.81 (s, 1H), 3.08 (s, 1H), 2.28 (s, 3H), 2.17 (s, 3H). MS calcd. for C₈H₁₁OS (M+H⁺) 155.1, found 155.0.
**Step B:** 4-Mercapto-2,5-dimethyl-phenol **44** obtained in step A above is dissolved in acetonitrile (30 mL). Powdered cesium carbonate (7.06 g, 21.7 mmol) is added, followed by 2-bromo-2-methyl-propionic acid methyl ester (2.40 mL, 18.5 mmol). The mixture is stirred at rt for 2 h. Filtration and concentration, followed by silica gel chromatography (0-50% ethyl acetate in hexanes) yielded 2-(4-hydroxy-2,5-dimethyl-phenylsulfanyl)-2-methyl-propionic acid methyl ester **45** (0.45 g, 13%) as a white waxy solid: ¹H-NMR (400 MHz, CDCl₃) δ = 7.17 (s, 1H), 6.65 (s, 1H), 5.06 (s, 1H), 3.67 (s, 3H), 2.36 (s, 3H), 2.17 (s, 3H), 1.47 (s, 6H). MS calcd. for C₁₃H₁₉O₃S (M+H⁺) 255.1, found 255.1.
**Step C:** 2-(4-Hydroxy-2,5-dimethyl-phenylsulfanyl)-2-methyl-propionic acid methyl ester **45** (0.25 g, 1.0 mmol) is dissolved in acetonitrile (4 mL), followed by 1,2-dibromoethane (1.7 mL, 20 mmol) and potassium carbonate (0.90 mL, 4.6 mmol). The mixture is stirred at 50°C for 18 h, after which the solids are filtered off and the acetonitrile is evaporated. Silicagel chromatography (10-60% ethyl acetate in hexanes) afforded 2-[4-(2-bromo-ethoxy)-2,5-dimethyl-phenylsulfanyl]-2-methyl-propionic acid methyl ester **46** as a clear oil (0.18 g, 51%): ¹H-NMR (400 MHz, CDCl₃) δ = 7.19 (s, 1H), 6.66 (s, 1H), 4.28 (t, *J* = 6.2 Hz, 2H), 3.67 (s, 3H), 3.64 (t, *J* = 6.2 Hz, 2H), 2.41 (s, 3H), 2.18 (s, 3H), 1.46 (s, 6H). MS calcd. for C₁₅H₂₁BrO₃S (M+H⁺) 361.0, found 361.1.

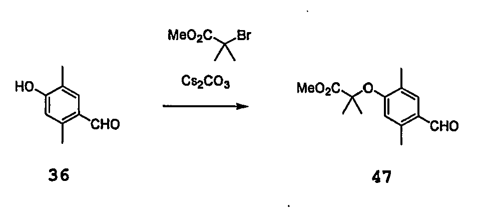

### Intermediate 47: 4-Hydroxy-2,5-dimethyl-benzaldehyde 36 (7.18 g, 47.8 mmol) is dissolved in acetonitrile (60 mL). Powdered cesium carbonate (22.63 g, 69.5 mmol) is added, followed by 2-bromo-2-methyl-propionic acid methyl ester (7.00 mL, 54.1 mmol).

The mixture is stirred at 50°C for 8 h. Filtration and concentration, followed by silica gel chromatography (0-50% ethyl acetate in hexanes) yielded 2-(4-formyl-2,5-dimethyl-phenoxy)-2-methyl-propionic acid methyl ester **47** (3.50 g, 29%) as a white solid: ¹H-NMR (400 MHz, CDCl₃) δ = 10.10 (s, 1H), 7.27 (s, 1H), 6.36 (s, 1H), 3.77 (s, 3H), 2.57 (s, 3H), 2.23 (s, 3H), 1.67 (s, 6H). MS calcd. for C₁₄H₁₉O₄ (M+H⁺) 251.1, found 251.1.

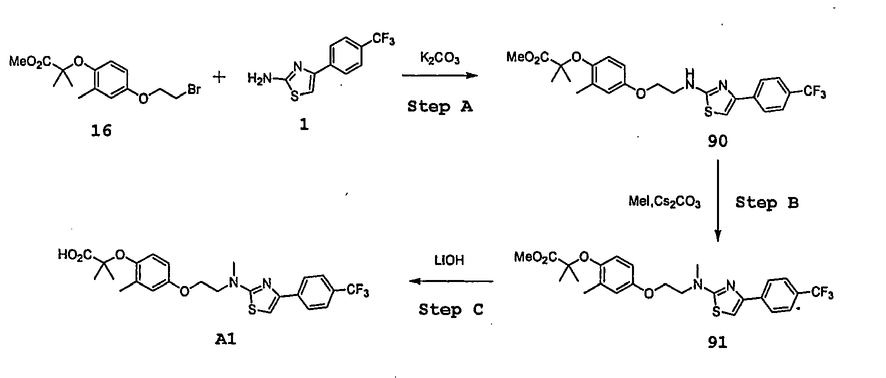

### Example A1: 2-Methyl-2-[2-methyl-4-(2-{methyl-[4-(4-trifluoromethyl-phenyl)-thiazol-2-yl]-amino}-ethoxy)-phenoxy]-propionic acid.

**Step A:** The aminothiazole 1 (0.61 g, 2.49 mmol), the bromide **16** (0.55 g, 1.66 mmol) and potassium carbonate (0.28 g, 1.99 mmol) are suspended in dry acetonitrile (15 mL) in a sealed tube. The mixture is stirred vigorously and heated to 120°C overnight. Then the reaction mixture is cooled to room temperature, filtered and the solvent is removed in vacuo. The remainder is dissolved in ethyl acetate and washed with water twice, the organic layer is dried over MgSO₄ and concentrated. The remainder is purified by flash chromatography (silica, DCM/MeOH gradient) to afford 2-methyl-2-(2-methyl-4-{2-[4-(4-trifluoromethylphenyl)-thiazol-2-ylamino]-ethoxy}-phenoxy)-propionic acid methyl ester **90** (0.35 g, 43%) as a colourless oil: MS calculated for C₂₄H₂₆F₃N₂O₄S (M+H⁺) 495.2, found 495.1.

**Step B:** The 2-Methyl-2-(2-methyl-4-{2-[4-(4-trifluoromethyl-phenyl)-thiazol-2-ylamino]-ethoxy}-phenoxy)-propionic acid methyl ester **90** (50 mg, 0.10 mmol), iodomethane (32 µL, 0.48 mmol) and Cs₂CO₃ (100 mg, 0.30 mmol) are suspended in dry acetonitrile (1 mL) in a sealed tube. The mixture is stirred vigorously and heated to 120°C overnight, cooled to room temperature, and then used directly in the next step.

**Step C:** THF (3 mL) and 1 N LiOH (1 mL) are added to the solution derived from Step B. The mixture is stirred at 50°C for 5 h, then acidified with 1 N HCl (∼1.5 mL). The reaction mixture is extracted with DCM (3 mL), the organic layer is separated and concentrated in vacuo. The remainder is taken up in DMSO (1 mL) and purified on reverse phase HPLC (H₂O/MeCN gradient) to afford the title compound **A1** (26 mg, 53%) as a white solid: ¹H-NMR (600 MHz, (CD₃)₂SO) δ = 7.89 (d, *J* = 8.0 Hz, 2H), 7.63 (d, *J* = 8.0 Hz, 2H), , 6.82-6.63 (m, 4H), 4.24 (t, *J* = 5.1 Hz, 2H), 4.02 (t, *J* = 5.1 Hz, 2H), 3.29 (s, 3H), 2.20 (s, 3H), 1.54 (s, 6H). MS calculated for C₂₄H₂₆F₃N₂O₄S (M+H⁺) 495.2, found 495.1.

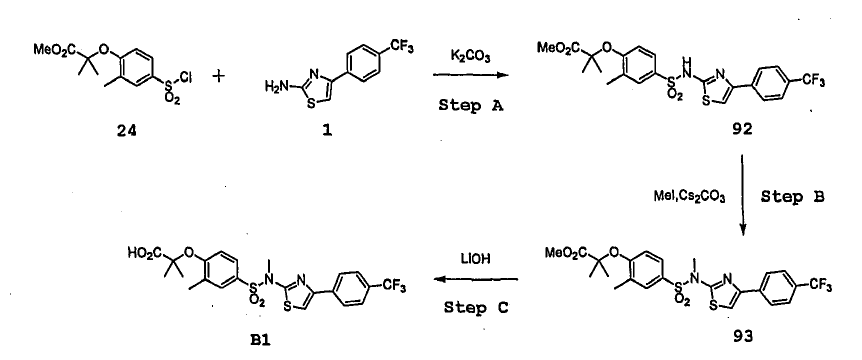

### Example B1: 2-Methyl-2-(2-methyl-4-{methyl-[4-(4-trifluoromethyl-phenyl)-thiazol-2-yl]-sulfamoyl}-phenoxy)-propionic acid.

**Step A:** The aminothiazole **1** (24 mg, 0.10 mmol), the sufonyl chloride **24** (37 mg, 0.12 mmol) and triethylamine (28 µL, 0.20 mmol) are suspended in dry DCM (1 mL) and stirred at rt overnight. Then the reaction mixture is diluted with DCM and washed with water twice, the organic layer is separated, dried over MgSO₄ and concentrated. The remainder is used in the next step without further purification.
**Step B:** The crude 2-methyl-2-{2-methyl-4-[4-(4-trifluoromethyl-phenyl)-thiazol-2-ylsulfamoyl]-phenoxy}-propionic acid methyl ester **92** is dissolved in DMF (1 mL) and cooled to 0°C. Sodium hydride (60% dispersion, 8 mg, 0.11 mmol) is added and the mixture is stirred for 5 min. Then iodomethane (7 µL, 0.11 mmol) is added, and the ice-bath is removed. The mixture is stirred for 6 h at rt and used directly in the next step.
**Step C:** THF (1 mL) and 1 N LiOH (1 mL) are added to the solution derived from Step B. The mixture is stirred at 40°C for 5 h, then acidified with 1 N HCl (∼1.2 mL). The reaction mixture is extracted with DCM (3 mL), the organic layer is separated and concentrated *in vacuo.* The remainder is taken up in DMSO (1 mL) and purified on reverse phase HPLC (H₂O/MeCN gradient) to afford the title compound **B1** (13 mg, 25%) as a colourless glass: ¹H-NMR (600 MHz, (CD₃)₂SO) δ = 7.90 (d, *J* = 8.2 Hz, 2H), 7.66-7.60 (m, 4H), 7.24 (s, 1H), 6.71 (d, *J* = 8.7 Hz, 1H), 3.52 (s, 3H), 2.24 (s, 3H), 1.67 (s, 6H). MS calculated for C₂₂H₂₂F₃N₂O₅S₂ (M+H⁺) 515.1, found 515.0.

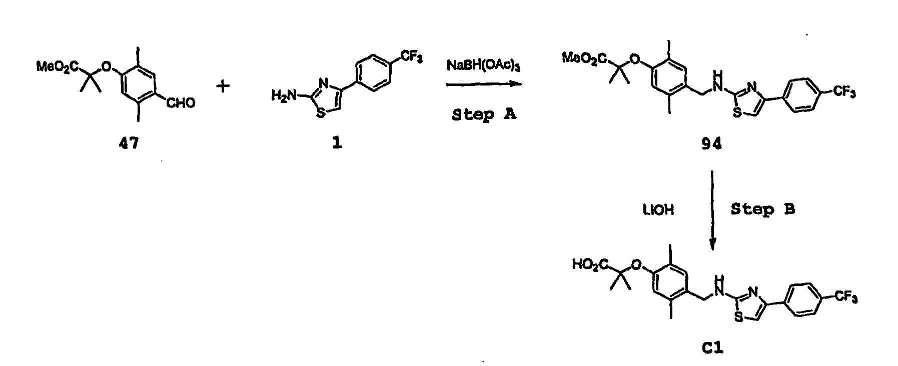

### Reference Example C1: 2-(2,5-Dimethyl-4-{[4-(4-trifluoromethyl-phenyl)-thiazol-2-ylamino]-methyl}-phenoxy)-2-methyl-propionic acid.

**Step A:** The aminothiazole **1** (85 mg, 0.34 mmol) and the aldehyde **47** (91 mg, 0.37 mmol) are dissolved in dry THF (3 mL). Triethylorthoacetate (0.2 mL, 1 mmol) is added, then the mixture is stirred at rt for 30 min. Solid sodium triacetoxyborohydride (0.15 mmol, 0.7 mmol) is added and the mixture is stirred overnight at art The reaction mixture is diluted with 1N HCl and extracted with ethyl acetate twice, the organic layer is washed with brine, dried (MgSO₄) and concentrated. The remainder is used in the next step without further purification.
**Step B:** The crude 2-(2,5-dimethyl-4-{[4-(4-trifluoromethyl-phenyl)-thiazol-2-ylamino]-mefhyl}-phenoxy)-2-methyl-propionic acid methyl ester **94** obtained in step A is dissolved in dimethoxyethane (2 mL). Lithium hydroxide monohydrate (0.10 g) is added, followed by water (0.5 mL). The mixture is vigorously stirred at 50°C for 3 h. Purification on reverse phase HPLC (H₂O/MeCN gradient) afforded the title compound C1 as a colourless glass: ¹H-NMR (400 MHz, CDCl₃) δ = 7.81 (d, *J* = 8.2 Hz, 2H), 7.74 (d, *J* = 8.7 Hz, 1H), 7.13 (s, 1H), 6.68 (s, 1H), 6.63 (s, 1H), 4.44 (s, 2H), 2.32 (s, 3H), 2.22 (s, 3H), 1.63 (s, 6H). MS calculated for C₂₃H₂₄F₃N₂O₃S₂ (M+H⁺) 465.2, found 465.2.

By repeating the procedure described in the above examples A1 and B1, using appropriate starting materials, the following compounds of Formula I, as identified in Table 1, are obtained.

**Table 1**

| **Compound Number** | **Compound Structure** | **Physical Data ¹H NMR 400 MHz and/or MS (m/z)** |
|---|---|---|
| **A2** | 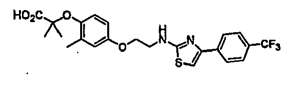 | ¹H-NMR (600 MHz, CDCl₃) δ = 7.81 (d, J = 8.4 Hz, 2H), 7.74 (d, J = 8.4 Hz, 2H), 6.82-6.60 (m, 4H), 4.20 (t, J = 5.0 Hz, 2H), 3.74 (t, J = 5.0 Hz, 2H), 2.21 (s, 3H), 1.54 (s, 6H). MS calculated for C₂₃H₂₄F₃N₂O₄S (M+H⁺) 481.1, found 481.1. |
| **A3** | 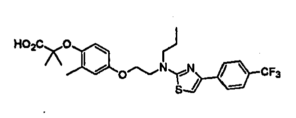 | ¹H-NMR (600 MHz, CDCl₃) δ = 7.85 (d, J = 8.2 Hz, 2H), 7.63 (d, J = 8.2 Hz, 2H), 6.80 (d, J = 8.9 Hz, 1H), 6.75 (s, 1H), 6.72 (d, J = 3.0 Hz, 1H), 6.63 (dd, J = 3.0 Hz, J = 8.9 Hz, 1H), 4.24 (t, J = 5.2 Hz, 2H), 4.03 (t, J = 5.2 Hz, 2H), 3.55 (t, J = 7.7 Hz, 2H), 2.20 (s, 3H), 1.80 (m, 2H), 1.54 (s, 6H), 1.00 (t, J = 7.4 Hz, 3H). MS calculated for C₂₆H₃₀F₃N₂O₄S (M+H⁺) 523.2, found 523.1. |
| **A4** | 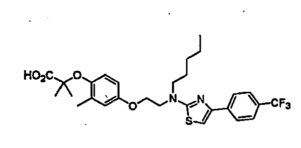 | ¹H-NMR (600 MHz, CDCl₃) δ = 7.87 (d, J = 8.2 Hz, 2H), 7.63 (d, J = 8.2 Hz, 2H), 6.80 (d, J = 8.9 Hz, 1H), 6.75 (s, 1H), 6.72 (d, J = 3.0 Hz, 1H), 6.63 (dd, J = 3.0 Hz, J = 8.9 Hz, 1H), 4.23 (t, J = 5.2 Hz, 2H), 4.03 (t, J = 5.2 Hz, 2H), 3.57 (t, J = 7.7 Hz, 2H), 2.20 (s, 3H), 1.77 (m, 2H), 1.54 (s, 6H), 1.38 (m, 4H), 0.93 (t, J = 7.0 Hz, 3H). MS calculated for C₂₈H₃₄F₃N₂O₄S (M+H⁺) 551.2, found 551.1. |
| **A5** | 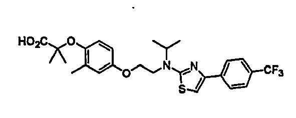 | ¹H-NMR (600 MHz, CDCl₃) δ = 7.89 (d, J = 8.2 Hz, 2H), 7.63 (d, J = 8.2 Hz, 2H), 6.81 (d, J = 8.9 Hz, 1H), 6.80 (s, 1H), 6.75 (d, J = 3.0 Hz, 1H), 6.70 (dd, J = 3.0 Hz, J = 8.9 Hz, 1H), 4.23 (t, J = 6.1 Hz, 2H), 4.09 (m, 1H), 3.87 (t, J = 6.1 Hz, 2H), 2.21 (s, 3H), 1.54 (s, 6H), 1.37 (d, J = 6.6 Hz, 3H). MS calculated for C₂₆H₃₀F₃N₂O₄S (M+H⁺) 523.2, found 523.1. |
| **A6** | 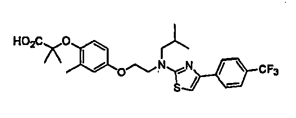 | ¹H-NMR (600 MHz, CDCl₃) δ = 7.85 (d, J = 8.2 Hz, 2H), 7.64 (d, J = 8.2 Hz, 2H), 6.80 (d, J = 8.9 Hz, 1H), 6.75 (s, 1H), 6.71 (d, J = 3.0 Hz, 1H), 6.63 (dd, J = 3.0 Hz, J = 8.9 Hz, 1H), 4.25 (t, J = 5.4 Hz, 2H), 4.05 (t, J = 5.4 Hz, 2H), 3.41 (d, J = 7.6 Hz, 2H), 2.25 (m, 1H), 2.20 (s, 3H), 1.54 (s, 6H), 1.01 (d, J = 6.6 Hz, 3H). MS calculated for C₂₇H₃₂F₃N₂O₄S (M+H⁺) 537.2, found 537.1. |
| **A7** | 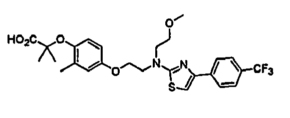 | ¹H-NMR (600 MHz, CDCl₃) δ = 7.86 (d, J = 8.2 Hz, 2H), 7.63 (d, J = 8.2 Hz, 2H), 6.80 (d, J = 8.9 Hz, 1H), 6.77 (s, 1H), 6.72 (d, J = 3.0 Hz, 1H), 6.62 (dd, J = 3.0 Hz, J = 8.9 Hz, 1H), 4.23 (t, J = 5.4 Hz, 2H), 4.03 (t, J = 5.4 Hz, 2H), 3.85 (t, J = 5.4 Hz, 2H), 3.72 (t, J = 5.4 Hz, 2H), 3.37 (s, 3H), 2.20 (s, 3H), |
| | | 1.54 (s, 6H). MS calculated for C₂₇H₃₀F₃N₂O₅S (M+H⁺) 539.2, found 539.1. |
| **A8** | 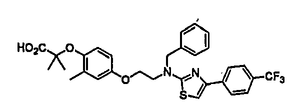 | ¹H-NMR (600 MHz,CDCl₃) δ = 7.91 (d, J = 8.2 Hz, 2H), 7.63 (d, J = 8.2 Hz, 2H), 7.34 (m, 5H), 6.80 (d, J = 8.9 Hz, 1H), 6.80 (s, 1H), 6.70 (d, J = 3.0 Hz, 1H), 6.62 (dd, J = 3.0 Hz, J = 8.9 Hz, 1H), 4.85 (s, 2H), 4.24 (t, J = 5.4 Hz, 2H), 3.99 (t, J = 5.4 Hz, 2H), 2.20 (s, 3H), 1.54 (s, 6H). MS calculated for C₃₀H₃₀F₃N₂O₄S (M+H⁺) 571.2, found 571.1. |
| **A9** | 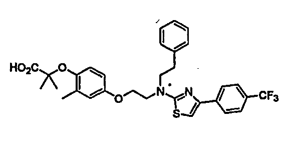 | ¹H-NMR (600 MHz, CDCl₃) δ = 7.90 (d, J = 8.2 Hz, 2H), 7.64 (d, J = 8.2 Hz, 2H), 7.35-7.24 (m, 5H), 6.80 (d, J = 8.9 Hz, 1H), 6.80 (s, 1H), 6.71 (d, J = 3.0 Hz, 1H), 6.63 (dd, J = 3.0 Hz, J = 8.9 Hz, 1H), 4.18 (t, J = 5.2 Hz, 2H), 3.92 (t, J = 5.2 Hz, 2H), 3.82 (t, J = 7.7 Hz, 2H), 3.08 (t, J = 7.7 Hz, 2H), 2.20 (s, 3H), 1.54 (s, 6H). MS calculated for C₃₁H₃₂F₃N₂O₄S (M+H⁺) 585.2, found 585.1. |
| **A10** | 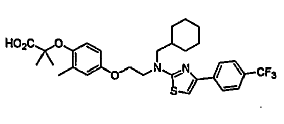 | ¹H-NMR (600 MHz, CDCl₃) δ = 7.85 (d, J = 8.2 Hz, 2H), 7.63 (d, J = 8.2 Hz, 2H), 6.79 (d, J = 8.9 Hz, 1H), 6.74 (s, 1H), 6.71 (d, J = 3.0 Hz, 1H), 6.63 (dd, J = 3.0 Hz, J = 8.9 Hz, 1H), 4.24 (t, J = 5.3 Hz, 2H), 4.04 (t, J = 5.3 Hz, 2H), 3.42 (d, J = 7.4 Hz, 2H), 2.20 (s, 3H), 1.90 (m, 1H), 1.76 (m, 4H), 1.70 (m, 1H), 1.54 (s, 6H), 1.22 (m, 6H), 1.03 (m, 4H). MS calculated for C₃₀H₃₆F₃N₂O₄S (M+H⁺) 577.2, found 577.1. |
| **A11** | 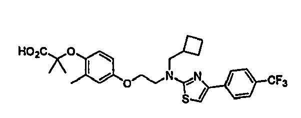 | ¹H-NMR (600 MHz, CDCl₃) δ = 7.85 (d, J = 8.2 Hz, 2H), 7.63 (d, J = 8.2 Hz, 2H), 6.80 (d, J = 8.9 Hz, 1H), 6.74 (s, 1H), 6.72 (d, J = 3.0 Hz, 1H), 6.64 (dd, J = 3.0 Hz, J = 8.9 Hz, 1H), 4.22 (t, J = 5.4 Hz, 2H), 4.00 (t, J = 5.3 Hz, 2H), 3.63 (d, J = 7.3 Hz, 2H), 2.84 (m, 1H), 2.20 (s, 3H), 2.14 (m, 2H), 1.97-1.81 (m, 4H), 1.54 (s, 6H). MS calculated for C₂₈H₃₂F₃N₂O₄S (M+H⁺) 549.2, found 549.2. |
| **A12** | 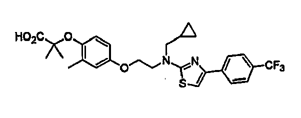 | ¹H-NMR (600 MHz, CDCl₃) δ = 7.89 (d, J = 8.2 Hz, 2H), 7.63 (d, J = 8.2 Hz, 2H), 6.80 (d, J = 8.9 Hz, 1H), 6.78 (s, 1H), 6.72 (d, J = 3.0 Hz, 1H), 6.66 (dd, J = 3.0 Hz, J = 8.9 Hz, 1H), 4.26 (t, J = 5.4 Hz, 2H), 4.09 (t, J = 5.3 Hz, 2H), 3.49 (d, J = 6.8 Hz, 2H), 2.20 (s, 3H), 1.54 (s, 6H), 1.21 (m, 1H), 0.64 (m, 2H), 0.38 (m, 2H). MS calculated for C₂₇H₃₀F₃N₂O₄S (M+H⁺) 535.2, found 535.2. |
| **A13** | 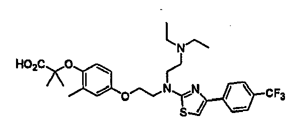 | ¹H-NMR (600 MHz, CDCl₃) δ = 7.86 (d, J = 8.2 Hz, 2H), 7.62 (d, J = 8.2 Hz, 2H), 6.88 (s, 1H), 6.78 (d, J = 8.9 Hz, 1H), 6.69 (d, J = 3.0 Hz, 1H), 6.54 (dd, J = 3.0 Hz, J = 8.9 Hz, 1H), 4.12 (m, 4H), 3.80 (t, J = 4.9 Hz, 2H), 3.41 (m, 2H), 3.23 (q, J = 7.2 Hz, 4H), 2.18 (s, 3H), 1.54 (s, 6H), 1.35 (t, J = 7.2 Hz, 6H). MS calculated for C₂₉H₃₇F₃N₃O₄S (M+H⁺) 580.2, found 580.2. |
| **A14** | 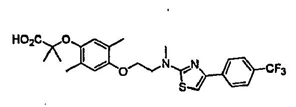 | ¹H-NMR (600 MHz, CDCl₃) δ = 7.93 (d, J = 8.1 Hz, 2H), 7.62 (d, J = 8.1 Hz, 2H), 6.82 (s, 1H), 6.70 (s, 1H), 6.61 (s, 1H), 4.23 (t, J = 5.1 Hz, 2H), 4.01 (t, J = 5.1 Hz, 2H), 3.27 (s, 3H), 2.18 (s, 3H), 2.13 (s, 3H), 1.53 (s, 6H). MS calculated for C₂₅H₂₈F₃N₂O₄S (M+H⁺) 509.2, found 509.1. |
| **A15** | 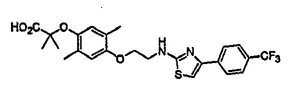 | ¹H-NMR (600 MHz, CDCl₃) δ = 7.80 (d, J = 8.2 Hz, 2H), 7.66 (d, J = 8.2 Hz, 2H), 6.72 (s,1 1H), 6.68 (s, 1H), 6.61 (s, 1H), 4.15 (t, J = 4.9 Hz, 2H), 3.74 (t, J = 4.9 Hz, 2H), 2.19 (s, 3H), 2.03 (s, 3H), 1.54 (s, 6H). MS calculated for C₂₄H₂₆F₃N₂O₄S (M+H⁺) 495.2, found 495.1. |
| **A16** | 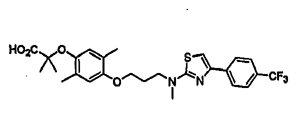 | ¹H-NMR (600 MHz, CDCl₃) δ = 7.90 (d, J = 8.1 Hz, 2H), 7.60 (d, J = 8.1 Hz, 2H), 6.78 (s, 1H), 6.71 (s, 1H), 6.59 (s, 1H), 4.01 (t, J = 4.8 Hz, 2H), 3.78 (t, J = 7.0 Hz, 2H), 3.19 (s, 3H), 2.20 (s, 3H), 2.19 (m, 2H), 2.17 (s, 3H), 1.53 (s, 6H). MS calculated for C₂₆H₃₀F₃N₂O₄S (M+H⁺) 523.2, found 523.1. |
| **A17** | 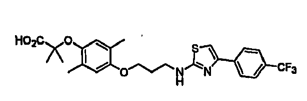 | ¹H-NMR (600 MHz, CDCl₃) δ = 7.84 (d, J = 8.1 Hz, 2H), 7.70 (d, J = 8.1 Hz, 2H), 6.72 (s, 1H), 6.71 (s, 1H), 6.64 (s, 1H), 4.08 (t, J = 5.6 Hz, 2H), 3.58 (m, 2H), 2.26 (m, 2H), 2.20 (s, 3H), 2.18 (m, 2H), 2.18 (s, 3H), 1.54 (s, 6H). MS calculated for C₂₅H₂₈F₃N₂O₄S (M+H⁺) 509.2, found 509.1. |
| **A18** | 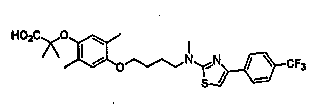 | ¹H-NMR (600 MHz, CDCl₃) δ = 7.91 (d, J = 8.1 Hz, 2H), 7.59 (d, J = 8.1 Hz, 2H), 6.79 (s, 1H), 6.69 (s, 1H), 6.60 (s, 1H), 3.98 (t, J = 5.8 Hz, 2H), 3.64 (t, J = 6.9 Hz, 2H), 3.16 (s, 3H), 2.19 (s, 3H), 2.14 (s, 3H), 1.89 (m, 4H), 1.53 (s, 6H). MS calculated for C₂₆H₃₀F₃N₂O₄S (M+H⁺) 537.2, found 537.1. |
| **A19** | 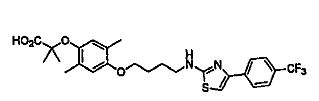 | ¹H-NMR (600 MHz, CDCl₃) δ = 7.78 (d, J = 8.2 Hz, 2H), 7.63 (d, J = 8.2 Hz, 2H), 6.72 (s, 1H), 6.68 (s, 1H), 6.58 (s, 1H), 3.93 (t, J = 5.5 Hz, 2H), 3.34 (t; J = 6.7 Hz, 2H), 2.20 (s, 3H), 2.08 (s, 3H), 1.92 (m, 4H), 1.55 (s, 6H). MS calculated for C₂₆H₃₀F₃N₂O₄S (M+H⁺) 523.2, found 523.1. |
| **A20** | 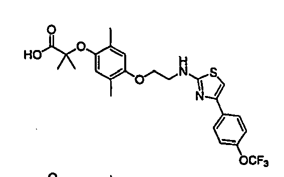 | ¹H-NMR (600 MHz, CDCl₃) δ = 7.73 (d, J = 8.8 Hz, 2H), 7.28 (d, J = 8.4 Hz, 2H), 6.68 (s, 1H), 6.63 (s, 1H), 6.60 (s, 1H), 4.17 (t, J = 5.2 Hz, 2H), 3.75 (t, J = 5.2 Hz, 2H), 2.20 (s, 3H), 2.08 (s, 3H), 1.53 (s, 6H). MS calculated for C₂₄H₂₆F₃N₂O₅S (M+H⁺) 511.1, found 511.2. |
| **A21** | 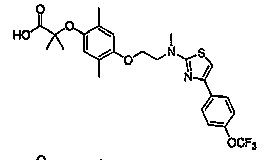 | ¹H-NMR (600 MHz, CDCl₃) δ = 7.78 (d, J = 8.8 Hz, 2H), 7.24 (d, J = 8.4 Hz, 2H), 6.70 (s, 1H), 6.66 (s, 1H), 6.62 (s, 1H), 4.24 (t, J = 5.2 Hz, 2H), 4.11 (t, J = 5.2 Hz, 2H), 3.43 (s, 3H), 2.19 (s, 3H), 2.12 (s, 3H), 1.53 (s, 6H). MS calculated C₂₅H₂₈F₃N₂O₅S (M+H⁺) 525.2, found 525.2. |
| **A22** | 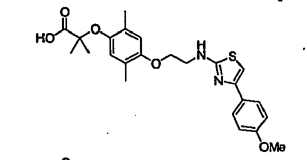 | ¹H-NMR (600 MHz, CDCl₃) δ = 7.62 (d, J = 8.8 Hz, 2H), 6.98 (d, J = 8.8 Hz, 2H), 6.68 (s, 1H), 6.64 (s, 1H), 6.41 (s, 1H), 4.19 (t, J = 5.2 Hz, 2H), 3.85 (s, 3H), 3.74 (t, J = 5.2 Hz, 2H), 2.20 (s, 3H), 2.11 (s, 3H), 1.52 (s, 6H). MS calculated for C₂₄H₂₉N₂O₅S (M+H⁺) 457.2, found 457.2. |
| **A23** | 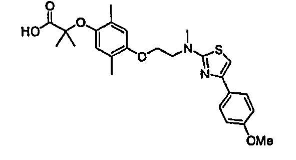 | ¹H-NMR (600 MHz, CDCl₃) δ = 7.68 (d, J = 8.8 Hz, 2H), 6.92 (d, J = 8.8 Hz, 2H), 6.68 (s, 1H), 6.62 (s, 1H), 6.52 (s, 1H), 4.24 (t, J = 4.8 Hz, 2H), 4.13 (t, J = 4.8 Hz, 2H), 3.83 (s, 1H), 3.36 (s, 3H), 2.18 (s, 3H), 2.12 (s, 3H), 1.52 (s, 6H). MS calculated for C₂₅H₃₁N₂O5S (M+H⁺) 471.2, found 471.2. |
| **A24** | 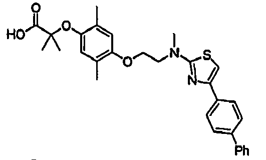 | ¹H-NMR (600 MHz, CDCl₃) δ = 7.84 (d, J = 8.8 Hz, 2H), 7.63 (t, J = 8.4 Hz, 4H), 7.45 (t, J = 7.6 Hz, 2H), 7.36 (t, J = 7.6 Hz, 1H), 6.69 (s, 2H), 6.64 (s, 1H), 4.28 (t, J = 5.2 Hz, 2H), 4.21 (t, J = 5.2 Hz, 2H), 3.39 (s, 3H), 2.19 (s, 3H), 2.13 (s, 3H), 1.52 (s, 6H). MS calculated for C₃₀H₃₃N₂O₄S (M+H⁺) 517.2, found 517.3. |
| **A25** | 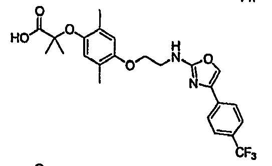 | ¹H-NMR (600 MHz, CDCl₃) δ = 7.70 (s, 4H), 7.52 (s, 1H), 6.68 (s, 1H), 6.60 (s, 1H), 4.10 (t, J = 5.2 Hz, 2H), 3.86 (t, J = 4.8 Hz, 2H), 2.20 (s, 3H), 2.12 (s, 3H), 1.53 (s, 6H). MS calculated for C₂₄H₂₆F₃N₂O₅ (M+H⁺) 479.2, found 479.2. |
| **A26** | 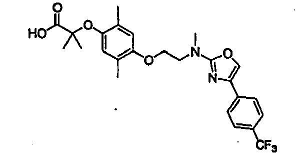 | ¹H-NMR (600 MHz, CDCl₃) δ = 7.78 (d, J = 8.8 Hz, 2H), 7.62 (d, J = 8.0 Hz, 2H), 7.55 (s, 1H), 6.69 (s, 1H), 6.62 (s, 1H), 4.18 (t, J = 5.0 Hz, 2H), 3.94 (t, J = 5.0 Hz, 2H), 3.31 (s, 3H), 2.19 (s, 3H), 2.10 (s, 3H), 1.52 (s, 6H). MS calculated for C₂₅H₂₈F₃N₂O₅ (M+H⁺) 493.2, found 493.2. |
| **A27** | 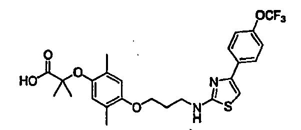 | ¹H-NMR (600 MHz, CDCl₃) δ = 7.73 (d, J = 8.8 Hz, 2H), 7.27 (d, J = 8.4 Hz, 2H), 6.71 (s, 1H), 6.63 (s, 1H), 6.57 (s, 1H), 4.06 (t, J = 5.6 Hz, 2H), 3.54 (t, J = 6.8 Hz, 2H), 2.25 (m, 2H), 2.19 (s, 3H), 2.16 (s, 3H), 1.53 (s, 6H). MS calculated for C₂₅H₂₈F₃N₂O5S (M+H⁺) 525.2, found 525.2. |
| **A28** | 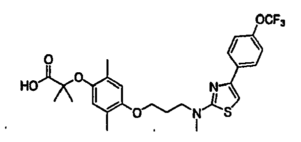 | ¹H-NMR (600 MHz, CDCl₃) δ = 7.75 (d, J = 8.8 Hz, 2H), 7.22 (d, J = 8.0 Hz, 2H), 6.70 (s, 1H), 6.61 (s, 1H), 6.59 (s, 1H), 4.02 (t, J = 5.6 Hz, 2H), 3.82 (t, J = 7.2 Hz, 2H), 3.26 (s, 3H), 2.22 (m, 2H), 2.17 (s, 6H), 1.53 (s, 6H). MS calculated for C₂₆H₃₀F₃N₂O₅S (M+H⁺) 539.2, found 539.2. |
| **A29** | 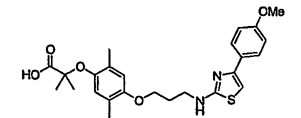 | ¹H-NMR (600 MHz, CDCl₃) δ = 7.61 (d, J = 8.8 Hz, 2H), 6.97 (d, J = 9.2 Hz, 2H), 6.70 (s, 1H), 6.63 (s, 1H), 6.39 (s, 1H), 4.06 (t, J = 5.6 Hz, 2H), 3.84 (s, 3H), 3.52 (t, J = 6.8 Hz, 2H), 2.27 (m, 2H), 2.20 (s, 3H), 2.16 (s, 3H), 1.53 (s, 6H). MS calculated for C₂₅H₃₁N₂O₅S (M+H⁺) 471.2, found 471.2. |
| **A30** | 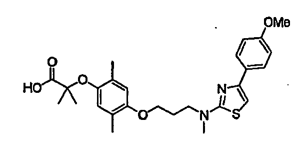 | ¹H-NMR (600 MHz, CDCl₃) δ = 7.65 (d, J = 8.8 Hz, 2H), 6.90 (d, J = 9.2 Hz, 2H), 6.70 (s, 1H), 6.60 (s, 1H), 6.46 (s, 1H), 4.02 (t, J = 5.6 Hz, 2H), 3.83 (m, 5H), 3.28 (s, 3H), 2.23 (m, 2H), 2.18 (s, 6H), 1.53 (s, 6H). MS calculated for C₂₆H₃₃N₂O₅S (M+H⁺) 485.2, found 485.2. |
| **A31** | 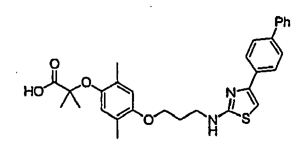 | ¹H-NMR (600 MHz, CDCl₃) δ = 7.76 (d, J = 8.4 Hz, 2H), 7.70 (d, J = 8.4 Hz, 2H), 7.46 (t, J = 7.2 Hz, 2H), 7.38 (t, J = 7.2 Hz, 1H), 6.71 (s, 1H), 6.64 (s, 1H), 6.57 (s, 1H), 4.07 (t, J = 5.6 Hz, 2H), 3.55 (m, 2H), 2.29 (m, 2H), 2.20 (s, 3H), 2.17 (s, 3H), 1.54 (s, 6H). MS calculated for C₃₀H₃₃N₂O₄S (M+H⁺) 517.2, found 517.3. |
| **A32** | 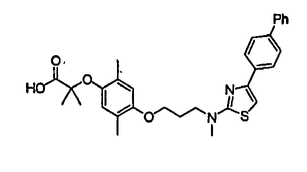 | ¹H-NMR (600 MHz, CDCl₃) δ = 7.76 (d, J = 8.4 Hz, 2H), 7.62 (t, J = 6.8 Hz, 4H), 7.45 (t, J = 7.6 Hz, 2H), 7.36 (t, J = 7.2 Hz, 1H), 6.71 (s, 1H), 6.63 (s, 1H), 6.61 (s, 1H), 4.04 (t, J = 5.6 Hz, 2H), 3.87 (t, J = 6.8 Hz, 2H), 3.33 (s, 3H), 2.25 (m, 2H), 2.18 (s, 6H), 1.53 (s, 6H). MS calculated for C₃₁H₃₅N₂O₄S |
| | | (M+H⁺) 531.2, found 531.2. |
| **A33** | 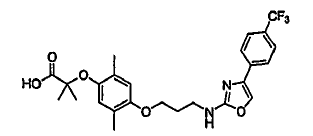 | ¹H-NMR (600 MHz, CDCl₃) δ = 7.69 (s, 4H), 7.47 (s, 1H), 6.70 (s, 1H), 6.62 (s, 1H), 4.05 (t, J = 5.6 Hz, 2H), 3.68 (m, 2H), 2.20 (s, 3H), 2.17 (m, 2H), 2.16 (s, 3H), 1.53 (s, 6H). MS calculated for C₂₅H₂₈F₃N₂O₅ (M+H⁺) 493.2, found 493.2. |
| **A34** | 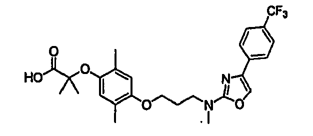 | ¹H-NMR (600 MHz, CDCl₃) δ = 7.77 (d, J = 8.8 Hz, 2H), 7.65 (d, J = 8.8 Hz, 2H), 7.45 (s, 1H), 6.70 (s, 1H), 6.62 (s, 1H), 4.04 (t, J = 5.6 Hz, 2H), 3.83 (m, 2H), 3.28 (s, 3H), 2.18 (s, 3H), 2.16 (s, 3H), 1.53 (s, 6H). MS calculated for C₂₆H₃₀F₃N₂O₅ (M+H⁺) 507.2, found 507.2. |
| **A35** | 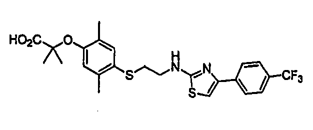 | ¹H-NMR (400 MHz, CDCl₃) δ = 7.76 (d, *J* = 8.4 Hz, 2H), 7.71 (d, *J* = 8.4 Hz, 2H), 7.23 (s, 1H), 6.66 (s, 1H), 6.65 (s, 1H), 3.47 (t, *J* = 6.8 Hz, 2H), 3.07 (t, *J* = 7.2 Hz, 2H), 2.33 (s, 3H), 2.15 (s, 3H), 1.60 (s, 6H). MS calcd. for C₂₄H₂₆F₃N₂O₃S₂ (M+H⁺) 511.1, found 511.2. |
| **A36** | 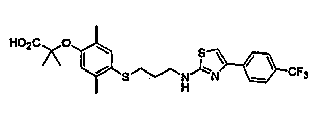 | ¹H-NMR (400 MHz, CDCl₃) δ = 7.89 (d, *J* = 8.4 Hz, 2H), 7.71 (d, *J* = 8.4 Hz, 2H), 7.15 (s, 1H), 6.68 (s, 1H), 6.65 (s, 1H), 3.42 (t, *J* = 6.8 Hz, 2H), 2.94 (t, *J* = 7.2 Hz, 2H), 2.33 (s, 3H), 2.16 (s, 3H), 2.11 (m, 2H), 1.60 (s, 6H). MS calcd. for C₂₅H₂₈F₃N₂O₃S₂ (M+H⁺) 525.1, found 525.2. |
| **A37** | 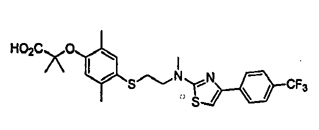 | ¹H-NMR (400 MHz, CDCl₃) δ = 7.75 (d, *J* = 8.0 Hz, 2H), 7.65 (d, *J* = 8.4 Hz, 2H), 7.18 (s, 1H), 6.72 (s, 1H), 6.66 (s, 1H), 3.75 (t, *J* = 6.8 Hz, 2H), 3.12 (t, J = 7.2 Hz, 2H), 3.10 (s, 3H), 2.29 (s, 3H), 2.11 (s, 3H), 1.63 (s, 6H). MS calcd. for C₂₅H₂₈F₃N₂O₃S₂ (M+H⁺) 525.1, found 525.2. |
| **A38** | 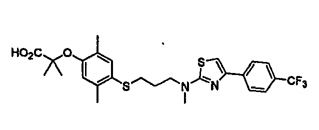 | ¹H-NMR (400 MHz, CDCl₃) δ = 7.78 (d, *J* = 8.4 Hz, 2H), 7.65 (d, *J* = 8.4 Hz, 2H), 7.14 (s, 1H), 6.70 (s, 1H), 6.65 (s, 1H), 3.70 (t, *J* = 6.8 Hz, 2H), 3.23 (s, 3H), 2.86 (t, *J* = 7.2 Hz, 2H), 2.32 (s, 3H), 2.15 (s, 3H), 2.00 (m, 2H), 1.60 (s, 6H). MS calcd. for C₂₆H₃₀F₃N₂O₃S₂ (M+H⁺) 539.1, found 539.2. |
| **A39** | 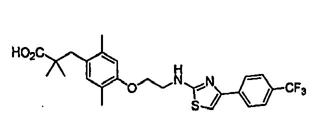 | ¹H-NMR (400 MHz, CDCl₃) δ = 7.75 (d, *J* = 8.0 Hz, 2H), 7.58 (d, *J* = 8.0 Hz, 2H), 6.82 (s, 1H), 6.66 (s, 1H), 6.52 (s, 1H), 4.10 (t, *J* = 5.2 Hz, 2H), 3.67 (t, *J* = 4.8 Hz, 2H), 2.78 (s, 2H), 2.19 (s, 3H), 2.02 (s, 3H), 1.12 (s, 6H). MS calcd. for C₂₅H₂₈F₃N₂O₃S (M+H⁺) 493.2, found 493.2. |
| **A40** | 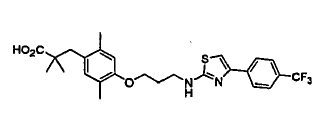 | ¹H-NMR (400 MHz, CDCl₃) δ = 7.81 (d, *J* = 8.0 Hz, 2H), 7.67 (d, *J* = 8.0 Hz, 2H), 6.90 (s, 1H), 6.69 (s, 1H), 6.59 (s, 1H), 4.06 (t, *J* = 5.6 Hz, 2H), 3.52 (t, *J* = 6.8 Hz, 2H), 2.85 (s, 2H), 2.26 (s, 3H), 2.21 (m, 2H), 2.16 (s, 3H), 1.12 (s, 6H). MS calcd. for C₂₆H₃₀F₃N₂O₃S (M+H⁺) 507.2, found 507.3. |
| **A41** | 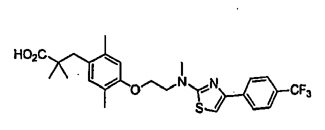 | ¹H-NMR (400 MHz, CDCl₃) δ = 7.91 (d, *J* = 8.0 Hz, 2H), 7.62 (d, *J* = 8.0 Hz, 2H), 6.88 (s, 1H), 6.79 (s, 1H), 6.59 (s, 1H), 4.25 (t, *J* = 5.0 Hz, 2H), 4.05 (t, *J* = 4.8 Hz, 2H), 3.30 (s, 3H), 2.85 (s, 2H), 2.25 (s, 3H), 2.12 (s, 3H), 1.18 (s, 6H). MS calcd. for C₂₆H₃₀F₃N₂O₃S (M+H⁺) 507.2, found 507.2. |
| **A42** | 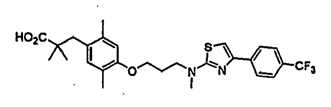 | ¹H-NMR (400 MHz, CDCl₃) δ = 7.79 (d, *J* = 8.4 Hz, 2H), 7.51 (d, *J* = 8.4 Hz, 2H), 6.80 (s, 1H), 6.66 (s, 1H), 6.48 (s, 1H), 3.94 (t, *J* = 5.6 Hz, 2H), 3.69 (t, *J* = 6.8 Hz, 2H), 3.12 (s, 3H), 2.77 (s, 2H), 2.15 (s, |
| | | 3H), 2.11 (m, 2H), 2.09 (s, 3H), 1.10 (s, 6H). MS calcd. for C₂₆H₃₀F₃N₂O₃S (M+H⁺) 521.2, found 521.3. |
| **A43** | 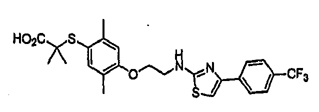 | ¹H-NMR (400 MHz, CDCl₃) δ = 7.81 (d, *J* = 8.2 Hz, 2H), 7.74 (d, *J* = 8.4 Hz, 2H), 7.24 (s, 1H), 6.74 (s, 1H), 6.70 (s, 1H), 4.25 (t, *J* = 6.8 Hz, 2H), 3.78 (t, *J* = 7.2 Hz, 2H), 2.44 (s, 3H), 2.10 (s, 3H), 1.46 (s, 6H). MS calcd. for C₂₄H₂₆F₃N₂O₂S₂ (M+H⁺) 511.1, found 511.1. |
| **B2** | 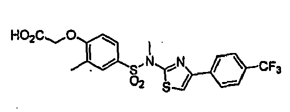 | ¹H-NMR (600 MHz, CDCl₃) δ = 7.90 (d, J = 8.2 Hz, 2H), 7.69-7.61 (m, 5H), 7.24 (s, 1H), 6.75 (d, J = 8.4 Hz, 1H), 4.74 (s, 2H), 3.52 (s, 3H), 2.30 (s, 3H). MS calculated for C₂₀H₁₈F₃N₂O₅S₂ (M+H⁺) 487.1, found 487.0. |
| **B3** | 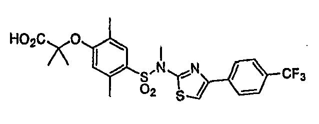 | ¹H-NMR (400 MHz, CDCl₃) δ = 7.84 (d, *J* = 8.4 Hz, 2H), 7.78 (s, 1H), 7.56 (d, *J* = 8.4 Hz, 2H), 7.15 (s, 1H), 6.48 (s, 1H), 3.43 (s, 3H), 2.34 (s, 3H), 2.17 (s, 3H), 1.61 (s, 6H). MS calcd. for C₂₃H₂₄F₃N₂O₅S₂ (M+H⁺) 244.1, found 244.0. |

### Transcriptional Assay

Transfection assays are used to assess the ability of compounds of the invention to modulate the transcriptional activity of the PPARs. Briefly, expression vectors for chimeric proteins containing the DNA binding domain of yeast GAL4 fused to the ligand-binding domain (LBD) of either PPARδ, PPARα or PPARγ are introduced via transient transfection into mammalian cells, together with a reporter plasmid where the luciferase gene is under the control of a GAL4 binding site. Upon exposure to a PPAR modulator, PPAR transcriptional activity varies, and this can be monitored by changes in luciferase levels. If transfected cells are exposed to a PPAR agonist, PPAR-dependent transcriptional activity increases and luciferase levels rise.

293T human embryonic kidney cells (8x10⁶) are seeded in a 175cm² flask a day prior to the start of the experiment in 10% FBS, 1%
Penicillin/Streptomycin/Fungizome, DMEM Media. The cells are harvested by washing with PBS (30ml) and then dissociating using trypsin (0.05%; 3ml). The trypsin is inactivated by the addition of assay media (DMEM, CA-dextran fetal bovine serum (5%). The cells are spun down and resuspended to 170,000cells/ml. A Transfection mixture of GAL4-PPAR LBD expression plasmid (1µg), UAS-luciferase reporter plasmid (1µg), Fugene (3:1 ratio; 6µL) and serum-free media (200µL) was prepared and incubated for 15-40 minutes at room temperature. Transfection mixtures are added to the cells to give 0.16M cells/mL, and cells (50µl/well) are then plated into 384 white, solid-bottom, TC-treated plates. The cells are further incubated at 37°C, 5.0% CO₂ for 5-7 hours. A 12-point series of dilutions (3 fold serial dilutions) are prepared for each test compound in DMSO with a starting compound concentration of 10µM. Test compound (500nl) is added to each well of cells in the assay plate and the cells are incubated at 37°C, 5.0% CO₂ for 18-24 hours. The cell lysis/luciferase assay buffer, Bright-Glo^{™} (25%; 25µl; Promega), is added to each well. After a further incubation for 5 minutes at room temperature, the luciferase activity is measured.

Raw luminescence values are normalized by dividing them by the value of the DMSO control present on each plate. Normalized data is analyzed and dose-response curves are fitted using Prizm graph fitting program. EC50 is defined as the concentration at which the compound elicits a response that is half way between the maximum and minimum values. Relative efficacy (or percent efficacy) is calculated by comparison of the response elicited by the compound with the maximum value obtained for a reference PPAR modulator.

Compounds of Formula I, in free form or in pharmaceutically acceptable salt form, exhibit valuable pharmacological properties, for example, as indicated by the *in vitro* tests described in this application. Compounds of the invention preferably have an EC50 for PPARδ and/or PPARα and/or PPARγ, of less than 5µM, more preferably less than 1µM, more preferably less than 500nm, more preferably less than 100nM. Compounds of the invention preferably have an EC50 for PEARδ that is less than or equal to PPARα which in turn has an EC50 that is at least 10-fold less than PPARγ.

## Claims

1. A compound of Formula I: 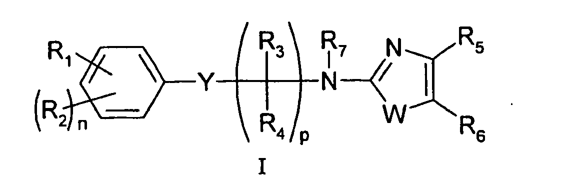 in which
n is selected from 0, 1, 2 and 3;
p is selected from 0, 1, 2 and 3;
Y is selected from O, S(O)₀₋₂, and NR₇ₐ; wherein R₇ₐ is selected from hydrogen and C₁₋₆alkyl;
W is selected from O and S;
R₁ is selected from -X₁CR₉R₁₀X₂CO₂R₁₁, -X₁SCR₉R₁₀X₂CO₂R₁₁ and - X₁OCR₉R₁₀X₂CO₂R₁₁; wherein X₁ and X₂ are independently selected from a bond and C₁₋₄alkylene; and R₉ and R₁₀ are independently selected from hydrogen, C₁₋₄alkyl and C₁₋₄alkoxy; or R₉ and R₁₀ together with the carbon atom to which R₉ and R₁₀ are attached form C₃₋₁₂cycloalkyl; and R₁₁ is selected from hydrogen and C₁₋₆alkyl; each
R₂ is independently selected from halo, C₁₋₆alkyl, C₂₋₆alkenyl, C₁₋₄alkoxy, C₁₋₄alkylthio, C₃₋₁₂cycloalkyl, C₃₋₈heterocycloalkyl, C₆₋₁₀aryl and C₅₋₁₀heteroaryl; wherein any aryl, heteroaryl, cycloalkyl or heterocycloalkyl of R₂ is optionally substituted with 1 to 3 radicals independently selected from halo, C₁₋₆alkyl, C₁₋₆alkoxy, C₂₋₆alkenyl, C₁₋₆alkylthio, halo-substituted-C₁₋₆alkyl, halo-substituted-C₁₋₆alkoxy, -C(O)R₁₄ₐ and NR₁₄ₐR_{14b}; wherein R₁₄ₐ and R_{14b} are independently selected from hydrogen and C₁₋₆alkyl;
R₃ and R₄ are independently selected from hydrogen and C₁₋₆alkyl;
R₅ is C₆₋₁₀aryl optionally substituted with 1 to 3 radicals independently selected from halo, nitro, cyano, C₁₋₆alkyl, C_{1-b}alkoxy, C₁₋₆alkylthio, hydroxy-C₁₋₆alkyl, halo-substituted-C₁₋₆alkyl, halo-substituted-C₁₋₆alkoxy, C₃₋₁₂cycloalkyl, C₃₋₈heterocycloalkyl, C₆₋₁₀aryl, C₅₋₁₃heteroaryl and -XNR¹²R¹²; wherein R¹² is selected from hydrogen and C₁₋₆alkyl;
R₆ is selected from hydrogen and methyl; and
R₇ is selected from hydrogen, C₁₋₆alkyl, C₆₋₁₂aryl-C₀₋₄alkyl, C₃₋₁₂cycloakl-C₀₋₄alkyl, -XOR₁₄ₐ and-XNR₁₄ₐR_{14b}; wherein X is a bond or C₁₋₄alkylene; and R₁₄ₐ and R_{14b} are independently selected from hydrogen and C₁₋₆alkyl;
and the pharmaceutically acceptable salts, hydrates, solvates, and isomers thereof.

2. The compound of claim 1 in which:
n is selected from 0, 1, 2 and 3;
p is selected from 0, 1 and 2;
Y is selected from O and S(O)₀₋₂;
W is selected from O and S;
R₁ is selected from -X₁CR₉R₁₀X₂CO₂R₁₁, -X₁SCR₉R₁₀X₂CO₂R₁₁ and - X₁OCR₉R₁₀X₂CO₂R₁₁; wherein X₁ and X₂ are independently selected from a bond and C₁₋₄alkylene; and R₉ and R₁₀ are independently selected from hydrogen, C₁₋₄alkyl and C₁₋₄alkoxy; or R₉ and R₁₀ together with the carbon atom to which R₉ and R₁₀ are attached form C₃₋₁₂cycloalkyl; and R₁₁ is selected from hydrogen and C₁₋₆alkyl; each
R₂ is independently selected from C₁₋₆alkyl, C₂₋₆alkenyl, C₁₋₄alkoxy, C₁₋₄alkylthio, C₃₋₁₂cycloalkyl, C₃₋₈heterocycloalkyl, C₆₋₁₀aryl and C₅₋₁₀heteroaryl; wherein any aryl, heteroaryl, cycloalkyl or heterocycloalkyl of R₂ is optionally substituted with 1 to 3 radicals independently selected from halo, C₁₋₆alkoxy, C₁₋₆alkylthio, halo-substituted-C₁₋₆alkoxy, -C(O)R₁₄ₐ and NR₁₄ₐR_{14b}; wherein R₁₄ₐ and R_{14b} are independently selected from hydrogen and C₁₋₆alkyl;
R₃ and R₄ are independently selected from hydrogen and C₁₋₆alkyl;
R₅ is C₆₋₁₀aryl optionally substituted with 1 to 3 radicals independently selected from halo, nitro, cyano, C₁₋₆alkyl, C₁₋₆alkoxy, C₁₋₆alkylthio, hydroxy-C₁₋₆alkyl, halo-substituted-C₁₋₆alkyl, halo-substituted-C₁₋₆alkoxy, C₃₋₁₂cycloalkyl, C₃₋₈heterocycloalkyl, C₆₋₁₀aryl, C₅₋₁₃heteroaryl and -XNR¹²R¹²; wherein R¹² is selected from hydrogen and C₁₋₆alkyl;
R₆ is selected from hydrogen and methyl; and
R₇ is selected from hydrogen, C₁₋₆alkyl, C₆₋₁₂aryl-C₀₋₄alkyl, C₃₋₁₂cycloalkyl-C₀₋₄alkyl, -XOR₁₄ₐ and -XNR₁₄ₐR_{14b}; wherein X is a bond or C₁₋₄alkylene; and R₁₄ₐ and R_{14b} are independently selected from hydrogen and C₁₋₆alkyl.

3. The compound of claim 2 in which R₁ is selected from -CH₂CR₅R₆CO₂H, - OCR₅R₆CO₂H, -SCR₅R₆CO₂H, -CR₅R₆CH₂CO₂H and -CR₅R₆CO₂H; wherein R₅ and R₆ are independently selected from hydrogen, methyl, methoxy and ethoxy; or R₅ and R₆ together with the carbon atom to which R₅ and R₆ are attached form cyclopentyl.

4. The compound of claim 3 in which each R₂ is independently selected from methyl, ethyl, cyclopropyl, methoxy, furanyl, phenyl, pyridinyl, thienyl, pyrrolidinyl and benzo[1,3]dioxolyl; wherein said pyridinyl or phenyl of R₂ is optionally substituted with 1 to 3 radicals independently selected from halo, methyl-carbonyl, dimethyl-amino, methoxy, halo-substituted-methoxy, methyl-thio, ethenyl, hexenyl and propyloxy; and R₇ is selected from hydrogen, methyl, isopropyl, propyl, pentyl, isobutyl, methoxy-ethyl, benzyl, phenethyl, cyclohexyl-methyl, cyclobutyl-methyl, cyclopropyl-methyl and diethyl-amino-ethyl.

5. The compound of claim 1 selected from: 2-Methyl-2-[2-methyl-4-(2-{methyl-[4-(4-trifluoromethyl-phenyl)-thiazol-2-yl]-amino}-ethoxy)-phenoxy]-propionic acid; 2-Methyl-2-(2-methyl-4-{2-[4-(4-trifluoromethyl-phenyl)-thiazol-2-ylamino]-ethoxy}-phenoxy)-propionic acid; 2-Methyl-2-[2-methyl-4-(2-{propyl-[4-(4-trifluoromethyl-phenyl)-thiazol-2-yl]-amino}-ethoxy)-phenoxy]-propionic acid; 2-Methyl-2-[2-methyl-4-(2-{pentyl-[4-(4-trifluoromethyl-phenyl)-thiazol-2-yl]-amino}-ethoxy)-phenoxy]-propionic acid; 2-[4-(2-{Isopropyl-[4-(4-trifluoromethyl-phenyl)-thiazol-2-yl]-amino}-ethoxy)-2-methyl-phenoxy]-2-methyl-propionic acid; 2-[4-(2-{Isobutyl-[4-(4-trifluoromethyl-phenyl)-thiazol-2-yl]-amino}-ethoxy)-2-methyl-phenoxy]-2-methyl-propionic acid; 2-[4-(2- {(2-Methoxy-ethyl)-[4-(4-trifluoromethyl-phenyl)-thiazol-2-yl]-amino}-ethoxy)-2-methyl-phenoxy]-2-methyl-propionic acid; 2-[4-(2-{Benzyl-[4-(4-trifluoromethyl-phenyl)-thiazol-2-yl]-amino}-ethoxy)-2-methyl-phenoxy]-2-methyl-propionic acid; 2-Methyl-2-[2-methyl-4-(2-{phenethyl-[4-(4-trifluoromethyl-phenyl)-thiazol-2-yl]-amino}-ethoxy)-phenoxy]-propionic acid; 2-[4-(2-{Cyclohexylmethyl-[4-(4-trifluoromethyl-phenyl)-thiazol-2-yl]-amino}-ethoxy)-2-methyl-phenoxy]-2-methyl-propionic acid; 2-[4-(2-{Cyclobutylmethyl-[4-(4-trifluoromethyl-phenyl)-thiazol-2-yl]-amino}-ethoxy)-2-methyl-phenoxy]-2-methyl-propionic acid; 2-[4-(2-{Cyclopropylmethyl-[4-(4-trifluoromethyl-phenyl)-thiazol-2-yl]-amino}-ethoxy)-2-methyl-phenoxy]-2-methyl-propionic acid; 2-[4-(2-{(2-Dietbylamino-ethyl)-[4-(4-trifluoromethyl-phenyl)-thiazol-2-yl]-amino}-ethoxy)-2-methyl-phenoxy]-2-methyl-propionic acid; 2-[2,5-Dimethyl-4-(2-{methyl-[4-(4-trifluoromethyl-phenyl)-thiazol-2-yl]-amino}-ethoxy)-phenoxy]-2-methyl-propionic acid; 2-(2,5-Dimethyl-4-{2-[4-(4-trifluoromethyl-phenyl)-thiazol-2-ylamino]-ethoxy}-phenoxy)-2-methyl-propiomic acid; 2-[2,5-Dimethyl-4-(3-{methyl-[4-(4-trifluoromethyl-phenyl)-thiazol-2-yl]-ammo}-propoxy)-phenoxy]-2-methyl-propionic acid; 2-(2,5-Dimethyl-4-{3-[4-(4-trifluoromethyl-phenyl)-thiazol-2-ylamino]-propoxy}-phenoxy)-2-methyl-propionic acid; 2-[2,5-Dimethyl-4-(4-{methyl-[4-(4-trifluoromethyl-phenyl)-thiazol-2-yl]-amino}-butoxy)-phenoxy]-2-methyl-propionic acid; 2-(2,5-Dimethyl-4-{4-[4-(4-trifluoromethyl-phenyl)-thiazol-2-ylamino]-butoxy}-phenoxy)-2-methyl-propionic acid; 2-(2,5-Dimethyl-4-{2-[4-(4-trifluoromethoxyphenyl)-thiazol-2-ylamino]-ethoxy}-phenoxy)-2-methyl-propionic acid; 2-[2,5-Dimethyl-4-(2-{methyl-[4-(4-trifluoromethoxy-phenyl)-thiazol-2-yl]-amino}-ethoxy)-phenoxy]-2-methyl-propionic acid; 2-(4-{2-[4-(4-Methoxy-phenyl)-thiazol-2-ylamino]-ethoxy}-2,5-dimethyl-phenoxy)-2-methyl-propionic acid; 2-[4-(2-{[4-(4-Methoxy-phenyl)-thiazol-2-yl]-methyl-amino}-ethoxy)-2,5-dimethyl-phenoxy]-2-methyl-propionic acid; 2-(4-{2-[(4-Biphenyl-4-yl-thiazol-2-yl)-methyl-amino]-ethoxy}-2,5-dimethyl-phenoxy)-2-methyl-propionic acid; 2-(2,5-Dimethyl-4-{2-[4-(4-trifluoromethyl-phenyl)-oxazol-2-ylamino]-ethoxy}-phenoxy)-2-methyl-propionic acid; 2-[2,5-Dimethyl-4-(2-{methyl-[4-(4-trifluoromethyl-phenyl)-oxazol-2-yl]-amino}-ethoxy)-phenoxy]-2-methyl-propionic acid; 2-(2,5-Dimethyl-4-{3-[4-(4-trifluoromethoxy-phenyl)-thiazol-2-ylamino]-propoxy}-phenoxy)-2-methyl-propionic acid; 2-[2,5-Dimethyl-4-(3-{methyl-[4-(4-trifluoramethoxy-phenyl)-thiazol-2-yl]-amino}-propoxy)-phenoxy]-2-methyl-propionic acid; 2-(4-{3-[4-(4-Methoxyphenyl)-thiazol-2-ylamino]-propoxy}-2,5-dimethyl-phenoxy)-2-methyl-propionic acid; 2-[4-(3-{[4-(4-Methoxy-phenyl)-thiazol-2-yl]-methyl-amino}-propoxy)-2,5-dimethyl-phenoxy]-2-methyl-propionic acid; 2-{4-[3-(4-Biphenyl-4-yl-thiazol-2-ylamino)-propoxy]-2,5-dimethyl-phenoxy}-2-methyl-propionic acid; 2-(4-{3-[(4-Biphenyl-4-yl-thiazol-2-yl)-methyl-amino]-propoxy}-2,5-dimethyl-phenoxy)-2-methyl-propionic acid; 2-(2,5-Dimethyl-4-{3-[4-(4-trifluoromethyl-phenyl)-oxazol-2-ylamino]-propoxy}-phenoxy)-2-methyl-propionic acid; 2-[2,5-Dimethyl-4-(3-{methyl-[4-(4-trifluoromethyl-phenyl)-oxazol-2-yl]-amino}-propoxy)-phenoxy]-2-methyl-propionic acid; 2-(2,5-Dimethyl-4-{2-[4-(4-trifluoromethyl-phenyl)-thiazol-2-ylamino]-ethylsulfanyl}-phenoxy)-2-methyl-propionic acid; 2-(2,5-Dimethyl-4-{3-[4-(4-trifluoromethyl-phenyl)-thiazol-2-ylamino]-propylsulfanyl}-phenoxy)-2-methyl-propionic acid; 2-[2,5-Dimethyl-4-(2- {methyl-[4-(4-uifluoromethyl-phenyl)-thiazol-2-yl]-amino}-ethylsulfanyl)-phenoxy]-2-methyl-propionic acid; 2-[2,5-Dimethyl-4-(3-{methyl-[4-(4-trifluoromethyl-phenyl)-thiazol-2-yl]-amino}-propylsulfanyl)-phenoxy]-2-methyl-propionic acid; 3-(2,5-Dimethyl-4-{2-[4-(4-trifluoromethyl-phenyl)-thiazol-2-ylamino]-ethoxy}-phenyl)-2,2-dimethyl-propionic acid; 3-(2,5-Dimethyl-4-{3-[4-(4-trifluoromethyl-phenyl)-thiazol-2-ylamino]-propoxy}-phenyl)-2,2-dimethyl-propionic acid; 3-[2,5-Dimethyl-4-(2-{methyl-[4-(4-trifluoromethyl-phenyl)-thiazol-2-yl]-amino}-ethoxy)-phenyl]-2,2-dimethyl-propionic acid; 3-[2,5-Dimethyl-4-(3-{methyl-[4-(4-trifluoromethyl-phenyl)-thiazol-2-yl]-amino}-propoxy)-phenyl]-2,2-dimethyl-propionic acid; 2-(2,5-Dimethyl-4-{2-[4-(4-trifluommethyl-phenyl)-thiazol-2-ylamino]-ethoxy}-phenylsulfanyl)-2-methyl-propionic acid; 2-Methyl-2-(2-methyl-4-{methyl-[4-(4-trifluoromethyl-phenyl)-thiazol-2-yl]-sulfamoyl}-phenoxy)-propionic acid; (2-Methyl-4-{methyl-[4-(4-trifluoromethyl-phenyl)-thiazol-2-yl]-sulfamoyl}-phenoxy)-acetic acid; and 2-(2,5-Dimethyl-4-{methyl-[4-(4-trifluoromethyl-phenyl)-thiazol-2-yl]-sulfamoyl}-phenoxy)-2-methyl-propionic acid.

6. A compound of any one of claim 1 to 5 for use in a method of treating a disease or disorder in an animal in which modulation of PPAR activity can prevent, inhibit or ameliorate the pathology and/or symptomology of the disease.

7. The compound for use in a method of treatment of claim 6 in which the PPAR activity is at least one PPAR selected from PPARα, PPARδ and PPARγ.

8. The compound for use in a method of treatment of claim 7 in which the PPAR activity is both PPARα and PEARδ.

9. The compound for use in a method of treatment of claim 6 in which the disease or disorder is selected from the treatment or prophylaxis of dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, cachexia, inflammation, arthritis, cancer, anorexia, anorexia nervosa, bulimia, Alzheimer's disease, skin disorders, respiratory diseases, ophthalmic disorders, irritable bowel diseases, ulcerative colitis, Crohn's disease, type-1 diabetes, type-2 diabetes and Syndrome X.

10. The compound for use in a method of treatment of claim 6 in which the disease or disorder is selected from HIV wasting syndrome, long term critical illness, decreased muscle mass and/or muscle strength, decreased lean body mass, maintenance of muscle strength and function in the elderly, diminished muscle endurance and muscle function, and frailty in the elderly.

11. Use of a compound according to any of claims 1 to 5 in the manufacture of a medicament for treating a disease in an animal in which PPAR activity contributes to the pathology and/or symptomology of the disease.

12. The use of claim 11 in which the PPAR activity is at least one PPAR selected from PPARα, PPARδ and PPARγ.

13. The use of claim 12 in which the PPAR activity is both PPARα and PPARδ.

14. A pharmaceutical composition comprising a therapeutically effective amount of a compound of any of claim 1 to 5 in combination with one or more pharmaceutically acceptable excipients.

15. A pharmaceutical combination, especially a pharmaceutical composition, comprising: 1) a compound of any of claims 1 to 5 or a pharmaceutical acceptable salt thereof; and 2) at least one active ingredient selected from:
a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; dipeptidyl peptidase IV inhibitors such as DPP728, vildagliptin, MK-0431, saxagliptin, GSK23A ; an AGE breaker; a thiazolidone derivative (glitazone) such as pioglitazone, rosiglitazone, or *(R)*-1-{4-[5-methyl-2-(4-trifluommethyl-phenyl)-oxazol-4-ylmethoxy]-benzenesulfonyl}-2,3-dihydro-1*H*-indole-2-carboxylic acid, a non-glitazone type PPARγ agonist e.g. GI-262570;
b) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin;
c) an anti-obesity agent or appetite regulating agent such as phentermine, leptin, bromocriptine, dexamphetamine, amphetamine, fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;
d) anti-hypertensive agents, e.g., loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;
e) a HDL increasing compound;
f) a cholesterol absorption modulator such as Zetia® and KT6-971;
g) Apo-Al analogues and mimetics;
h) thrombin inhibitors such as Ximelagatran;
i) aldosterone inhibitors such as anastrazole, fadrazole, eplerenone;
j) Inhibitors of platelet aggregation such as aspirin, clopidogrel bisulfate;
k) estrogen, testosterone, a selective estrogen receptor modulator, a selective androgen receptor modulator;
l) a chemotherapeutic agent such as aromatase inhibitors e.g. femara, anti-estrogen, topoisomerase I inhibitors, topoisomerase II inhibitors, microtubule active agents, alkylating agents, antineoplastic antimetabolites, platin compounds, compounds decreasing the protein kinase activity such as a PDGF receptor tyrosine kinase inhibitor preferably Imatinib or 4-Methyl-N-[3-(4-methyl-imidazol-1-yl)-5-trifluoromethyl-phenyl]-3-(4-pyridin-3-yl-pyrimidin-2-ylamino)-benzamide; and
m) an agent interacting with a 5-HT₃ receptor and/or an agent interacting with 5-HT₄ receptor such as tegaserod, tegaserod hydrogen maleate, cisapride, cilansetron;
or, in each case a pharmaceutically acceptable salt thereof; and optionally a pharmaceutically acceptable carrier.

16. A pharmaceutical composition according to claim 14 or a combination according to claim 15, for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

17. A compound according to any of claims 1 to 5, or a pharmaceutical composition according to claim 10 or a combination according to claim 11, for use as a medicament

18. Use of a compound according to any of claims 1 to 5, or a pharmaceutical composition according to claim 14 or a combination according to claim 15, for the manufacture of a medicament for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGI), Impaired Fasting Glucose (IFG), and Syndrome-X.

## Patentansprüche

1. Verbindung der Formel I: 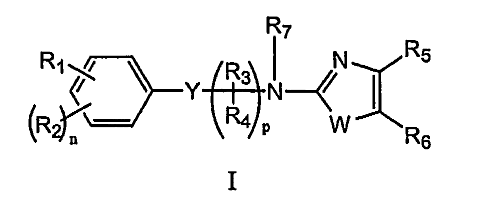 worin
n aus 0, 1, 2 und 3 ausgewählt ist;
p aus 0, 1, 2 und 3 ausgewählt ist;
Y aus O, S(O)₀₋₂ und NR₇ₐ ausgewählt ist; worin R₇ₐ aus Wasserstoff und C₁₋₆-Alkyl ausgewählt ist;
W aus O und S ausgewählt ist;
R₁ aus -X₁CR₉R₁₀X₂CO₂R₁₁, -X₁SCR₉R₁₀X₂CO₂R₁₁ und -X₁OC-R₉R₁₀X₂CO₂R₁₁ ausgewählt ist; worin X₁ und X₂ unabhängig voneinander aus einer Bindung und C₁₋₄-Alkylen ausgewählt sind; und R₉ und R₁₀ unabhängig voneinander aus Wasserstoff, C₁₋₄-Alkyl und C₁₋₄-Alkoxy ausgewählt sind; oder R₉ und R₁₀ zusammen mit dem Kohlenstoffatom, an das R₉ und R₁₀ gebunden sind, C₃₋₁₂-Cycloalkyl bilden; und R₁₁ aus Wasserstoff und C₁₋₆-Alkyl ausgewählt ist;
die R₂ jeweils unabhängig voneinander aus Halogen, C₁₋₆-Alkyl, C₂₋₆-Alkenyl, C₁₋₄-Alkoxy, C₁₋₄-Alkylthio, C₃₋₁₂-Cycloalkyl, C₃₋₈-Heterocycloalkyl, C₆₋₁₀-Aryl und C₅₋₁₀-Heteroaryl ausgewählt ist; worin jedes Aryl, Heteroaryl, Cycloalkyl oder Heterocycloalkyl an R₂ gegebenenfalls mit 1 bis 3 unabhängig voneinander aus Halogen, C₁₋₆-Alkyl, C₁₋₆-Alkoxy, C₂₋₆-Alkenyl, C₁₋₆-Alkylthio, halogensubstituiertem C₁₋₆-Alkyl, halogensubstituiertem C₁₋₆-Alkoxy, -C(O)R₁₄ₐ und NR₁₄ₐR_{14b} ausgewählten Resten substituiert ist; worin R₁₄ₐ und R_{14b} unabhängig voneinander aus Wasserstoff und C₁₋₆-Alkyl ausgewählt sind;
R₃ und R₄ unabhängig voneinander aus Wasserstoff und C₁₋₆-Alkyl ausgewählt sind;
R₅ C₆₋₁₀-Aryl ist, das gegebenenfalls mit 1 bis 3 unabhängig voneinander aus Halogen, Nitro, Cyano, C₁₋₆-Alkyl, C₁₋₆-Alkoxy, C₁₋₆-Alkylthio, Hydroxy-C₁₋₆-alkyl, halogensubstituiertem C₁₋₆-Alkyl, halogensubstituiertem C₁₋₆-Alkoxy, C₃₋₁₂-Cycloalkyl, C₃₋₈-Heterocycloalkyl, C₆₋₁₀-Aryl, C₅₋₁₃-Heteroaryl und -XNR¹²R¹² ausgewählten Resten substituiert ist; worin R¹² aus Wasserstoff und C₁₋₆-Alkyl ausgewählt ist;
R₆ aus Wasserstoff und Methyl ausgewählt ist; und
R₇ aus Wasserstoff, C₁₋₆-Alkyl, C₆₋₁₂-Aryl-C₀₋₄-alkyl, C₃₋₁₂-Cycloalkyl-C₀₋₄-alkyl, -XOR₁₄ₐ und -XNR₁₄ₐR_{14b} ausgewählt ist; worin X eine Bindung oder C₁₋₄-Alkylen ist; und R₁₄ₐ und R_{14b} unabhängig voneinander aus Wasserstoff und C₁₋₆-Alkyl ausgewählt sind;
sowie pharmazeutisch annehmbare Salze, Hydrate, Solvate und Isomere davon.

2. Verbindung nach Anspruch 1, worin:
n aus 0, 1, 2 und 3 ausgewählt ist;
p aus 0, 1 und 2 ausgewählt ist;
Y aus O und S(O)₀₋₂ ausgewählt ist;
W aus O und S ausgewählt ist;
R₁ aus -X₁CR₉R₁₀X₂CO₂R₁₁, -X₁SCR₉R₁₀X₂CO₂R₁₁ und -X₁OCR₉-R₁₀X₂CO₂R₁₁ ausgewählt ist; worin X₁ und X₂ unabhängig voneinander aus einer Bindung und C₁₋₄-Alkylen ausgewählt sind; und R₉ und R₁₀ unabhängig voneinander aus Wasserstoff, C₁₋₄-Alkyl und C₁₋₄-Alkoxy ausgewählt sind; oder R₉ und R₁₀ zusammen mit dem Kohlenstoffatom, an das R₉ und R₁₀ gebunden sind, C₃₋₁₂-Cycloalkyl bilden; und R₁₁ aus Wasserstoff und C₁₋₆-Alkyl ausgewählt ist;
die R₂ jeweils unabhängig voneinander aus C₁₋₆-Alkyl, C₂₋₆-Alkenyl, C₁₋₄-Alkoxy, C₁₋₄-Alkylthio, C₃₋₁₂-Cycloalkyl, C₃₋₈-Heterocycloalkyl, C₆₋₁₀-Aryl und C₅₋₁₀-Heteroaryl ausgewählt sind; worin jedes Aryl, Heteroaryl, Cycloalkyl oder Heterocycloalkyl von R₂ gegebenenfalls mit 1 bis 3 unabhängig voneinander aus Halogen, C₁₋₆-Alkoxy, C₁₋₆-Alkylthio, halogensubstituiertem C₁₋₆-Alkoxy, -C(O)R₁₄ₐ und NR₁₄ₐR_{14b} ausgewählten Resten substituiert ist; worin R₁₄ₐ und R_{14b} unabhängig voneinander aus Wasserstoff und C₁₋₆-Alkyl ausgewählt sind;
R₃ und R₄ unabhängig voneinander aus Wasserstoff und C₁₋₆-Alkyl ausgewählt sind;
R₅ C₆₋₁₀-Aryl ist, das gegebenenfalls mit 1 bis 3 unabhängig voneinander aus Halogen, Nitro, Cyano, C₁₋₆-Alkyl, C₁₋₆-Alkoxy, C₁₋₆-Alkylthio, Hydroxy-C₁₋₆-alkyl, halogensubstituiertem C₁₋₆-Alkyl, halogensubstituiertem C₁₋₆-Alkoxy, C₃₋₁₂-Cycloalkyl, C₃₋₈-Heterocycloalkyl, C₆₋₁₀-Aryl, C₅₋₁₃-Heteroaryl und -XNR¹²R¹² ausgewählten Resten substituiert ist; worin R¹² aus Wasserstoff und C₁₋₆-Alkyl ausgewählt ist;
R₆ aus Wasserstoff und Methyl ausgewählt ist; und
R₇ aus Wasserstoff, C₁₋₆-Alkyl, C₆₋₁₂-Aryl-C₀₋₄-Alkyl, C₃₋₁₂-Cycloalkyl-C₀₋₄-alkyl, -XOR₁₄ₐ und -XNR₁₄ₐR_{14b} ausgewählt ist; worin X eine Bindung oder C₁₋₄-Alkylen ist; und R₁₄ₐ und R_{14b} unabhängig voneinander aus Wasserstoff und C₁₋₆-Alkyl ausgewählt sind.

3. Verbindung nach Anspruch 2, worin R₁ aus -CH₂CR₅R₆CO₂H, -OCR₅R₆CO₂H, -SCR₅R₆CO₂H, -CR₅R₆CH₂CO₂H und -CR₅R₆CO₂H ausgewählt ist; worin R₅ und R₆ unabhängig voneinander aus Wasserstoff, Methyl, Methoxy und Ethoxy ausgewählt sind; oder R₅ und R₆ zusammen mit dem Kohlenstoffatom, an das R₅ und R₆ gebunden sind, Cyclopentyl bilden.

4. Verbindung nach Anspruch 3, worin dir R₂ jeweils unabhängig voneinander aus Methyl, Ethyl, Cyclopropyl, Methoxy, Furanyl, Phenyl, Pyridinyl, Thienyl, Pyrrolidinyl und Benzo[1,3]dioxolyl ausgewählt sind; worin das Pyridinyl oder Phenyl von R₂ gegebenenfalls mit 1 bis 3 unabhängig voneinander aus Halogen, Methylcarbonyl, Dimethylamino, Methoxy, halogensubstituiertem Methoxy, Methylthio, Ethenyl, Hexenyl und Propyloxy ausgewählten Resten substituiert ist; und R₇ aus Wasserstoff, Methyl, Isopropyl, Propyl, Pentyl, Isobutyl, Methoxyethyl, Benzyl, Phenethyl, Cyclohexylmethyl, Cyclobutylmethyl, Cyclopropylmethyl und Diethylaminoethyl ausgewählt ist.

5. Verbindung nach Anspruch 1, ausgewählt aus:
2-Methyl-2-[2-methyl-4-(2-{methyl[4-(4-trifluormethylphenyl)thiazol-2-yl]-amino}ethoxy)phenoxy]propionsäure;
2-Methyl-2-(2-methyl-4-{2-[4-(4-trifluormethylphenyl)thiazol-2-ylamino]ethoxy}-phenoxy)propionsäure;
2-Methyl-2-[2-methyl-4-(2-{propyl-[4-(4-trifluormethylphenyl)thiazol-2-yl]amino}-ethoxy)phenoxy]propionsäure;
2-Methyl-2-[2-methyl-4-(2-{pentyl-[4-(4-trifluormethylphenyl)thiazol-2-yl]amino}-ethoxy)phenoxy]propionsäure;
2-[4-(2-{Isopropyl-[4-(4-trifluormethylphenyl)thiazol-2-yl]amino}ethoxy)-2-methyl-phenoxy]-2-methylpropionsäure;
2-[4-(2-{Isobutyl-[4-(4-trifluormethylphenyl)thiazol-2-yl]amino}ethoxy)-2-methyl-phenoxy]-2-methylpropionsäure;
2-[4-(2-{(2-Methoxyethyl)-[4-(4-trifluormethylphenyl)thiazol-2-yl]amino}ethoxy)-2-methylphenoxy]-2-methylpropionsäure;
2-[4-(2-{Benzyl-[4-(4-trifluormethylphenyl)thiazol-2-yl]amino}ethoxy)-2-methyl-phenoxy]-2-methylpropionsäure;
2-Methyl-2-[2-methyl-4-(2-{phenethyl-[4-(4-trifluormethylphenyl)thiazol-2-yl]amino}-ethoxy)phenoxy]propionsäure;
2-[4-(2-{Cyclohexylmethyl-[4-(4-trifluormethylphenyl)thiazol-2₋yl]amino}ethoxy)-2-methylphenoxy]-2-methylpropionsäure;
2-[4-(2-{Cyclobutylmethyl-[4-(4-trifluormethylphenyl)thiazol-2-yl]amino}ethoxy)-2-methylphenoxy]-2-methylpropionsäure;
2-[4-(2-{Cyclopropylmethyl-[4-(4-trifluormethylphenyl)-thiazol-2-yl]amino}ethoxy)-2-methylphenoxy]-2-methylpropionsäure;
2-[4-(2-{(2-Diethylaminoethyl)-[4-(4-trifluormethylphenyl)thiazol-2-yl]amino}ethoxy)-2-methylphenoxy]-2-methylpropionsäure;
2-[2,5-Dimethyl-4-(2-{methyl-[4-(4-trifluormethylphenyl)thiazol-2-yl]amino}ethoxy)-phenoxy]-2-methylpropionsäure;
2-(2,5-Dimethyl-4-{2-[4-(4-trifluormethylphenyl)thiazol-2-ylamino]ethoxy}phenoxy)-2-methylpropionsäure;
2-[2,5-Dimethyl-4-(3-{methyl-[4-(4-trifluormethylphenyl)thiazol-2-yl]amino}propoxy)-phenoxy]-2-methylpropionsäure;
2-(2,5-Dimethyl-4-(3-[4-(4-trifluormethylphenyl)thiazol-2-ylamino]propoxy)phenoxy)-2-methylpropionsäure;
2-[2,5-Dimethyl-4-(4-{methyl-[4-(4-trifluormethylphenyl)-thiazol-2-yl]amino}butoxy)-phenoxy]-2-methylpropionsäure;
2-(2,5-Dimethyl-4-{4-[4-(4-trifluormethylphenyl)-thiazol-2-ylamino]butoxy}phenoxy)-2-methylpropionsäure;
2-(2,5-Dimethyl-4-{2-[4-(4-trifluormethoxyphenyl)thiazol-2-ylamino]ethoxy}phenoxy)-2-methylpropionsäure;
2-[2,5-Dimethyl-4-(2-{methyl-[4-(4-trifluormethoxyphenyl)thiazol-2-yl]amino}ethoxy)-phenoxy]-2-methylpropionsäure;
2-(4-{2-[4-(4-Methoxyphenyl)thiazol-2-ylamino]ethoxy}-2,5-dimethylphenoxy)-2-methylpropionsäure;
2-[4-(2-{[4-(4-Methoxyphenyl)thiazol-2-yl]methylamino}ethoxy)-2,5-dimethylphenoxy]-2-methylpropionsäure;
2-(4-{2-[(4-Biphenyl-4-ylthiazol-2-yl)methylamino]ethoxy}-2,5-dimethylphenoxy)-2-methylpropionsäure;
2-(2,5-Dimethyl-4-{2-[4-(4-trifluormethylphenyl)oxazol-2-ylamino]ethoxy}-phenoxy)-2-methylpropionsäure;
2-[2,5-Dimethyl-4-(2-{methyl-[4-(4-trifluormethylphenyl)oxazol-2-yl]amino}ethoxy)-phenoxy]-2-methylpropionsäure;
2-(2,5-Dimethyl-4-{3-[4-(4-trifluormethoxyphenyl)thiazol-2-ylamino]propoxy}phenoxy)-2-methylpropionsäure;
2-[2,5-Dimethyl-4-(3-{methyl-[4-(4-trifluormethoxyphenyl)thiazol-2-yl]amino}propoxy)-phenoxy]-2-methylpropionsäure;
2-(4-{3-[4-(4-Methoxyphenyl)thiazol-2-ylamino]propoxy}-2,5-dimethylphenoxy)-2-methylpropionsäure;
2-[4-(3-{[4-(4-Methoxyphenyl)thiazol-2-yl]methylamino}propoxy)-2,5-dimethyl-phenoxy]-2-methylpropionsäure;
2-{4-[3-(4-Biphenyl-4-ylthiazol-2-ylamino)propoxy]-2,5-dimethylphenoxy}-2-methyl-propionsäure;
2-(4-{3-[(4-Biphenyl-4-ylthiazol-2-yl)methylamino]propoxy}-2,5-dimethylphenoxy)-2-methylpropionsäure;
2-(2,5-Dimethyl-4-{3-[4-(4-trifluormethylphenyl)oxazol-2-ylamino]propoxy}phenoxy)-2-methylpropionsäure;
2-[2,5-Dimethyl-4-(3-{methyl-[4-(4-trifluormethylphenyl)oxazol-2-yl]amino}propoxy)-phenoxy]-2-methylpropionsäure;
2-(2,5-Dimethyl-4-{2-[4-(4-trifluormethylphenyl)thiazol-2-ylamino]ethylsulfanyl}-phenoxy)-2-methylpropionsäure;
2-(2,5-Dimethyl-4-{3-[4-(4-trifluormethylphenyl)thiazol-2-ylamino]propylsulfanyl}-phenoxy)-2-methylpropionsäure;
2-[2,5-Dimethyl-4-(2-{methyl-[4-(4-trifluormethylphenyl)-thiazol-2-yl]amino}ethyl-sulfanyl)phenoxy]-2-methylpropionsäure;
2-[2,5-Dimethyl-4-(3-{methyl-[4-(4-trifluormethylphenyl)thiazol-2-yl]amino}propyl-sulfanyl)phenoxy]-2-methylpropionsäure;
3-(2,5-Dimethyl-4-{2-[4-(4-trifluormethylphenyl)thiazol-2-ylamino]ethoxy}phenyl)-2,2-dimethylpropionsäure;
3-(2,5-Dimethyl-4-{3-[4-(4-trifluormethylphenyl)thiazol-2-ylamino]propoxy}phenyl)-2,2-dimethylpropionsäure;
3-[2,5-Dimethyl-4-(2-{methyl-[4-(4-trifluormethylphenyl)thiazol-2-yl]amino}ethoxy)-phenyl]-2,2-dimethylpropionsäure;
3-[2,5-Dimethyl-4-(3-{methyl-[4-(4-trifluormethylphenyl)thiazol-2-yl]amino}propoxy)-phenyl]-2,2-dimethylpropionsäure;
2-(2,5-Dimethyl-4-{2-[4-(4-trifluormethylphenyl)thiazol-2-ylamino]ethoxy}phenyl-sulfanyl)-2-methylpropionsäure;
2-Methyl-2-(2-methyl-4-{methyl-[4-(4-trifluormethylphenyl)thiazol-2-yl]sulfamoyl}-phenoxy)propionsäure;
(2-Methyl-4-{methyl-[4-(4-trifluormethylphenyl)thiazol-2-yl]sulfamoyl}phenoxy)essigsäure und
2-(2,5-Dimethyl-4-{methyl-[4-(4-trifluormethylphenyl)thiazol-2-yl]sulfamoyl}phenoxy)-2-methylpropionsäure.

6. Verbindung nach einem der Ansprüche 1 bis 5 zur Verwendung in einem Verfahren zur Behandlung einer Erkrankung oder eines Leidens bei einem Tier, wobei die Modulation der PPAR-Aktivität die Pathologie und/oder die Symptomatik der Erkrankung vermeiden, hemmen oder lindern kann.

7. Verbindung nach Anspruch 6 zur Verwendung in einem Behandlungsverfahren, worin die PPAR-Aktivität zumindest ein aus PPARα, PPARδ und PPARγ ausgewählter PPAR ist.

8. Verbindung nach Anspruch 7 zur Verwendung in einem Behandlungsverfahren, worin die PPAR-Aktivität sowohl PPARα als auch PPARδ ist.

9. Verbindung nach Anspruch 6 zur Verwendung in einem Behandlungsverfahren, worin die Erkrankung oder das Leiden aus der Behandlung oder Prophylaxe von Dyslipidämie, Hyperlipidämie, Hypercholesterinämie, Atherosklerose, Atherogenese, Hypertriglyceridämie, Herzinsuffizienz, Myokardinfarkt, Gefäßerkrankungen, Herz-Gefäß-Erkrankungen, Hypertonie, Adipositas, Kachexie, Entzündung, Arthritis, Krebs, Anorexie, Anorexia nervosa, Bulimie, Alzheimer-Erkrankung, Reizdarmerkrankungen, Colitis ulcerosa, Morbus Crohn, Typ-1-Diabetes, Typ-2-Diabetes und Syndrom X ausgewählt ist.

10. Verbindung nach Anspruch 6 zur Verwendung in einem Behandlungsverfahren, worin die Erkrankung oder das Leiden aus dem HIV-Wasting-Syndrom, lang anhaltender kritischer Erkrankung, Rückgang der Muskelmasse und/oder Muskelkraft, Rückgang der Magerkörpermasse, Aufrechterhaltung der Muskelkraft und -funktion im Alter, verminderte Muskelausdauer und Muskelfunktion, und Gebrechlichkeit im Alter ausgewählt ist.

11. Verwendung einer Verbindung nach einem der Ansprüche 1 bis 5 bei der Herstellung eines Medikaments zur Behandlung einer Erkrankung bei einem Tier, wobei die PPAR-Aktivität zur Pathologie und/oder Symptomatik der Erkrankung beiträgt.

12. Verwendung nach Anspruch 11, worin die PPAR-Aktivität zumindest ein aus PPARα, PPARδ und PPARγ ausgewählter PPAR ist.

13. Verwendung nach Anspruch 12, worin die PPAR-Aktivität sowohl PPARα als auch PPARδ ist.

14. Pharmazeutische Zusammensetzung, umfassend eine therapeutisch wirksame Menge einer Verbindung nach einem der Ansprüche 1 bis 5 in Kombination mit einem oder mehreren pharmazeutisch annehmbaren Exzipienten.

15. Pharmazeutische Kombination, insbesondere pharmazeutische Zusammensetzung, umfassend: 1) eine Verbindung nach einem der Ansprüche 1 bis 5 oder ein pharmazeutisch annehmbares Salz davon; und 2) zumindest einen aus:
a) antidiabetischen Mitteln, wie z.B. Insulin, Insulinderivaten und Mimetika; Insulinsekretagoga, wie z.B. den Sulfonylharnstoffen, z.B., Glipizid, Glyburid und Amaryl; Rezeptorliganden von insulinotropen Sulfonylharnstoff, wie z.B. Meglitiniden, z.B. Nateglinid und Repaglinid; Insulinsensibilisierungsstoffe, wie z.B. Proteintyrosinphosphatase-IB- (PTP-IB-) Inhibitoren, wie z.B. PTP-112; GSK3- (Glykogensynthase-kinase-3-) Inhibitoren, wie z.B. SB-517955, SB-4195052, SB-216763, NN-57-05441 und NN-57-05445; RXR-Liganden, wie z.B. GW-0791 und AGN-194204; natriumabhängige Glucosecotransporterinhibitoren, wie z.B. T-1095; Glykogenphosphorylase-A-Inhibitoren, wie z.B. BAY R3401; Biguaniden, wie z.B. Metformin; α-Glucosidaseinhibitoren, wie z.B. Acarbose; GLP-1 (glucagonartigem Peptid-1), GLP-1-Analoga, wie z.B. Exendin-4 und GLP-1-Mimetika; Dipeptidylpeptidase-IV-Inhibitoren, wie z.B. DPP728, Vildagliptin, MK-0431, Saxagliptin, GSK23A; einem AGE-Brecher; einem Thiazolidonderivat (Glitazone), wie z.B. Pioglitazon, Rosiglitazone oder (R)-1-{4-[5-Methyl-2-(4-trifluormethylphenyl)oxazol-4-ylmethoxy]benzolsulfonyl}-2,3-dihydro-1H-indol-2-carboxylsäure, einem PPARγ-Agonist nicht vom Typ Glitazon, z.B. Gl-262570;
b) hypolipidämischen Mitteln, wie z.B. 3-Hydroxy-3-methylglutarylcoenzym-A-(HMG-CoA)-Reductaseinhibitoren, z.B. Lovastatin, Pravastatin, Simvastatin, Pravastatin, Cerivastatin, Mevastatin, Velostatin, Fluvastatin, Dalvastatin, Atorvastatin, Rosuvastatin und Rivastatin; Squalensynthaseinhibitoren; FXR- (Farnesoid-X-Rezeptor-) und LXR- (Leber-X-Rezeptor-) Liganden; Cholestyramin; Fibrate; Nikotinsäure und Aspirin;
c) einem Mittel gegen Adipositas oder einem Appetit steuernden Mittel, wie z.B. Phentermin, Leptin, Bromocriptin, Dexamphetamin, Amphetamin, Fenfluramin, Dexfenfluramin, Sibutramin, Orlistat, Dexfenfluramin, Mazindol, Phentermin, Phendimetrazin, Diethylpropion, Fluoxetin, Bupropion, Topiramat, Diethylpropion, Benzphetamin, Phenylpropanolamin oder Ecopipam, Ephedrin, Pseudoephedrin oder Cannabinoidrezeptor-Antagonisten;
d) blutdrucksenkenden Mitteln, z.B. Schleifendiuretika, wie z.B. Etacrynsäure, Furosemid und Torsemid; Diuretika, wie z.B. Thiazidderivate, Chlorithiazid, Hydrochlorothiazid, Amilorid; Inhibitoren des Angiotensin kovenrtierenden Enzyms (ACE), wie z.B. Benazepril, Captopril, Enalapril, Fosinopril, Lisinopril, Moexipril, Perinodopril, Quinapril, Ramipril und Trandolapril; Inhibitoren der Na-K-ATPase-Membranpumpe, wie z.B. Digoxin; Neutralendopeptidase- (NEP-) Inhibitoren, z.B. Thiorphan, Terteo-thiorphan, SQ29072; ECE-Inhibitoren, z.B. SLV306; ACE/NEP-Inhibitoren, wie z.B. Omapatrilat, Sampatrilat und Fasidotril; Angiotensin-II-Antagonisten, wie z.B. Candesartan, Eprosartan, Irbesartan, Losartan, Telmisartan und Valsartan, insbesondere Valsartan; Renininhibitoren, wie z.B. Aliskiren, Terlakiren, Ditekiren, RO 66-1132, RO- 66-1168; β-adrenerger-Rezeptor-Blockern, wie z.B. Acebutolol, Atenolol, Betaxolol, Bisoprolol, Metoprolol, Nadolol, Propranolol, Sotalol und Timolol; inotropen Mitteln, wie z.B. Digoxin, Dobutamin und Milrinon; Calciumkanalblockern, wie z.B. Amlodipin, Bepridil, Diltiazem, Felodipin, Nicardipin, Nimodipin, Nifedipin, Nisoldipin und Verapamil; Aldosteronrezeptorantagonisten; und Aldosteronsynthaseinhibitoren;
e) einer HDL erhöhenden Verbindung;
f) einem Cholesterinabsorptionsmodulator, wie z.B. Zetia^{®} und KT6-971;
g) Apo-Al-Analoga und -Mimetika;
h) Thrombininhibitoren, wie z.B. Ximelagatran;
i) Aldosteroninhibitoren, wie z.B. Anastrazol, Fadrazol, Eplerenon;
j) Inhibitoren der Plättchenaggregation, wie z.B. Aspirin, Clopidogrelbisulfat;
k) Östrogen, Testosteron, einem selektiven Östrogenrezeptormodulator, einem selektiven Androgenrezeptormodulator;
l) einem chemotherapeutischen Mittel, wie z.B. Aromataseinhibitoren, z.B. Femara, Antiöstrogenen, Topoisomerase-I-Inhibitoren, Topoisomerase-II-Inhibitoren, Mikrotubuliwirkstoffe, Alkylierungsmitteln, antineoplastischen Antimetaboliten, Platinverbindungen, die Proteinkinaseaktivität senkenden Verbindungen, wie z.B. einem PDGF-Rezeptor-Tyrosinkinase-Inhibitor, vorzugsweise Imatinib oder 4-Methyl-N-[3-(4-methylimidazol-1-yl)-5-trifluormethylphenyl]-3-(4-pyridin-3-ylpyrimidin-2-ylamino)-benzamid; und
m) einem Mittel, das mit einem 5-HT₃-Receptor wechselwirkt, und/oder einem Mittel, das mit dem 5-HT₄-Rezeptor, wie z.B. Tegaserod, Tegaserodhydrogenmaleat, Cisaprid, Cilansetron;
oder jeweils einem pharmazeutisch annehmbaren Salz davon ausgewählten Wirkstoff und gegebenenfalls einen pharmazeutisch annehmbaren Träger.

16. Pharmazeutische Zusammensetzung nach Anspruch 14 oder Kombination nach Anspruch 15 zur Behandlung oder Prävention von Dyslipidämie, Hyperlipidämie, Hypercholesterinämie, Atherosklerose, Hypertriglyceridämie, Herzinsuffizienz, Myokardinfarkt, Gefäßerkrankungen, Herz-Gefäß-Erkrankungen, Hypertonie, Adipositas, Entzündung, Arthritis, Krebs, Alzheimer-Erkrankung, Hautkrankheiten, Atemwegserkrankungen, Augenerkrankungen, entzündlichen Darmerkrankungen, IBD (Reizdarmerkrankungen), Colitis ulcerosa, Morbus Crohn, Leiden, bei denen verminderte Glucosetoleranz, Hyperglykämie und Insulinresistenz impliziert sind, wie z.B. Typ-1-Diabetes und Typ-2-Diabetes, vermindertem Glucosemetabolismus (IGM), verminderter Glucosetoleranz (IGT), verschlimmerter Nüchtern-Glucose (IFG) und Syndrom X.

17. Verbindung nach einem der Ansprüche 1 bis 5, oder pharmazeutische Zusammensetzung nach Anspruch 10 oder Kombination nach Anspruch 11 zur Verwendung als Medikament.

18. Verwendung einer Verbindung nach einem der Ansprüche 1 bis 5 oder pharmazeutische Zusammensetzung nach Anspruch 14 oder Kombination nach Anspruch 15 zur Herstellung eines Medikaments zur Behandlung oder Prävention von Dyslipidämie, Hyperlipidämie, Hypercholesterinämie, Atherosklerose, Hypertriglyceridämie, Herzinsuffizienz, Myokardinfarkt, Gefäßerkrankungen, Herz-Gefäß-Erkrankungen, Hypertonie, Adipositas, Entzündung, Arthritis, Krebs, Alzheimer-Erkrankung, Hautkrankheiten, Atemwegserkrankungen, Augenerkrankungen, entzündlichen Darmerkrankungen, IBD (Reizdarmerkrankungen), Colitis ulcerosa, Morbus Crohn, Leiden, bei denen verminderte Glucosetoleranz, Hyperglykämie und Insulinresistenz impliziert sind, wie z.B. Typ-1-Diabetes und Typ-2-Diabetes, vermindertem Glucosemetabolismus (IGM), verminderter Glucosetoleranz (IGT), verschlimmerter Nüchtern-Glucose (IFG) und Syndrom X.

## Revendications

1. Composé de formule I : 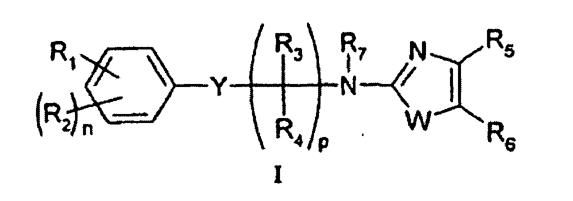 dans laquelle
n est choisi parmi 0, 1, 2 et 3 ;
p est choisi parmi 0, 1, 2 et 3 ;
Y est choisi parmi O, S(O)0 à 2 et NR7a, où R7a est choisi parmi un atome d'hydrogène et un groupe alkyle en C1 à 6 ;
W est choisi parmi O et S ;
R1 est choisi parmi -X1CR9R10X2CO2R11, - X1SCR9R10X2CO2R11 and -X1OCR9R10X2CO2R11 ; où X1 et X2 sont indépendamment choisis parmi une liaison et un groupe alkylène en C1 à 4 ; et R9 et R10 sont indépendamment choisis parmi un atome d'hydrogène, un groupe alkyle en C1 à 4 et alcoxy en C1 à 4 ; ou bien R9 et R10 conjointement avec l'atome de carbone auquel R9 et R10 sont attachés forment un groupe cycloalkyle en C3 à 12 ; et R11 est choisi parmi un atome d'hydrogène et un groupe alkyle en C1 à 6 ; chaque
R2 est indépendamment choisi parmi un groupe halogéno, alkyle en C1 à 6, alcényle en C2 à 6, alcoxy en C1 à 4, alkylthio en C1 à 4, cycloalkyle en C3 à 12, hétérocycloalkyle en C3 à 8, aryle en C6 à 10 et hétéroaryle en C5 à 10 ; où tout aryle, hétéroaryle, cycloalkyle ou hétérocycloalkyle de R2 est facultativement substitué par 1 à 3 radicaux indépendamment choisis parmi un groupe halogéno, alkyle en C1 à 6, alcoxy en C1 à 6, alcényle en C2 à 6, alkylthio en C1 à 6, alkyle en C1 à 6 substitué par halogéno, alcoxy en C1 à 6 substitué par halogéno, -C(O)R14a et NR14aR14b ; où R14a et R14b sont indépendamment choisis parmi un atome d'hydrogène et un groupe alkyle en C1 à 6 ;
R3 et R4 sont indépendamment choisis parmi un atome d'hydrogène et un groupe alkyle en C1 à 6 ;
R5 est un groupe aryle en C6 à 10 facultativement substitué par 1 à 3 radicaux indépendamment choisis parmi un groupe halogéno, nitro, cyano, alkyle en C1 à 6, alcoxy en C1 à 6, alkylthio en C1 à 6, hydroxy-alkyle en C1 à 6, alkyle en C1 à 6 substitué par halogéno, alcoxy en C1 à 6 substitué par halogéno, cycloalkyle en C3 à 12, hétérocycloalkyle en C3 à 8, aryle en C6 à 10, hétéroaryle en C5 à 13 et -XNR12R12 ; où R12 est choisi parmi un atome d'hydrogène et un groupe alkyle en C1 à 6 ;
R6 est choisi parmi un atome d'hydrogène et un groupe méthyle ; et
R7 est choisi parmi un atome d'hydrogène, un groupe alkyle en C1 à 6, aryl en C6 à 12-alkyle en C0 à 4, cycloalkyl en C3 à 12-alkyle en C0 à 4, -XOR14a et -XNR14aR14b ; où X est une liaison ou un groupe alkylène en C1 à 4 ; et R14a et R14b sont indépendamment choisis parmi un atome d'hydrogène et un groupe alkyle en C1 à 6 ;
et sels, hydrates, solvates et isomères pharmaceutiquement acceptables de celui-ci.

2. Composé selon la revendication 1, dans lequel :
n est choisi parmi 0, 1, 2 et 3 ;
p est choisi parmi 0, 1 et 2 ;
Y est choisi parmi O et S(O)0 à 2 ;
W est choisi parmi O et S ;
R1 est choisi parmi -X1CR9R10X2CO2R11, - X1SCR9R10X2CO2R11 et -X1OCR9R10X2CO2R11 ; où X1 et X2 sont indépendamment choisis parmi une liaison et un groupe alkylène en C1 à 4 ; et R9 et R10 sont indépendamment choisis parmi un atome d'hydrogène, un groupe alkyle en C1 à 4 et alcoxy en C1 à 4 ; ou bien R9 et R10 conjointement avec l'atome de carbone auquel R9 et R10 sont attachés forment un groupe cycloalkyle en C3 à 12 ; et R11 est choisi parmi un atome d'hydrogène et un groupe alkyle en C1 à 6 ; chaque
R2 est indépendamment choisi parmi un groupe alkyle en C1 à 6, alcényle en C2 à 6, alcoxy en C1 à 4, alkylthio en C1 à 4, cycloalkyle en C3 à 12, hétérocycloalkyle en C3 à 8, aryle en C6 à 10 et hétéroaryle en C5 à 10 ; où tout aryle, hétéroaryle, cycloalkyle ou hétérocycloalkyle de R2 est facultativement substitué par 1 à 3 radicaux indépendamment choisis parmi un groupe halogéno, alcoxy en C1 à 6, alkylthio en C1 à 6, alcoxy en C1 à 6 substitué par halogéno, -C(O)R14a et NR14aR14b ; où R14a et R14b sont indépendamment choisis parmi un atome d'hydrogène et un groupe alkyle en C1 à 6 ;
R3 et R4 sont indépendamment choisis parmi un atome d'hydrogène et un groupe alkyle en C1 à 6 ;
R5 est un groupe aryle en C6 à 10 facultativement substitué par 1 à 3 radicaux indépendamment choisis parmi un groupe halogéno, nitro, cyano, alkyle en C1 à 6, alcoxy en C1 à 6, alkylthio en C1 à 6, hydroxy-alkyl en C1 à 6, alkyle en C1 à 6 substitué par halogéno, alcoxy en C1 à 6 substitué par halogéno, cycloalkyle en C3 à 12, hétérocycloalkyle en C3 à 8, aryle en C6 à 10, hétéroaryle en C5 à 13 et -XNR12R12 ; où R12 est choisi parmi un atome d'hydrogène et un groupe alkyle en C1 à 6 ;
R6 est choisi parmi un atome d'hydrogène et un groupe méthyle ; et
R7 est choisi parmi un atome d'hydrogène, un groupe alkyle en C1 à 6, aryl en C6 à 12-alkyle en C0 à 4, cycloalkyl en C3 à 12-alkyle en C0 à 4, -XOR14a et -XNR14aR14b ; où X est une liaison ou un groupe alkylène en C1 à 4 ; et R14a et R14b sont indépendamment choisis parmi un atome d'hydrogène et un groupe alkyle en C1 à 6.

3. Composé selon la revendication 2, dans lequel R1 est choisi parmi -CH2CRSR6CO2H, -OCR5R6CO2H, -SCR5R6CO2H, - CR5R6CH2CO2H et -CRSR6CO2H ; où R5 et R6 sont indépendamment choisis parmi un atome d'hydrogène, un groupe méthyle, méthoxy et éthoxy ; ou R5 et R6 conjointement avec l'atome de carbone auquel R5 et R6 sont attachés forment un groupe cyclopentyle.

4. Composé selon la revendication 3, dans lequel chaque R2 est indépendamment choisi parmi un groupe méthyle, éthyle, cyclopropyle, méthoxy, furanyle, phényle, pyridinyle, thiényle, pyrrolidinyle et benzo[1,3]dioxolyle ; où ledit groupe pyridinyle ou phényle de R2 est facultativement substitué par 1 à 3 radicaux indépendamment choisis parmi un groupe halogéno, méthyl-carbonyle, diméthyl-amino, méthoxy, méthoxy substitué par halogéno, méthyl-thio, éthényle, hexényle et propyloxy ; et R7 est choisi parmi un atome d'hydrogène, un groupe méthyle, isopropyle, propyle, pentyle, isobutyle, méthoxy-éthyle, benzyle, phénéthyle, cyclohexyl-méthyle, cyclobutyl-méthyle, cyclopropyl-méthyle et diéthyl-amino-éthyle.

5. Composé selon la revendication 1, choisi parmi :
l'acide 2-méthyl-2-[2-méthyl-4-(2-{méthyl-[4-(4-trifluorométhyl-phényl)-thiazol-2-yl]-amino}-éthoxy)-phénoxy]-propionique ; l'acide 2-méthyl-2-(2-méthyl-4-{2-[4-(4-trifluorométhyl-phényl)-thiazol-2-ylamino]-éthoxy}-phénoxy)-propionique ; l'acide 2-méthyl-2-[2-méthyl-4-(2-{propyl-[4-(4-trifluorométhyl-phényl)-thiazol-2-yl]-amino}-éthoxy)-phénoxy]-propionique ; l'acide 2-méthyl-2-[2-méthyl-4-(2-{pentyl-[4-(4-trifluorométhyl-phényl)-thiazol-2-yl]-amino}-éthoxy)-phénoxy]propionique ; l'acide 2-[4-(2-{isopropyl-[4-(4-trifluorométhyl-phényl)-thiazol-2-yl]-amino}-éthoxy)-2-méthyl-phénoxy]-2-méthyl-propionique ; l'acide 2-[4-(2-{isobutyl-[4-(4-trifluorométhyl-phényl)-thiazol-2-yl]-amino}-éthoxy)-2-méthyl-phénoxy]-2-méthyl-propionique ; l'acide 2-[4-(2-{(2-méthoxy-éthyl)-[4-(4-trifluorométhyl-phényl)-thiazol-2-yl]amino}-éthoxy)-2-méthyl-phénoxy]-2-méthyl-propionique ;
l'acide 2-[4-(2-{benzyl-[4-(4-trifluorométhyl-phényl)-thiazol-2-yl]-amino}-éthoxy)-2-méthyl-phénoxy]-2-méthyl-propionique ;
l'acide 2-méthyl-2-[2-méthyl-4-(2-{phénéthyl-[4-(4-trifluorométhyl-phényl)-thiazol-2-yl]amino}-éthoxy)-phénoxy]-propionique ; l'acide 2-[4-(2-{cyclohexylméthyl-[4-(4-trifluorométhyl-phényl)-thiazol-2-yl]amino}-éthoxy)-2-méthyl-phénoxy]-2-méthyl-propionique ; l'acide 2-[4-(2-{cyclobutylméthyl-[4-(4-trifluorométhyl-phényl)-thiazol-2-yl]amino}-éthoxy)-2-méthyl-phénoxy]-2-méthyl-propionique ;
l'acide 2-[4-(2-{cyclopropylméthyl-[4-(4-trifluorométhyl-phényl)-thiazol-2-yl]-amino}-éthoxy)-2-méthyl-phénoxy]-2-méthyl-propionique ; l'acide 2-[4-(2-{(2-diéthylamino-éthyl)-[4-(4-trifluorométhyl-phényl)-thiazol-2-yl]-amino}-éthoxy)-2-méthyl-phénoxy]-2-méthyl-propionique ; l'acide 2-(2,5-diméthyl-4-(2-{méthyl-[4-(4-trifluorométhyl-phényl)-thiazol-2-yl]-amino)-éthoxy)-phénoxy)-2-méthyl-propionique ; l'acide 2-(2,5-diméthyl-4-{2-[4-(4-trifluorométhyl-phényl)-thiazol-2-ylamino}-éthoxy}-phénoxy)-2-méthyl-propionique ; l'acide 2-[2,5-diméthyl-4-(3-{méthyl-[4-(4-trifluorométhyl-phényl)-thiazol-2-yl]-amino}-propoxy)-phénoxy]-2-méthyl-propionique ;
l'acide 2-(2,5-diméthyl-4-{3-[4-(4-trifluorométhyl-phényl)-thiazol-2-ylamino]-propoxy}-phénoxy)-2-méthyl-propionique ;
l'acide 2-(2,5-diméthyl-4-(4-{méthyl-[4-(4-trifluorométhyl-phényl)-thiazol-2-yl]-amino}-butoxy)-phénoxy]-2-méthyl-propionique ; l'acide 2-(2,5-diméthyl-4-{4-[4-(4-trifluorométhyl-phényl)-thiazol-2-ylamino]-butoxy}-phénoxy)-2-méthyl-propionique ; l'acide 2-(2,5-diméthyl-4-{2-[4-(4-trifluoroéthoxy-phényl)-thiazol-2-ylamino]éthoxy}-phénoxy)-2-méthyl-propionique ; l'acide 2-[2,5-diméthyl-4-(2-{méthyl-[4-(4-trifluorométhoxy-phényl)-thiazol-2-yl]-amino}-éthoxy)-phénoxy]-2-méthyl-propionique ; l'acide 2-(4-{2-[4-(4-méthoxy-phényl)-thiazol-2-ylamino]-éthoxy)-2,5-diméthyl-phénoxy)-2-méthyl-propionique ; l'acide 2-[4-(2-{[4-(4-méthoxy-phényl)-thiazol-2-yl]-méthyl-amino}-éthoxy)-2,5-diméthyl-phénoxy]-2-méthyl-propionique ; l'acide 2-(4-{2-[(4-biphényl-4-yl-thiazol-2-yl)-méthyl-amino]-éthoxy}-2,5-diméthyl-phénoxy)-2-méthyl-propionique ; l'acide 2-(2,5-diméthyl-4-{2-[4-(4-trifluorométhyl-phényl)-oxazol-2-ylamino]-éthoxy}-phénoxy)-2-méthyl-propionique ; l'acide 2-(2,5-diméthyl-4-(2-{méthyl-[4-(4-trifluorométhyl-phényl)-oxazol-2-yl]-amino}-éthoxy)-phénoxy]-2-méthyl-propionique ; l'acide 2-(2,5-diméthyl-4-{3-[4-(4-trifluorométhoxy-phényl)-thiazol-2-ylamino]-propoxy}-phénoxy)-2-méthyl-propionique ; l'acide 2-[2,5-diméthyl-4-(3-{méthyl-[4-(4-trifluorométhoxy-phényl)-thiazol-2-yl]-amino}-propoxy)-phénoxy]-2-méthyl-propionique ; l'acide 2-{4-(3-[4-(4-méthoxy-phényl)-thiazol-2-ylamino]-propoxy}-2,5-diméthyl-phénoxy)-2-méthyl-propionique ; l'acide 2-{4-(3-{[4-(4-méthoxy-phényl)-thiazol-2-yl]-méthyl-amino}-propoxy)-2,5-diméthyl-phénoxy]-2-méthyl-propionique ; l'acide 2-(4-[3-(4-biphényl-4-yl-thiazol-2-ylamino)-propoxy]-2,5-diméthyl-phénoxy}-2-méthyl-propionique ; l'acide 2-{4-{3-[(4-biphényl-4-yl-thiazol-2-yl)-méthyl-amino]-propoxy}-2,5-diméthyl-phénoxy)-2-méthyl-propionique ; l'acide 2-(2,5-diméthyl-4-{3-[4-(4-trifluorométhyl-phényl)-oxazol-2-ylamino]-propoxy}-phénoxy)-2-méthyl-propionique ; l'acide 2-[2,5-diméthyl-4-(3-{méthyl-[4-(4-trifluorométhyl-phényl)-oxazol-2-yl]-amino}-propoxy)-phénoxy]-2-méthyl-propionique ; l'acide 2-(2,5-diméthyl-4-{2-[4-(4-trifluorométhyl-phényl)-thiazol-2-ylamino]-éthylsulfanyl}-phénoxy)-2-méthyl-propionique ;
l'acide 2-(2,5-diméthyl-4-(3-[4-(4-trifluorométhyl-phényl)-thiazol-2-ylamino]-propylsulfanyl}-phénoxy)-2-méthyl-propionique ; l'acide 2-[2,5-diméthyl-4-(2-{méthyl-[4-(4-trifluorométhyl-phényl)-thiazol-2-yl]-amino}-éthylsulfanyl)-phénoxyl-2-méthyl-propionique ; l'acide 2-[2,5-diméthyl-4-(3-{méthyl-[4-(4-trifluorométhyl-phényl)-thiazol-2-yl]-amino}-propylsulfanyl)-phénoxyl-2-méthyl-propionique ; l'acide 3-(2,5-diméthyl-4-{2-[4-(4-trifluorométhyl-phényl)-thiazol-2-ylamino]-éthoxy}-phényl)-2,2-diméthyl-propionique ; l'acide 3-(2,5-diméthyl-4-{3-[4-(4-trifluorométhyl-phényl)-thiazol-2-ylamino]-propoxy}-phényl)-2,2-diméthyl-propionique ; l'acide 3-[2,5-diméthyl-4-(2-{méthyl-[4-(4-trifluorométhyl-phényl)-thiazol-2-yl]-amino}-éthoxy)-phényl]-2,2-diméthyl-propionique ; l'acide 3-[2,5-diméthyl-4-(3-{méthyl-[4-(4-trifluorométhyl-phényl)-thiazol-2-yl]-amino}-propoxy)-phényl]-2,2-diméthyl-propionique ; l'acide 2-(2,5-diméthyl-4-{2-[4-(4-trifluorométhyl-phényl)-thiazol-2-ylamino]-éthoxy}-phénylsulfanyl)-2-méthyl-propionique ; l'acide 2-méthyl-2-(2-méthyl-4-{méthyl-[4-(4-trifluorométhyl-phényl)-thiazol-2-yl]-sulfamoyl}-phénoxy)-propionique ; l'acide (2-méthyl-4-{méthyl-[4-(4-trifluorométhyl-phényl)-thiazol-2-yl]-sulfamoyl}-phénoxy)-acétique ; et l'acide 2-(2,5-diméthyl-4-(méthyl-{4-(4-trifluorométhyl-phényl)-thiazol-2-yl]-sulfamoyl}-phénoxy)-2-méthyl-propionique.

6. Composé selon l'une quelconque des revendications 1 à 5, à utiliser dans un procédé de traitement d'une maladie ou d'un trouble chez un animal dans lequel une modulation de l'activité de PPAR peut prévenir, inhiber ou améliorer la pathologie et/ou la symptomatologie de la maladie.

7. Composé à utiliser dans un procédé de traitement selon la revendication 6, dans lequel l'activité de PPAR concerne au moins un PPAR choisi parmi PPARα, PPARδ et PPARγ.

8. Composé à utiliser dans un procédé de traitement selon la revendication 7, dans lequel l'activité de PPAR concerne à la fois PPARα et PPARδ.

9. Composé à utiliser dans un procédé de traitement selon la revendication 6, dans lequel la maladie ou le trouble est choisi par rapport au traitement ou à la prophylaxie de la dyslipidémie, l'hyperlipidémie, l'hypercholestérémie, l'athérosclérose, l'athérogenèse, l'hypertriglycéridémie, une insuffisance cardiaque, un infarctus du myocarde, les maladies vasculaires, les maladies cardiovasculaires, l'hypertension, l'obésité, la cachexie, une inflammation, l'arthrite, le cancer, l'anorexie, l'anorexie nerveuse, la boulimie, la maladie d'Alzheimer, les troubles cutanés, les maladies respiratoires, les troubles ophtalmiques, les syndromes du côlon irritable, la recto-colite ulcérative, la maladie de Crohn, le diabète de type 1, le diabète de type 2 et le syndrome X.

10. Composé à utiliser dans un procédé de traitement selon la revendication 6, dans lequel la maladie ou le trouble est choisi parmi le syndrome cachectique lié au VIH, une longue maladie critique, une diminution de la masse musculaire et/ou de la force musculaire, une diminution de la masse maigre, le maintien de la force musculaire et de la fonction musculaire chez les personnes âgées, une diminution de l'endurance musculaire et de la fonction musculaire, et une fragilité chez les personnes âgées.

11. Utilisation d'un composé selon l'une quelconque des revendications 1 à 5, dans la fabrication d'un médicament destiné à traiter les maladies chez un animal dans lequel une activité de PPAR contribue à la pathologie et/ou à la symptomatologie de la maladie.

12. Utilisation selon la revendication 11, dans laquelle l'activité de PPAR concerne au moins un PPAR choisi parmi PPARα, PPARδ et PPARγ.

13. Utilisation selon la revendication 12, dans laquelle l'activité de PPAR concerne à la fois PPARα et PPARδ.

14. Composition pharmaceutique comprenant une quantité thérapeutiquement efficace d'un composé selon l'une quelconque des revendications 1 à 5 en combinaison avec un ou plusieurs excipients pharmaceutiquement acceptables.

15. Combinaison pharmaceutique, en particulier composition pharmaceutique, comprenant : 1) un composé selon l'une quelconque des revendications 1 à 5 ou un sel pharmaceutiquement acceptable de celui-ci ; et 2) au moins un ingrédient actif choisi parmi :
a) les agents antidiabétiques tels que l'insuline, les dérivés et substances mimétiques de l'insuline ; les secrétagogues de l'insuline tels que les sulfonylurées, par exemple Glipizide, glyburide et Amaryl ; les ligands du récepteur de sulfonylurée insulinotropes tels que les méglitinides, par exemple natéglinide et répaglinide ; un insulinosensibilisant tel que les inhibiteurs de protéine tyrosine phosphatase-1B (PTP-1B) tels que PTP-112 ; les inhibiteurs de GSK3 (glycogène synthase kinase-3) tels que SB-517955, SB-4195052, SB-216763, NN-57-05441 et NN-57-05445 ; les ligands de RXR tels que GW-0791 et AGN-194204 ; les inhibiteurs du co-transporteur de glucose dépendant du sodium tels que T-1095 ; les inhibiteurs de glycogène phosphorylase A tels que BAY R3401 ; les biguanides tels que metformine ; les inhibiteurs d'alpha-glucosidase tels qu'acarbose ; le GLP-1 (peptide-1 de type glucagon), les analogues de GLP-1 tels que l'Exendin-4 et les substances mimétiques de GLP-1 ; les inhibiteurs de dipeptidyl peptidase IV tels que DPP728, vildagliptine, MC-0431, saxagliptine, GSK23A ; une substance de rupture d'AGE ; un dérivé de thiazolidone (glitazone) tel que pioglitazone, rosiglitazone, ou l'acide (R)-1-{4-[5-méthyl-2-(4-trifluorométhyl-phényl)-oxazol-4-ylméthoxy]-benzènesulfonyl}-2,3-dihydro-1H-indole-2-carboxylique, un agoniste de PPARγ de type non-glitazone, par exemple GI-262570 ;
b) les agents hypolipidémiants tels que les inhibiteurs de 3-hydroxy-3-méthyl-glutaryle coenzyme A (HMG-CoA) réductase, par exemple lovastatine, pitavastatine, simvastatine, pravastatine, cérivastatine, mévastatine, vélostatine, fluvastatine, dalvastatine, atorvastatine, rosuvastatine et rivastatine ; les inhibiteurs de squalène synthase ; les ligands de FXR (récepteur de farnésoïde X) et LXR (récepteur de foie X) ; la cholestyramine ; les fibrates ; l'acide nicotinique et l'aspirine ;
c) un agent anti-obésité ou un agent régulant l'appétit tel que phentermine, leptine, bromocriptine, dexamphétamine, amphétamine, fenfluramine, dexfenfluramine, sibutramine, orlistat, dexfenfluramine, mazindol, phentermine, phendimétrazine, diéthylpropion, fluoxétine, bupropion, topiramate, diéthylpropion, benzphétamine, phénylpropanolamine ou écopipam, éphédrine, pseudoéphédrine ou les antagonistes de récepteur cannabinoïde ;
d) les agents anti-hypertenseurs, par exemple les diurétiques de l'anse de Henle tels qu'acide éthacrynique, furosémide et torsémide ; les diurétiques tels que les dérivés de thiazide, chlorothiazide, hydrochlorothiazide, amiloride ; les inhibiteurs de l'enzyme de conversion de l'angiotensine (ACE) tels que bénazépril, captopril, énalapril, fosinopril, lisinopril, moéxipril, perinodopril, quinapril, ramipril et trandolapril ; les inhibiteurs de la pompe de membrane de Na-K-ATPase tels que digoxine ; les inhibiteurs de neutralendopeptidase (NEP) par exemple thiorphan, tertéothiorphan, SQ29072 ; les inhibiteurs d'ECE, par exemple SLV306 ; les inhibiteurs d'ACE/NEP tels qu'omapatrilat, sampatrilat et fasidotril ; les antagonistes de l'angiotensine II tels que candésartan, éprosartan, irbésartan, losartan, telmisartan et valsartan, en particulier valsartan ; les inhibiteurs de rénine tels qu'aliskirène, terlakirène, ditékirène, RO 66-1132, RO-66-1168 ; les inhibiteurs du récepteur β-adrénergique tels qu'acébutolol, aténolol, bétaxolol, bisoprolol, métoprolol, nadolol, propranolol, sotalol et timolol ; les agents inotropes tels que digoxine, dobutamine et milrinone ; les inhibiteurs des canaux calciques tels que amlodipine, bépridil, diltiazem, félodipine, nicardipine, nimodipine, nifédipine, nisoldipine et vérapamil ; les antagonistes du récepteur de l'aldostérone ; et les inhibiteurs de l'aldostérone synthase ;
e) un composé augmentant la HDL ;
f) un modulateur d'absorption du cholestérol tel que Zetia^{®} et KT6-971 ;
g) les analogues et substances mimétiques d'Apo-A1 ;
h) les inhibiteurs de la thrombine tels que Ximélagatran ;
i) les inhibiteurs de l'aldostérone tels qu'anastrazole, fadrazole, éplérénone ;
j) les inhibiteurs d'agrégation plaquettaire tels qu'aspirine, bisulfate de clopidogrel ;
k) l'oestrogène, la testostérone, un modulateur sélectif des récepteurs des oestrogènes, un modulateur sélectif des récepteurs des androgènes ;
l) un agent chimiothérapeutique tel que les inhibiteurs de l'aromatase par exemple fémara, les anti-oestrogènes, les inhibiteurs de topoisomérase I, les inhibiteurs de topoisomérase II, les agents actifs sur les microtubules, les agents d'alkylation, les antimétabolites antinéoplasiques, les composés du platine, les composés diminuant l'activité de la protéine kinase tels que l'inhibiteur de la tyrosine kinase du récepteur de PDGF, de préférence Imatinib ou 4-méthyl-N-[3-(4-méthyl-imidazol-1-yl)-5-trifluorométhyl-phényl]-3-(4-pyridin-3-yl-pyrimidin-2-ylamino)-benzamide ; et
m) un agent interagissant avec un récepteur de 5-HT3 et/ou un agent interagissant avec un récepteur de 5-HT4 tels que tégasérod, maléate d'hydrogène de tégasérod, cisapride, cilansétron ;
ou, dans chaque cas, un sel pharmaceutiquement acceptable de ceux-ci ; et facultativement un support pharmaceutiquement acceptable.

16. Composition pharmaceutique selon la revendication 14 ou combinaison selon la revendication 15, pour le traitement ou la prévention de la dyslipidémie, l'hyperlipidémie, l'hypercholestérémie, l'athérosclérose, l'hypertriglycéridémie, une insuffisance cardiaque, un infarctus du myocarde, les maladies vasculaires, les maladies cardiovasculaires, l'hypertension, l'obésité, une inflammation, l'arthrite, le cancer, la maladie d'Alzheimer, les troubles cutanés, les maladies respiratoires, les troubles ophtalmiques, les maladies abdominales inflammatoires, l'IBD (syndrome du côlon irritable), la rectocolite ulcérative, la maladie de Crohn, les affections dans lesquelles une altération de la tolérance au glucose, une hyperglycémie et une résistance à l'insuline sont impliquées, telles que diabète de type 1 et diabète de type 2, une altération du métabolisme du glucose (IGM), une altération de la tolérance au glucose (IGT), une altération du glucose à jeun (IFG) et le syndrome X.

17. Composé selon l'une quelconque des revendications 1 à 5, ou composition pharmaceutique selon la revendication 10 ou combinaison selon la revendication 11, pour utilisation comme médicament.

18. Utilisation d'un composé selon l'une quelconque des revendications 1 à 5, ou d'une composition pharmaceutique selon la revendication 14, ou d'une combinaison selon la revendication 15, pour la fabrication d'un médicament destiné au traitement ou à la prévention de la dyslipidémie, l'hyperlipidémie, l'hypercholestérémie, l'athérosclérose, l'hypertriglycéridémie, une insuffisance cardiaque, un infarctus du myocarde, les maladies vasculaires, les maladies cardiovasculaires, l'hypertension, l'obésité, une inflammation, l'arthrite, le cancer, la maladie d'Alzheimer, les troubles cutanés, les maladies respiratoires, les troubles ophtalmiques, les maladies abdominales inflammatoires, l'IBD (syndrome du côlon irritable), la rectocolite ulcérative, la maladie de Crohn, les affections dans lesquelles une altération de la tolérance au glucose, une hyperglycémie et une résistance à l'insuline sont impliquées, telles que diabète de type 1 et diabète de type 2, une altération du métabolisme du glucose (IGM), une altération de la tolérance au glucose (IGT), une altération du glucose à jeun (IFG) et le syndrome X.
